(19) Europäisches Patentamt
European Patent Office
Office européen des brevets

(11) EP 0 741 697 B1

(12) **EUROPEAN PATENT SPECIFICATION**

(45) Date of publication and mention of the grant of the patent:
**15.09.1999 Bulletin 1999/37**

(21) Application number: **95906504.6**

(22) Date of filing: **23.01.1995**

(51) Int Cl.[6]: **C07C 271/22**, C07C 233/83, C07C 233/51, C07C 271/54, C07C 311/42, C07C 229/24, A61K 31/325, A61K 31/16

(86) International application number:
**PCT/JP95/00068**

(87) International publication number:
**WO 95/19959 (27.07.1995 Gazette 1995/32)**

(54) **AMINO ACID DERIVATIVES AND THEIR USE AS PHOSPHOLIPASE A2 INHIBITORS**
AMINOSÄUREDERIVATE UND IHRE VERWENDUNG ALS PHOSPHOLIPASE-A2-INHIBITOREN
DERIVES D'ACIDE AMINE ET LEUR UTILISATION EN TANT QU'INHIBITEURS DE LA PHOSPHOLIPASE A2

(84) Designated Contracting States:
**AT BE CH DE DK ES FR GB GR IE IT LI LU NL PT SE**

(30) Priority: **24.01.1994 GB 9401268**
**31.10.1994 GB 9421962**

(43) Date of publication of application:
**13.11.1996 Bulletin 1996/46**

(73) Proprietor: **FUJISAWA PHARMACEUTICAL CO., LTD.**
**Osaka-shi Osaka 541-8514 (JP)**

(72) Inventors:
- **TAKASE, Shigehiro**
  **Ishioka-shi, Ibaraki 315 (JP)**
- **SHIGEMATSU, Nobuharu**
  **Tsukuba-shi, Ibaraki 305 (JP)**
- **YOSHIMURA, Seiji**
  **Tsukuba-shi, Ibaraki 305 (JP)**
- **OKADA, Satoshi**
  **Souraku-gun, Kyoto 619-02 (JP)**
- **HEMMI, Keiji**
  **Tsukuba-shi, Ibaraki 305 (JP)**
- **TANAKA, Hirokazu**
  **Takarazuka-shi, Hyogo 665 (JP)**
- **OTSUKA, Takanao**
  **Tsukuba-shi, Ibaraki 305 (JP)**
- **TSURUMI, Yasuhisa**
  **Tsukuba-shi, Ibaraki 305 (JP)**
- **OKAMOTO, Masanori**
  **Toyono-gun, Osaka 563-01 (JP)**
- **OKUHARA, Masakuni**
  **Tsukuba-shi, Ibaraki 305 (JP)**
- **FUKAMI, Naoki**
  **Yuuki-gun, Ibaraki 300-34 (JP)**

(74) Representative: **Türk, Gille, Hrabal**
**Patentanwälte - European Patent Attorneys,**
**Brucknerstrasse 20**
**40593 Düsseldorf (DE)**

(56) References cited:
- **CHEMICAL ABSTRACTS, vol. 120, no. 13, 28 March 1994, Columbus, Ohio, US; abstract no. 164910, CH. PENNEY, Z. BOULOS page 1266 ; & WO,A,93 20100 (BIOCHEM PHARM.)**
- **CHEMICAL ABSTRACTS, vol. 116, no. 1, 6 January 1992, Columbus, Ohio, US; abstract no. 6968, T. ICHIKAWA ET AL. page 691 ; & EP,A,0 443 592 (AJINOMOTO)**
- **CHEMICAL ABSTRACTS, vol. 112, no. 12, 19 March 1990, Columbus, Ohio, US; abstract no. 99361, K. HANABUSA ET AL. page 7 ; & J. MACROMOL. SCI., CHEM., vol.A26, no.12, 1989 pages 1571 - 1584**
- **CHEMICAL ABSTRACTS, vol. 112, no. 1, 1 January 1990, Columbus, Ohio, US; abstract no. 7867, D. CANTACUZENE, C. GUERREIRO page 774 ; & TETRAHEDRON, vol.45, no.3, 1989 pages 741 - 748**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- CHEMICAL ABSTRACTS, vol. 108, no. 9, 29 February 1988, Columbus, Ohio, US; abstract no. 75826, P. JOUIN ET AL. page 728 ; & TETRAHEDRON LETT., vol.28, no.15, 1987 pages 1665 - 1668

## Description

The present invention relates to a novel fatty acid derivative and a pharmaceutically acceptable salt thereof which are useful as a medicament.

BACKGROUND ART

A phospholipase $A_2$ inhibitor having the structure of that of the present invention has not been known.
CHEM. ABST: v. 120, n. 13, abstr. 164910 (1994) and WO-A-93 20100 discloses lipophilic oligopeptides with immunomodulating activity which can be used in conjunction with anti-viral or anti-cancer agents.
CHEM. ABST: v. 116, n. 1, abstr. 6968 (1992) and EP-A-0 443 592 discloses sterol esters with n-acylamino acids as emulsifying agents and moisturizers for cosmetics.
CHEM. ABST: v. 112, n. 12, abstr. 99361 (1990) and J. Macrom. Sci. Chem. v. A26, n. 12, p. 1571, 1989 discloses the synthesis of L-lysine and L-glutamic acid derivatives with long alkyl chains and their polycondensation in mono-layers and multilayers.
CHEM. ABST: v. 112, n.1, abst. 7867 (1990) and Tetrahedron, v. 45, n. 3, p. 741, 1989 discloses the papain-catalyzed esterification of amino acids by alcohols and diols.
CHEM. ABST: v. 108, n. 9, abstr. 75826 (1988) and Tetrahedron Lett. v. 28, n. 15, p. 1665, 1987 discloses monoesterification of N-protected aspartic and glutamic acids by chloroformate activation.

DISCLOSURE OF INVENTION

The present invention relates to novel fatty acid derivative and a pharmaceutically acceptable salt thereof, which are phospholipase $A_2$ inhibitors and are useful for the prevention and/or the treatment of pancreatitis, hepatitis, chronic renal failure, etc; shock (e.g. endotoxin shock, gram-negative septic shock, etc), arthritis (e.g. rheumatoid arthritis, osteoarthritis, etc), respiratory disease (e.g. bronchial asthma, bronchitis, adult respiratory distress syndrome, etc), heart disease (e.g. myocardial ischemia, etc), allergic disease, thrombosis, arteriosclerosis, pain, autoimmune disease, dermal disease (e.g. atopic dermatitis, psoriasis, contact dermatitis, etc), inflammatory bowel disease (e.g. Crohn's disease, ulcerative colitis, etc), ophthalmic disease (e.g. allergic ophthalmic disease, inflammatory ophthalmic disease, etc), nasal diseases (e.g. allergic rhinitis, etc), gout, trauma induced inflammation (e.g. spinal cord injury, etc), liver diseases (e.g. cirrhosis, hepatitis, etc), or the like; to a process for preparation thereof, to a pharmaceutical composition comprising the same, and to the use of the same for the manufacture of a medicament for the prevention and/or treatment of the aforesaid diseases.
The object fatty acid derivative can be represented by the following formula (I) :

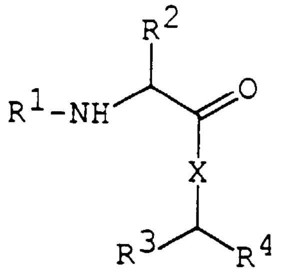

(I)

wherein

- $R^1$ is acyl group or lower aliphatic hydrocarbon group which may have one or more suitable substituent(s),
- $R^2$ is acyl(lower)alkyl,
- $R^3$ is lower alkyl or higher alkyl,
- $R^4$ is acyl group, acyl(lower)alkyl, or cyano(lower)alkyl, and
- X is -O- or

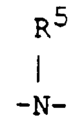

[wherein $R^5$ is hydrogen or acyl(lower)alkyl].

It is to be noted the object compound (I) may include one or more stereoisomers due to asymmetric carbon atom(s) and double bond, and all of such isomers and a mixture thereof are included within the scope of the present invention.

It is further to be noted isomerization or rearrangement of the object compound (I) may occur due to the effect of the light, acid, base or the like, and the compound obtained as the result of said isomerization or rearrangement is also included within the scope of the present invention.

It is also to be noted that the solvating form of the compound (I) (e.g. hydrate, etc) and any form of the crystal of the compound (I) are included within the scope of the present invention.

The object compound (I) or a salt thereof can be prepared according to the following reaction schemes.

Process 1

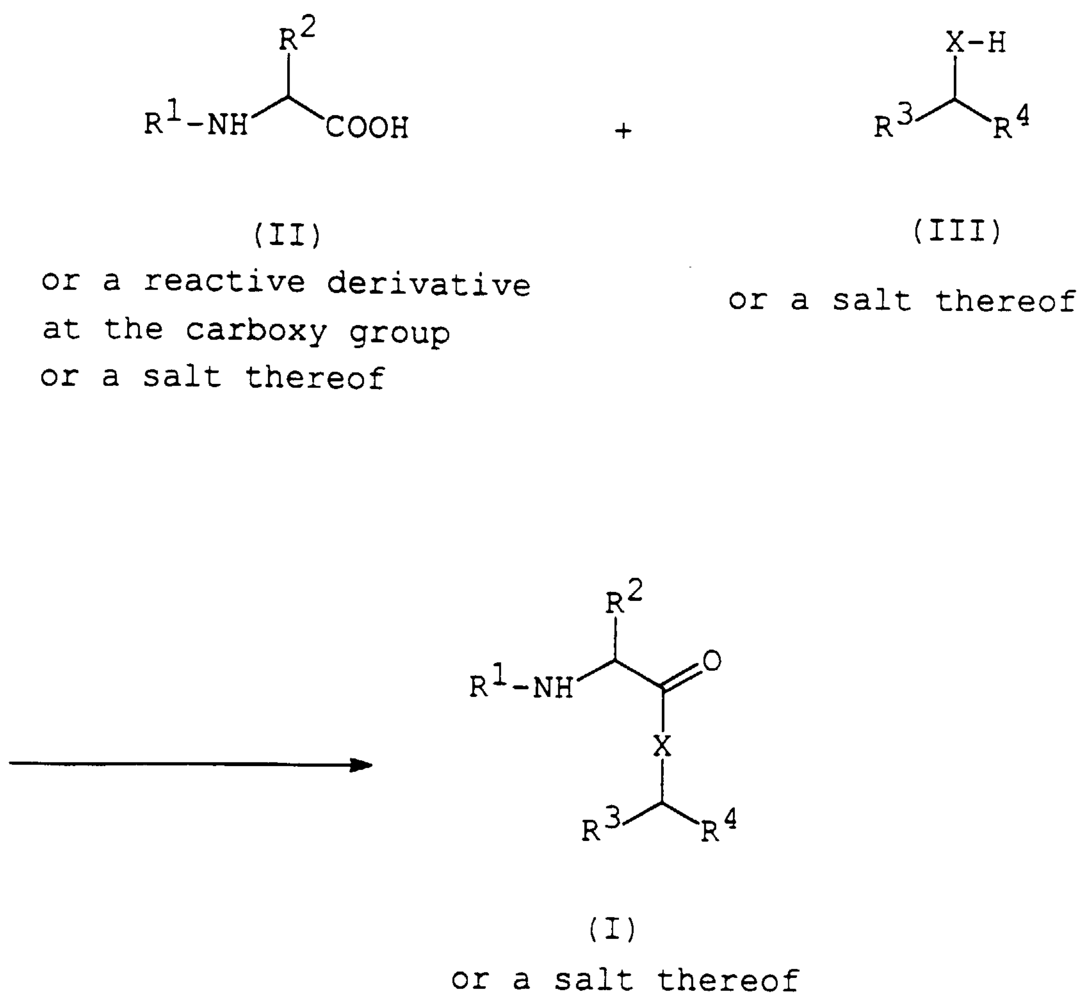

(II)
or a reactive derivative
at the carboxy group
or a salt thereof

+

(III)
or a salt thereof

(I)
or a salt thereof

Process 2

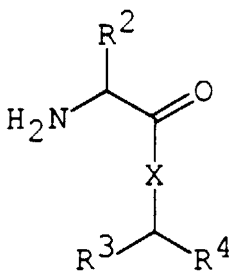

+

$R^1_a$-OH

(I)

or a reactive derivative at the amino group or a salt thereof

(V)

or a reactive derivative at the acyl group or a salt thereof

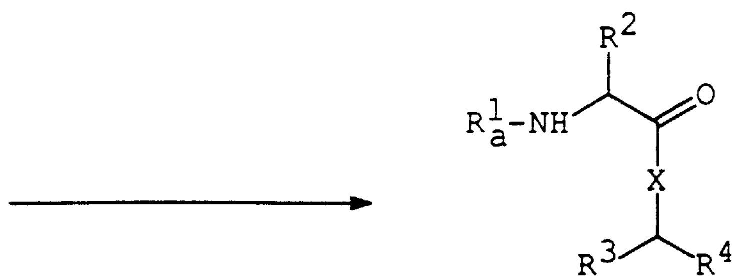

(I)

or a salt thereof

Process 3

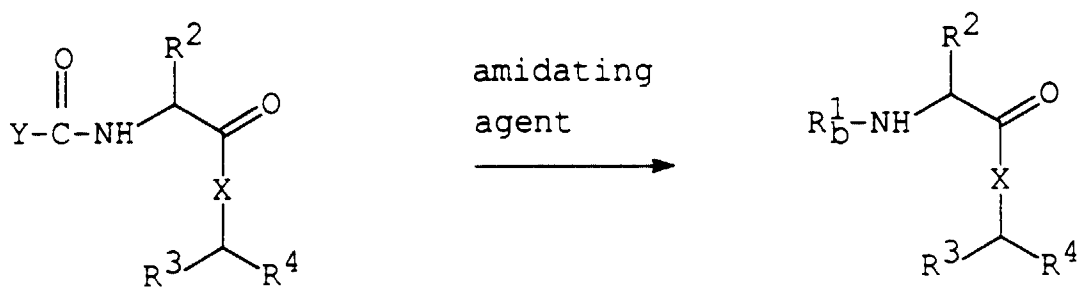


(VI)

or a salt thereof

(Ia)

or a salt thereof

Process 4

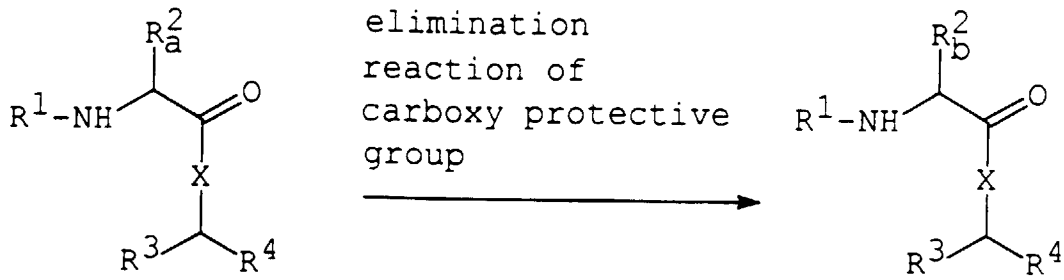


(Ib)
or a salt thereof

(Ic)
or a salt thereof

Process 5

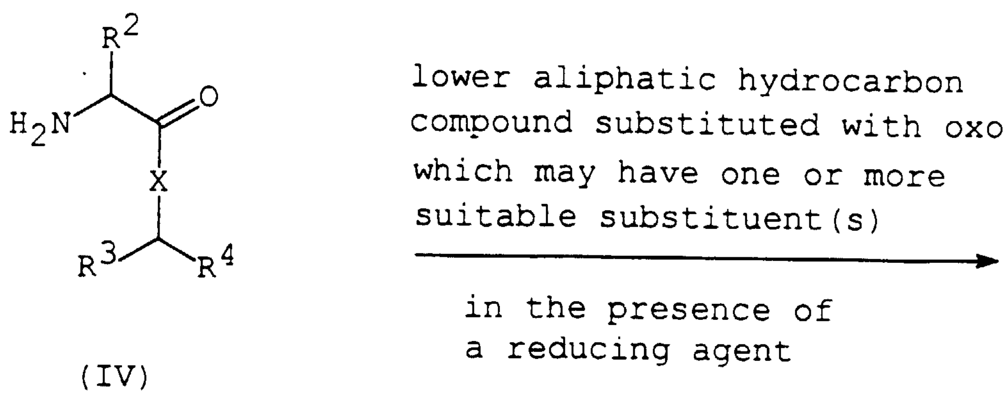


(IV)
or a reactive
derivative at the
amino group
or a salt thereof

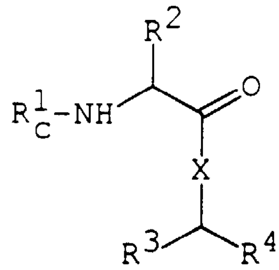

(Id)

or a salt thereof

Process 6

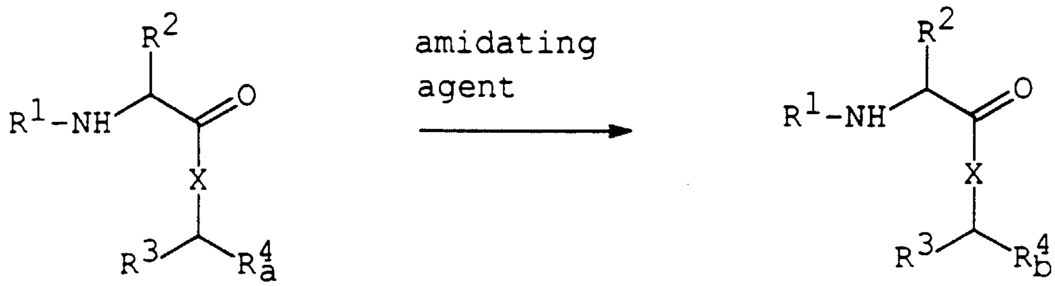


(Ie)
or a salt thereof

(If)
or a salt thereof

Process 7

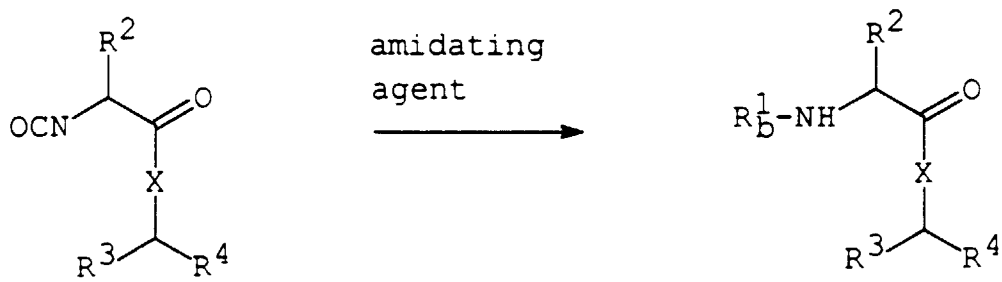


(VII)
or a salt thereof

(Ia)
or a salt thereof

wherein

| | |
|---|---|
| $R^1$, $R^2$, $R^3$, $R^4$ and X | are each as defined above, |
| $R^1_a$ | is acyl group, |
| $R^1_b$ | is amidated carboxy, |
| $R^1_c$ | is lower aliphatic hydrocarbon group which may have one or more suitable substituent(s), |
| $R^2_a$ | is protected carboxy(lower)alkyl, |
| $R^2_b$ | is carboxy(lower)alkyl, |
| $R^4_a$ | is carboxy or carboxy(lower)alkyl, |
| $R^4_b$ | is amidated carboxy or amidated carboxy(lower)alkyl, and |
| Y | is a leaving group. |

Suitable pharmaceutically acceptable salts of the object compound (I) are conventional ones and include a metal salt such as an alkali metal salt (e.g. sodium salt, potassium salt, etc) and an alkaline earth metal salt (e.g. calcium salt, magnesium salt, etc), an ammonium salt, an organic base salt (e.g. trimethylamine salt, triethylamine salt, pyridine salt, picoline salt, dicyclohexylamine salt, N,N'-dibenzylethylenediamine salt, etc), an organic acid salt (e.g. acetate, trifluoroacetate, maleate, tartrate, fumarate, methanesulfonate, benzenesulfonate, formate, toluenesulfonate, etc), an inorganic acid salt (e.g. hydrochloride, hydrobromide, hydriodide, sulfate, phosphate, etc), a salt with an amino acid (e.g. arginine, aspartic acid, glutamic acid, etc), and the like.

In the above and following descriptions of the present specification, suitable examples and illustrations of the various definitions which the present invention includes within the scope thereof are explained in detail as follows.

The term "lower" is intended to mean 1 to 6 carbon atom(s) unless otherwise indicated.

The term "higher" is intended to mean 7 to 20 carbon atoms unless otherwise indicated.

Suitable "lower aliphatic hydrocarbon group" in the term "lower aliphatic hydrocarbon group which may have one or more suitable substituent(s)" may include lower alkyl, lower alkenyl and lower alkynyl.

Suitable example of "lower alkyl" may include straight or branched one such as methyl, ethyl, propyl, isopropyl, butyl, t-butyl, pentyl, hexyl or the like, in which the preferred one may be $(C_1\text{-}C_4)$alkyl.

Suitable example of "lower alkenyl" may include straight or branched ones having 2 to 6 carbon atoms such as vinyl, allyl, 2-propenyl, 1,3-butadienyl, 3-methyl-2-butenyl, 4-pentenyl, 2-hexenyl, or the like, in which the preferred one may be $(C_2\text{-}C_4)$alkenyl.

Suitable example of "lower alkynyl" may include straight or branched ones having 2 to 6 carbon atoms such as ethynyl, 2-propynyl, 1,3-butadiynyl, 3-methyl-2-butynyl, 4-pentynyl, 2-hexynyl, or the like, in which the preferred one may be $(C_2\text{-}C_4)$alkynyl.

Said "lower aliphatic hydrocarbon group" may have one or more (preferably 1 to 3) suitable substituent(s) such as acyl group, heterocyclic group, acylamino, heterocyclic-amino, heterocyclic(lower)alkylamino, or the like, in which the preferred substituent may be heterocyclic group, the more preferred one may be unsaturated 3 to 8-membered heteromonocyclic group containing 1 to 4 nitrogen atom(s), and the most preferred one may be pyridyl.

Suitable "lower aliphatic hydrocarbon compound" moiety in the term "lower aliphatic hydrocarbon compound substituted with oxo" may be the corresponding hydrocarbon compound to the ones as exemplified for "lower aliphatic hydrocarbon group", and this "lower aliphatic hydrocarbon compound substituted with oxo" may have one or more (preferably 1 to 3) suitable substituent(s) as exemplified for those of "lower aliphatic hydrocarbon group".

Suitable "higher alkyl" may include straight or branched ones such as heptyl, 2-methylheptyl, octyl, nonyl, decyl, undecyl, dodecyl, tridecyl, 11-methyldodecyl, 12-methyltridecyl, tetradecyl, pentadecyl, hexadecyl, heptadecyl, octadecyl, nonadecyl, icosyl or the like, in which the preferred one may be $(C_7\text{-}C_{16})$alkyl, and the more preferred one may be heptyl, nonyl, undecyl, dodecyl, 11-methyldodecyl, tridecyl or 12-methyltridecyl.

Suitable "acyl group" may be aliphatic acyl, aromatic acyl, heterocyclic acyl, arylaliphatic acyl and heterocyclic-aliphatic acyl derived from carboxylic acid, carbonic acid, carbamic acid, sulfonic acid, and the like.

Suitable example of the "acyl group" thus explained may be :

(1) lower alkanoyl [e.g. formyl, acetyl, propionyl, butyryl, isobutyryl, valeryl, hexanoyl, pivaloyl, etc] which may have one or more (preferably 1 to 3) suitable substituent(s) such as halogen (e.g. fluoro, chloro, bromo, iodo); hydroxy; lower alkoxy (e.g. methoxy, ethoxy, propoxy, butoxy, t-butoxy, pentyloxy, hexyloxy, etc); amino; protected amino, preferably, acylamino such as lower alkoxycarbonylamino (e.g. methoxycarbonylamino, ethoxycarbonylamino, propoxycarbonylamino, butoxycarbonylamino, t-butoxycarbonylamino, pentyloxycarbonylamino, hexyloxycarbonylamino, etc); or the like; di(lower)alkylamino (e.g. dimethylamino, N-methylethylamino, diethylamino, N-propylbutylamino, dipentylamino, dihexylamino, etc); lower alkoxyimino (e.g. methoxyimino, ethoxyimino, propoxyimino, butoxyimino, t-butoxyimino, pentyloxyimino, hexyloxyimino, etc); ar(lower)alkoxyimino such as phenyl(lower) alkoxyimino (e.g. benzyloxyimino, phenethyloxyimino, benzhydryloxyimino, etc); or the like;

(2) higher alkanoyl [e.g. heptanoyl, octanoyl, nonanoyl, decanoyl, undecanoyl, lauroyl, tridecanoyl, myristoyl, pentadecanoyl, palmitoyl, 14-methylpentadecanoyl, 15-methylhexadecanoyl, 10, 12-dimethyltetradecanoyl, heptadecanoyl, stearoyl, nbnadecanoyl, icosanoyl, etc] which may have one or more (preferably 1 to 3) suitable substituent (s) as exemplified for those of "lower alkanoyl";

(3) lower alkenoyl [e.g. acryloyl, crotonoyl, isocrotonoyl, methacryloyl, 3-pentenoyl, 2,4-pentadienoyl, 5-hexenoyl, 2,4-hexadienoyl, etc] which may have one or more (preferably 1 to 3) suitable substituent(s) as exemplified for those of "lower alkanoyl";

(4) higher alkenoyl [e.g. 4-heptenoyl, 3-octenoyl, 3,6-decadienoyl, 3,7,11-trimethyl-2,6,10-dodecatrienoyl, 4,10-heptadecadienoyl, etc] which may have one or more (preferably 1 to 3) suitable substituent(s) as exemplified for those of "lower alkanoyl";

(5) protected carboxy, in which the preferred one may be esterified carboxy such as lower alkoxycarbonyl [e.g. methoxycarbonyl, ethoxycarbonyl, propoxycarbonyl, butoxycarbonyl, t-butoxycarbonyl, pentyloxycarbonyl, hexyloxycarbonyl, etc],

halo(lower)alkoxycarbonyl[e.g. (chloromethoxy)carbonyl, (2,2,2-trichloroethoxy)carbonyl, (2,2,2-trifluoroethoxy)carbonyl, (2-chloropropoxy)carbonyl, (1-fluoro-4-bromobutoxy) carbonyl, (4-chloropentyloxy)-carbonyl, (6-chlorohexyloxy)carbonyl, etc],

higher alkoxycarbonyl [e.g. heptyloxycarbonyl, octyloxycarbonyl, 2-ethylhexyloxycarbonyl, nonyloxycarbonyl, decyloxycarbonyl, 3,7-dimethyloctyloxycarbonyl, undecyloxycarbonyl, dodecyloxycarbonyl, tridecyloxycarbonyl, tetradecyloxycarbonyl, pentadecyloxycarbonyl, 3-methyl-10-ethyldodecyloxycarbonyl, hexadecyloxycarbonyl, heptadecyloxycarbonyl, octadecyloxycarbonyl, nonadecyloxycarbonyl, icosyloxycarbonyl, etc],

aryloxycarbonyl [e.g. phenoxycarbonyl, naphthyloxycarbonyl, etc],

ar(lower)alkoxycarbonyl which may have one or more (preferably 1 to 3) suitable substituent(s) such as phenyl (lower)alkoxycarbonyl which may have nitro or lower alkoxy [e.g. benzyloxycarbonyl, phenethyloxycarbonyl, p-nitrobenzyloxycarbonyl, p-methoxybenzyloxycarbonyl, etc], or the like;

(6) carboxy;

(7) lower alkylsulfonyl [e.g. methylsulfonyl, ethylsulfonyl, propylsulfonyl, isopropylsulfonyl, pentylsulfonyl, butylsulfonyl, etc];

(8) arylsulfonyl [e.g. phenylsulfonyl, 1-(or 2-)-naphthylsulfonyl, etc] which may have one or more (preferably 1 to 3) suitable substituent(s) such as lower alkyl, di(lower)alkylamino, lower alkylamino (e.g. methylamino, ethylamino, propylamino, butylamino, t-butylamino, pentylamino, hexylamino, etc), or the like;

(9) ar(lower)alkylsulfonyl such as phenyl(lower)alkylsulfonyl [e.g. benzylsulfonyl, phenethylsulfonyl, benzhydrylsulfonyl, etc], or the like;

(10) aryl(lower)alkanoyl [e.g. benzoyl, naphthoyl (e.g. 1-naphthoyl, 2-naphthoyl, etc), anthrylcarbonyl, 2-phenylacetyl, 2-phenylpropionyl, 4-(1-naphthyl)butyryl, 3-phenylvaleryl, 2,5-diphenylhexanoyl, etc] which may have one or more (preferably 1 to 3) suitable substituent(s) such as lower alkoxy, aryl (e.g. phenyl, naphthyl, anthryl, etc), or the like;

(11) aryl(lower)alkenoyl (e.g. 3-phenylacryloyl, 2-phenylacryloyl, 2-naphthylacryloyl, 3-phenylcrotonoyl, 4-phenylisocrotonoyl, 2-benzylacryloyl, 5-phenyl-3-pentenoyl, 3-naphthyl-2,4-pentadienoyl, 2-phenyl-5-hexenoyl, 6-phenyl-2,4-hexadienoyl, etc);

(12) heterocyclic(lower)alkanoyl which may have one or more (preferably 1 to 3) suitable substituent(s) such as lower alkyl, acyl, ar(lower)alkyl (e.g. benzyl, phenethyl, etc) which may have lower alkoxy, lower alkylenedioxy (e.g. methylenedioxy, etc) or the like;

(13) heterocyclicsulfonyl;

(14) amidated carboxy such as carbamoyl,

N-heterocyclic-carbamoyl which may have one or more (preferably 1 to 3) suitable substituent(s) such as lower alkyl, halogen, or the like,

N-lower alkyl-N-heterocyclic-carbamoyl,

N-lower alkylcarbamoyl (e.g. N-methylcarbamoyl, N-ethylcarbamoyl, N-propylcarbamoyl, N-butylcarbamoyl, N-t-butylcarbamoyl, N-pentylcarbamoyl, N-hexylcarbamoyl, etc) which may have one or more (preferably 1 to 3) suitable substituent(s) such as heterocyclic group, hydroxy, or the like,

N-ar(lower)alkylcarbamoyl such as N-(mono- or di- or tri-)phenyl(lower)alkylcarbamoyl (e.g. N-benzylcarbamoyl, N-phenethylcarbamoyl, N-benzhydrylcarbamoyl, N-tritylcarbamoyl, etc), or the like; or the like.

Suitable "heterocyclic group" and "heterocyclic" moiety in the terms "heterocyclic-amino", "heterocyclic(lower) alkylamino", "heterocyclic(lower)alkanoyl", "heterocyclicsulfonyl", "N-heterocyclic-carbamoyl", and "N-lower alkyl-N-heterocyclic-carbamoyl" may include saturated or unsaturated, monocyclic or polycyclic heterocyclic group such as

unsaturated 3 to 8-membered (more preferably 5 to 7-membered) heteromonocyclic group containing 1 to 4 nitrogen atom(s), for example, azepinyl (e.g. 1H-azepinyl, etc), pyrrolyl, pyrrolinyl, imidazolyl, pyrazolyl, pyridyl and its N-oxide, dihydropyridyl, pyrimidinyl, pyrazinyl, pyridazinyl, triazolyl (e.g. 4H-1,2,4-triazolyl, 1H-1,2,3-triazolyl, 2H-1,2,3-triazolyl, etc), tetrazolyl (e.g. 1H-tetrazolyl, 2H-tetrazolyl, etc) etc;
saturated 3 to 8-membered (more preferably 5 to 7 membered) heteromonocyclic group containing 1 to 4 nitrogen atom(s), for example, perhydroazepinyl (e.g. perhydro-1H-azepinyl, etc), pyrrolidinyl, imidazolidinyl, piperidyl, piperazinyl, etc;
unsaturated condensed heterocyclic group containing 1 to 4 nitrogen atom(s), for example, indolyl, isoindolyl, indolizinyl, benzimidazolyl, quinolyl, isoquinolyl, indazolyl, benzotriazolyl, quinoxalinyl, imidazopyridyl [e.g. imidazo [4,5-c]pyridyl, etc], tetrahydroimidazopyridyl [e.g. 4,5,6,7-tetrahydro[4,5-c]pyridyl, etc], etc;
saturated condensed heterocyclic group containing 1 to 4 nitrogen atom(s), for example, 7-azabicyclo[2.2.1]-heptyl, 3-azabicyclo[3.2.2]nonanyl, etc;
unsaturated 3 to 8-membered (more preferably 5 or 6-membered) heteromonocyclic group containing 1 to 2 oxygen atom(s) and 1 to 3 nitrogen atom(s), for example, oxazolyl, isoxazolyl, oxadiazolyl (e.g. 1,2,4-oxadiazolyl, 1,3,4-oxadiazolyl, 1,2,5-oxadiazolyl, etc), etc;
saturated 3 to 8-membered (more preferably 5 or 6-membered) heteromonocyclic group containing 1 to 2 oxygen atom(s) and 1 to 3 nitrogen atom(s), for example, morpholinyl, sydnonyl, etc;
unsaturated condensed heterocyclic group containing 1 to 2 oxygen atom(s) and 1 to 3 nitrogen atom(s), for example, benzoxazolyl, benzoxadiazolyl, etc;
unsaturated 3 to 8-membered (more preferably 5 or 6-membered) heteromonocyclic group containing 1 to 2 sulfur atom(s) and 1 to 3 nitrogen atom(s), for example, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc), dihydrothiazinyl, etc;
saturated 3 to 8-membered (more preferably 5 or 6-membered) heteromonocyclic group containing 1 to 2 sulfur atom(s) and 1 to 3 nitrogen atom(s), for example, thiazolidinyl, etc;
unsaturated condensed heterocyclic group containing 1 to 2 sulfur atom(s) and 1 to 3 nitrogen atom(s), for example, benzothiazolyl, benzothiadiazolyl, etc;
saturated condensed heterocyclic group containing 1 to 4 nitrogen atom(s), and
saturated 3 to 8-membered heteromonocyclic group containing 1 to 2 oxygen atom(s) and 1 to 3 nitrogen atom(s),
unsaturated 3 to 8-membered (more preferably 5 or 6-membered) heteromonocyclic group containing an oxygen atom, for example, furyl, etc;
unsaturated 3 to 8-membered (more preferably 5 or 6-membered) heteromonocyclic group containing an oxygen atom and 1 to 2 sulfur atom(s), for example, dihydrooxathiinyl, etc;
unsaturated condensed heterocyclic group containing 1 to 2 sulfur atom(s), for example, benzothienyl, benzodithiinyl, etc;
unsaturated condensed heterocyclic group containing an oxygen atom and 1 to 2 sulfur atom(s), for example, benzoxathiinyl, etc; or the like.

In aforesaid "acyl group", the preferred one may be

(1) lower alkoxycarbonyl, in which the more preferred one may be ($C_1$-$C_4$)alkoxycarbonyl, and the most preferred one may be t-butoxycarbonyl;
(2) lower alkenoyl, in which the more preferred one may be ($C_3$-$C_6$)alkenoyl, and the most preferred one may be crotonoyl, isocrotonoyl or 2,4-hexadienyl;
(3) aryl(lower)alkanoyl which may have one or more suitable substituent(s), in which the more preferred one may be phenyl(lower)alkanoyl or naphthyl(lower)alkanoyl, each of which may have 1 to 3 lower alkoxy or phenyl, the much more preferred one may be phenyl($C_1$-$C_4$)alkanoyl which may have ($C_1$-$C_4$)alkoxy or phenyl, or naphthyl ($C_1$-$C_4$)alkanoyl, the most preferred one may be benzoyl, 4-methoxybenzoyl, 2-biphenylcarbonyl, or 1-naphthoyl;
(4) aryl(lower)alkenoyl, in which the more preferred one may be phenyl($C_2$-$C_5$)alkenoyl, and the most preferred one may be 3-phenylacryloyl;
(5) aryloxycarbonyl, in which the more preferred one may be phenoxycarbonyl;
(6) arylsulfonyl which may have one or more suitable substituent(s), in which the more preferred one may be naphthylsulfonyl which may have 1 to 3 di(lower)alkylamino, the much more preferred one may be naphthylsulfonyl which may have di($C_1$-$C_4$)alkylamino, and the most preferred one may be 5-dimethylamino-1-naphthylsulfonyl;
(7) heterocyclic(lower)alkanoyl which may have one or more suitable substituent(s), in which the more preferred one may be

(i) heterocyclic(lower)alkanoyl, wherein heterocyclic moiety is unsaturated 3 to 8-membered heteromonocyclic group containing 1 to 4 nitrogen atom(s), which may have 1 to 3 lower alkyl, the more preferred one may be

pyridyl($C_1$-$C_4$)-alkanoyl which may have ($C_1$-$C_4$)alkyl, pyrazinyl($C_1$-$C_4$)-alkanoyl, or imidazolyl($C_1$-$C_4$)alkanoyl, the much more preferred one may be pyridylcarbonyl which may have ($C_1$-$C_4$)alkyl, 2-(pyridyl)acetyl, pyrazinylcarbonyl, or 2-(imidazolyl)-acetyl, and the most preferred one may be 2-(or 3-or 4-)-pyridylcarbonyl, (5-methyl-2-pyridyl)carbonyl, 2-(2-pyridyl)-acetyl, 2-pyrazinylcarbonyl, and 2-(2-imidazolyl)acetyl;

(ii) heterocyclic(lower)alkanoyl, wherein heterocyclic moiety is unsaturated condensed heterocyclic group containing 1 to 4 nitrogen atom(s), which may have 1 to 3 lower alkoxycarbonyl, the more preferred one may be quinolyl ($C_1$-$C_4$)alkanoyl, isoquinolyl($C_1$-$C_4$)alkanoyl, quinoxalinyl-($C_1$-$C_4$)alkanoyl, or tetrahydroimidazopyridyl($C_1$-$C_4$)alkanoyl, the much more preferred one may be quinolylcarbonyl, isoquinolylcarbonyl, quinoxalinylcarbonyl, tetrahydroimidazopyridylcarbonyl which may have 1 to 3 ($C_1$-$C_4$)alkoxycarbonyl, the most preferred one may be 2-(or 3-or 5-)quinolylcarbonyl, 1-(or 3-)-isoquinolylcarbonyl, 2-quinoxalinylcarbonyl, 4,5,6,7-tetrahydro-3H-imidazo[4,5-c]pyridin-6-ylcarbonyl, and 3-t-butoxycarbonyl-5-t-butoxycarbonyl-4,5,6,7-tetrahydro-3H-imidazo[4,5-c]pyridin-6-ylcarbonyl;

(iii) heterocyclic(lower)alkanoyl, wherein heterocyclic moiety is saturated 3 to 8-membered heteromonocyclic group containing 1 to 4 nitrogen atom(s), which may have 1 to 3 lower alkoxycarbonyl or phenyl(lower)alkyl which may have lower alkylenedioxy, the more preferred one may be pyrrolidinyl($C_1$-$C_4$)alkanoyl which may have 1 to 3 ($C_1$-$C_4$)-alkoxycarbonyl, piperidyl($C_1$-$C_4$)alkanoyl which may have 1 to 3 ($C_1$-$C_4$)alkoxycarbonyl, or piperazinyl which may have 1 to 3 phenyl($C_1$-$C_4$)alkyl having ($C_1$-$C_4$)alkylenedioxy, and the most preferred one may be 1-t-butoxycarbonyl-2-pyrrolidinylcarbonyl, 1-(or 2- or 4-)piperidylcarbonyl, and 1-t-butoxycarbonyl-2-(or 4-)piperidylcarbonyl;

(iv) heterocyclic(lower)alkanoyl, wherein heterocyclic moiety is saturated 3 to 8-membered heteromonocyclic group containing 1 to 2 oxygen atom(s) and 1 to 3 nitrogen atom(s), the more preferred one may be morpholinyl ($C_1$-$C_4$)alkanoyl, the much more preferred one may be morpholinylcarbonyl, and the most preferred one may be morpholinocarbonyl; or the like;

(8) heterocyclicsulfonyl, in which the more preferred one may be heterocyclicsulfonyl, wherein heterocyclic moiety is unsaturated condensed heterocyclic group containing 1 to 4 nitrogen atom(s), the much more preferred one may be quinolylsulfonyl, and the most preferred one may be 8-quinolylsulfonyl;

(9) carbamoyl;

(10) N-heterocyclic-carbamoyl which may have one or more suitable substituent(s), in which the more preferred one may be

(i) N-heterocycliccarbamoyl, wherein heterocyclic moiety is unsaturated 3 to 8-membered heteromonocyclic group containing 1 to 4 nitrogen atom(s), which may have 1 to 3 lower alkyl or halogen, the much more preferred one may be N-pyridylcarbamoyl which may have 1 to 3 ($C_1$-$C_4$)alkyl or halogen, and the most preferred one may be N-(2-pyridyl)carbamoyl, N-(5-methyl-2-pyridyl)carbamoyl, and N-(5-chloro-2-pyridyl)carbamoyl;

(ii) N-heterocyclic-carbamoyl, wherein heterocyclic moiety is unsaturated condensed heterocyclic group containing 1 to 4 nitrogen atoms, the more preferred one may be N-quinolylcarbamoyl, and the most preferred one may be N- [2- (or 3-)quinolyl]carbamoyl;

(iii) N-heterocyclic-carbamoyl, wherein heterocyclic moiety is unsaturated condensed heterocyclic group containing 1 to 2 oxygen atom(s) and 1 to 3 nitrogen atom(s), which may have 1 to 3 halogen, the more preferred one may be N-benzoxazolylcarbamoyl which may have 1 to 3 halogen, and the most preferred one may be N-(5-chloro-2-benzoxazolyl)carbamoyl;

(iv) N-heterocyclic-carbamoyl, wherein heterocyclic moiety is unsaturated condensed heterocyclic group containing 1 to 2 sulfur atom(s) and 1 to 3 nitrogen atom(s), the more preferred one may be N-benzothiazolylcarbamoyl, and the most preferred one may be N-(2-benzothiazolyl)carbamoyl;

(v) N-heterocyclic-carbamoyl, wherein heterocyclic moiety is saturated 3 to 8-membered heteromonocyclic group containing 1 to 2 oxygen atom(s) and 1 to 3 nitrogen atom(s), the more preferred one may be N-morpholinylcarbamoyl, and the most preferred one may be N-morpholinocarbamoyl;

(11) N-lower alkyl-N-heterocyclic-carbamoyl, in which the more preferred one may be N-lower alkyl-N-heterocyclic-carbamoyl, wherein heterocyclic moiety is unsaturated 3 to 8-membered heteromonocyclic group containing 1 to 4 nitrogen atom(s), the much more preferred one may be N-($C_1$-$C_4$)alkyl-N-pyridylcarbamoyl, and the most preferred one may be N-methyl-N-(2-pyridyl)carbamoyl;

(12) N-heterocyclic(lower)alkylcarbamoyl, in which the more preferred one may be N-heterocyclic(lower)alkylcarbamoyl, wherein heterocyclic moiety is unsaturated 3 to 8-membered heteromonocyclic group containing 1 to 4 nitrogen atom(s), the much more preferred one may be N-pyridyl($C_1$-$C_4$)-alkylcarbamoyl, and the most preferred one may be (2-pyridyl)methylcarbamoyl;

(13) N-lower alkylcarbamoyl which may have one or more hydroxy, in which the more preferred one may be N-

($C_1$-$C_4$)-alkylcarbamoyl which may have 1 to 3 hydroxy, and the most preferred one may be N-(2-hydroxypropyl) carbamoyl;

(14) higher alkanoyl which may have one or more suitable substituent(s), in which the more preferred one may be higher alkanoyl which may have 1 to 3 hydroxy, the much more preferred one may be ($C_{10}$-$C_{18}$)alkanoyl which may have hydroxy, and the most preferred one may be 3-hydroxy-15-methylhexadecanoyl.

Suitable "lower alkanoyl" moiety in the term "heterocyclic(lower)alkanoyl" can be referred to the ones as exemplified for "lower alkanoyl".

Suitable "lower alkyl" moiety in the term "N-heterocyclic(lower)alkylcarbamoyl" can be referred to the ones as exemplified for "lower alkyl".

Suitable example of "acyl" moiety in the term "acyl(lower)alkyl" may be the ones as exemplified for "acyl group".

The preferred "acyl" moiety in the term "acyl(lower)alkyl" may be carboxy;

protected carboxy, in which the more preferred one may be lower alkoxycarbonyl, halo(lower)alkoxycarbonyl, or ar (lower)alkoxycarbonyl, the much more preferred one may be ($C_1$-$C_4$)alkoxycarbonyl, halo($C_1$-$C_4$)alkoxycarbonyl or phenyl-($C_1$-$C_4$)alkoxycarbonyl, and the most preferred one may be methoxycarbonyl, 2,2,2-trichloroethoxycarbonyl or benzyloxycarbonyl;

amidated carboxy, in which the more preferred one may be carbamoyl, N-lower alkylcarbamoyl, or N-ar(lower)alkylcarbamoyl, the much more preferred one may be carbamoyl, N-($C_1$-$C_4$)alkylcarbamoyl, or N-phenyl($C_1$-$C_4$)-alkylcarbamoyl, and the most preferred one may be carbamoyl, N-methylcarbamoyl, or N-benzylcarbamoyl.

Suitable example of "lower alkyl" moiety in the terms "acyl (lower)alkyl", "protected carboxy(lower)alkyl", "carboxy(lower)alkyl", "amidated carboxy(lower)alkyl", and "cyano(lower)alkyl", in which the preferred one may be ($C_1$-$C_4$) alkyl.

Suitable "protected carboxy" moiety in the term of "protected carboxy(lower)alkyl" can be referred to the ones as exemplified for "protected carboxy".

Suitable "amidated carboxy" moiety in the term of "amidated carboxy(lower)alkyl" can be referred to the ones as exemplified for "amidated carboxy".

Suitable "a leaving group" may include halogen, lower alkoxy, aryloxy (e.g. phenoxy, etc), and the like.

Suitable "amidating agent" may be the amino compound corresponding to the object "amidated carboxy", and the concrete example thereof may be ammonia or its chemical equivalent, heterocyclic-amine, N-lower alkyl-heterocyclic-amine, lower alkylamine which may have one or more (preferably 1 to 3) suitable substituent(s), ar(lower) alkylamine, or the like.

Suitable "heterocyclic" moiety and "lower alkyl" moiety in these compounds can be referred to the ones as exemplified for "heterocyclic group" and "lower alkyl", respectively.

Suitable examples of "suitable substituent(s)" in the term "lower alkylamine which may have one or more suitable substituent(s)" can be referred to the ones as exemplified for the "suitable substituent(s)" of "N-lower alkylcarbamoyl which may have one or more suitable substituent(s)".

In the following, some of the preferred embodiments of the fatty acid derivative (I) of the present invention are shown.

(1) the derivative (I), wherein

$R^1$ is lower alkoxycarbonyl,
$R^2$ is carboxy(lower)alkyl,
$R^3$ is higher alkyl,
$R^4$ is carbamoyl(lower)alkyl, and
X is -O- .

(2) the derivative (I), wherein

$R^1$ is lower alkenoyl, and
$R^2$, $R^3$, $R^4$ and X are each as defined above in (1).

(3) the derivative (I), wherein

| | |
|---|---|
| $R^1$ | is phenyl(lower)alkanoyl or naphthyl(lower)alkanoyl, each of which may have 1 to 3 lower alkoxy or phenyl, and |
| $R^2$, $R^3$, $R^4$ and X | are each as defined above in (1). |

(4) the derivative (I), wherein

| | |
|---|---|
| $R^1$ | is phenyl(lower)alkenoyl, and |
| $R^2$, $R^3$, $R^4$ and X | are each as defined above in (1). |

(5) the derivative (I), wherein

| | |
|---|---|
| $R^1$ | is phenoxycarbonyl, and |
| $R^2$, $R^3$, $R^4$ and X | are each as defined above in (1). |

(6) the derivative (I), wherein

| | |
|---|---|
| $R^1$ | is naphthylsulfonyl which may have 1 to 3 di(lower)alkylamino, and |
| $R^2$, $R^3$, $R^4$ and X | are each as defined above in (1). |

(7) the derivative (I), wherein

| | |
|---|---|
| $R^1$ | is heterocyclic(lower)alkanoyl, wherein heterocyclic moiety is unsaturated 3 to 8-membered heteromonocyclic group containing 1 to 4 nitrogen atom(s), which may have 1 to 3 lower alkyl [the more preferred one may be pyridyl(lower)alkanoyl which may have (lower)alkyl, pyrazinyl(lower)alkanoyl, or imidazolyl(lower)alkanoyl], and |
| $R^2$, $R^3$, $R^4$ and X | are each as defined above in (1). |

(8) the derivative (I), wherein

| | |
|---|---|
| $R^1$ | is heterocyclic(lower)alkanoyl, wherein heterocyclic moiety is unsaturated condensed heterocyclic group containing 1 to 4 nitrogen atom(s), which may have 1 to 3 lower alkoxycarbonyl [the more preferred one may be quinolyl(lower) alkanoyl, isoquinolyl (lower)alkanoyl, quinoxalinyl (lower) alkanoyl, or tetrahydroimidazopyridyl(lower)alkanoyl], and |
| $R^2$, $R^3$, $R^4$ and X | are each as defined above in (1). |

(9) the derivative (I), wherein

| | |
|---|---|
| $R^1$ | is heterocyclic(lower)alkanoyl, wherein heterocyclic moiety is saturated 3 to 8-membered heteromonocyclic group containing 1 to 4 nitrogen atom(s), which may have 1 to 3 lower alkoxycarbonyl or phenyl(lower)alkyl which may have lower alkylenedioxy [the more preferred one may be pyrrolidinyl(lower)alkanoyl which may have 1 to 3 (lower)alkoxycarbonyl, piperidyl(lower)alkanoyl which may have 1 to 3 (lower)alkoxycarbonyl; or piperazinyl(lower)alkyl which may have 1 to 3 phenyl(lower)alkyl having (lower)alkylenedioxy], and |
| $R^2$, $R^3$, $R^4$ and X | are each as defined above in (1). |

(10) the derivative (I), wherein

| | |
|---|---|
| $R^1$ | is heterocyclic(lower)alkanoyl, wherein heterocyclic moiety is saturated 3 to 8-membered heteromonocyclic group containing 1 to 2 oxygen atom(s) and 1 to 3 nitrogen atom(s) [the more preferred one may be morpholinyl(lower)alkanoyl], and |
| $R^2$, $R^3$, $R^4$ and X | are each as defined above in (1). |

(11) the derivative (I), wherein

| | |
|---|---|
| $R^1$ | is heterocyclicsulfonyl, wherein heterocyclic moiety is unsaturated condensed heterocyclic group containing 1 to 4 nitrogen atom(s) [the much more preferred one may be quinolylsulfonyl], and |
| $R^2$, $R^3$, $R^4$ and X | are each as defined above in (1). |

(12) the derivative (I), wherein

| | |
|---|---|
| $R^1$ | is N-heterocyclic-carbamoyl, wherein heterocyclic moiety is unsaturated 3 to 8-membered heteromonocyclic group containing 1 to 4 nitrogen atom(s), which may have 1 to 3 lower alkyl or halogen [the more preferred one may be N-pyridylcarbamoyl which may have 1 to 3 (lower)alkyl or halogen], and |
| $R^2$, $R^3$, $R^4$ and X | are each as defined above in (1). |

(13) the derivative (I), wherein

| | |
|---|---|
| $R^1$ | is N-heterocyclic-carbamoyl, wherein heterocyclic moiety is unsaturated condensed heterocyclic group containing 1 to 4 nitrogen atoms [the more preferred one may be N-quinolylcarbamoyl], and |
| $R^2$, $R^3$, $R^4$ and X | are each as defined above in (1). |

(14) the derivative (I), wherein

| | |
|---|---|
| $R^1$ | is N-heterocyclic-carbamoyl, wherein heterocyclic moiety is unsaturated condensed heterocyclic group containing 1 to 2 oxygen atom(s) and 1 to 3 nitrogen atom(s), which may have 1 to 3 halogen, [the more preferred one may be N-benzoxazolylcarbamoyl which may have 1 to 3 halogen], and |
| $R^2$, $R^3$, $R^4$ and X | are each as defined above in (1). |

(15) the derivative (I), wherein

| | |
|---|---|
| $R^1$ | is N-heterocyclic-carbamoyl, wherein heterocyclic moiety is unsaturated condensed heterocyclic group containing 1 to 2 sulfur atom(s) and 1 to 3 nitrogen atom(s) [the more preferred one may be N-benzothiazolylcarbamoyl] and |
| $R^2$, $R^3$, $R^4$ and X | are each as defined above in (1). |

(16) the derivative (I), wherein

| | |
|---|---|
| $R^1$ | is N-heterocyclic-carbamoyl, wherein heterocyclic moiety is saturated 3 to 8-membered heteromonocyclic group containing 1 to 2 oxygen atom(s) and 1 to 3 nitrogen atom(s) [the more preferred one may be N-morpholinylcarbamoyl], and |
| $R^2$, $R^3$, $R^4$ and X | are each as defined above in (1). |

(17) the derivative (I), wherein

| | |
|---|---|
| $R^1$ | is N-lower alkyl-N-heterocyclic-carbamoyl, wherein heterocyclic moiety is unsaturated 3 to 8-membered heteromonocyclic group containing 1 to 4 nitrogen atom(s) [the more preferred one may be N-(lower)alkyl-N-pyridylcarbamoyl], and |
| $R^2$, $R^3$, $R^4$ and X | are each as defined above in (1). |

(18) the derivative (I), wherein

$R^1$ is N-heterocyclic(lower)alkylcarbamoyl, wherein heterocyclic moiety is unsaturated 3 to 8-membered heteromonocyclic group containing 1 to 4 nitrogen atom(s) [the more preferred one may be N-pyridyl(lower) alkylcarbamoyl], and

(19) the derivative (I), wherein

| | |
|---|---|
| $R^1$ | is N-lower alkylcarbamoyl which may have 1 to 3 hydroxy, and |
| $R^2$, $R^3$, $R^4$ and X | are each as defined above in (1). |

(20) the derivative (I), wherein

| | |
|---|---|
| $R^1$ | is higher alkanoyl which may have 1 to 3 hydroxy, and |

$R^2$, $R^3$, $R^4$ and X are each as defined above in (1).

The processes for preparing the object compound (I) of the present invention are explained in detail in the following.

Process 1

The compound (I) or a salt thereof can be prepared by reacting the compound (II) or a reactive derivative at the carboxy group or a salt thereof with the compound (III) or a salt thereof.
Suitable salts of the compounds (II) and (III) can be referred to the ones as exemplified for the compound (I).
Suitable reactive derivative at the carboxy group of the compound (II) may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc], dialkylphosphorous acid, sulfurous acid, thiosulfuric acid, sulfuric acid, sulfonic acid [e.g. methanesulfonic acid, etc], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc] or aromatic carboxylic acid [e.g. benzoic acid, etc]; a symmetrical acid anhydride; an activated amide with imidazole, 4-substituted imidazole, dimethylpyrazole, triazole, tetrazole or 1-hydroxy-1H-benzotriazole; or an activated ester [e.g. cyanomethyl ester, methoxymethyl ester, dimethyliminomethyl [$(CH_3)_2N=CH-$] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc] or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole, etc], and the like. These reactive derivatives can optionally be selected from them according to the kind of the compound (II) to be used.
In the case that the group X is

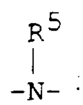

in the compound (III), the compound (III) can be used in the form of its reactive derivative at the amino group.
Suitable said reactive derivative at the amino group may include Schiff's base type imino or its tautomeric enamine type isomer formed by the reaction of the compound (III) with a carbonyl compound such as aldehyde, ketone or the like; a silyl derivative formed by the reaction of the compound (III) with a silyl compound such as bis(trimethylsilyl) acetamide, mono(trimethylsilyl)acetamide, bis(trimethylsilyl)urea or the like; a derivative formed by reaction of the compound (III) with phosphorus trichloride or phosgene, and the like.
The reaction is usually carried out in a conventional solvent such as water, alcohol [e.g. methanol, ethanol, etc], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.
In this reaction, when the compound (II) is used in a free acid form or its salt form, the reaction is preferably carried out in the presence of a conventional condensing agent such as N,N'-dicyclohexylcarbodiimide; 1-ethyl-3-(3-dimethylaminopropyl)carbodiimide, or the like.
The reaction may also be carried out in the presence of an inorganic or organic base such as an alkali metal carbonate, alkali metal bicarbonate, tri(lower)alkylamine (e.g. triethylamine, etc), pyridine, di(lower)alkylaminopyridine, N-(lower)alkylmorpholine, N,N-di(lower)alkylbenzylamine, or the like.
The reaction temperature is not critical, and the reaction is usually carried out under cooling to warming.

Process 2

The compound (I) or a salt thereof can be prepared by reacting the compound (IV) or a reactive derivative at the amino group or a salt thereof with the compound (V) or a reactive derivative at the acyl group or a salt thereof.
Suitable salts of the compounds (IV) and (V) can be referred to the ones as exemplified for the compound (I).
The reaction of this process can be carried out according to a similar manner to that of Process 1, and so the reaction condition can be referred to the explanation therein.

Process 3

The compound (Ia) or a salt thereof can be prepared by reacting the compound (VI) or a salt thereof with amidating agent.
Suitable salts of the compounds (Ia) and (VI) can be referred to the ones as exemplified for the compound (I).
The reaction of this process can be carried out according to the manner disclosed in Examples or a similar manner thereto.

Process 4

The compound (Ic) or a salt thereof can be prepared by subjecting a compound (Ib) or a salt thereof to elimination reaction of carboxy protective group.
This reaction is carried out in accordance with a conventional method such as hydrolysis, reduction or the like.
The hydrolysis is preferably carried out in the presence of a base or an acid including Lewis acid.
Suitable base may include an inorganic base and an organic base such as an alkali metal [e.g. sodium, potassium, etc], an alkaline earth metal [e.g. magnesium, calcium, etc], the hydroxide or carbonate or bicarbonate thereof, trialkylamine [e.g. trimethylamine, triethylamine, etc], picoline, 1,5-diazabicyclo[4.3.0]non-5-ene, 1,4-diazabicyclo[2.2.2]octane, 1,8-diazabicyclo[5.4.0]undec-7-ene, or the like.
Suitable acid may include an organic acid [e.g. formic acid, acetic acid, propionic acid, trichloroacetic acid, trifluoroacetic acid, etc] and an inorganic acid [e.g. hydrochloric acid, hydrobromic acid, sulfuric acid, hydrogen chloride, hydrogen bromide, etc].
The elimination using Lewis acid such as trihaloacetic acid [e.g. trichloroacetic-acid, trifluoroacetic acid, etc] aluminium halide (e.g. aluminium chloride, etc) or the like is preferably carried out in the presence of cation trapping agents [e.g. anisole, phenol, etc].
The reaction is usually carried out in a solvent such as water, an alcohol [e.g. methanol, ethanol, etc], nitromethane, methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.
The reaction method applicable for the elimination reaction may include chemical reduction and catalytic reduction.
Suitable reducing agents to be used in chemical reduction are a combination of metal [e.g. tin, zinc, iron, etc] or metallic compound [e.g. chromium chloride, chromium acetate, etc] and an organic or inorganic acid [e.g. formic acid, acetic acid, propionic acid, trifluoroacetic acid, p-toluenesulfonic acid, hydrochloric acid, hydrobromic acid, etc].
Suitable catalysts to be used in catalytic reduction are conventional ones such as platinum catalysts [e.g. platinum plate, spongy platinum, platinum black, colloidal platinum, platinum oxide, platinum wire, etc], palladium catalysts [e.g. spongy palladium, palladium black, palladium oxide, palladium on carbon, colloidal palladium, palladium on barium sulfate, palladium on barium carbonate, etc], nickel catalysts [e.g. reduced nickel, nickel oxide, Raney nickel, etc], cobalt catalysts [e.g. reduced cobalt, Raney cobalt, etc], iron catalysts [e.g. reduced iron, Raney iron, etc], copper catalysts [e.g. reduced copper, Raney copper, Ullman copper, etc] and the like.
The reduction is usually carried out in a conventional solvent which does not adversely influence the reaction such as water, methanol, ethanol, propanol, dioxane, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc, or a mixture thereof.
The reaction temperature of this reduction is not critical and the reaction is usually carried out under cooling to warming.

Process 5

The compound (Id) or a salt thereof can be prepared by reacting the compound (IV) or a reactive derivative at the amino group or a salt thereof with lower aliphatic hydrocarbon compound substituted with oxo which may have one or more suitable substituent(s) in the presence of a reducing agent.
Suitable salt of the compound (Id) can be referred to the ones as exemplified for the compound (I).
Suitable "a reducing agent" may include a conventional reducing agent such as sodium borohydride, lithium aluminum hydride, sodium cyanoborohydride, or the like.
The reaction of this process can be carried out according to the manner disclosed in Examples or a similar manner thereto.

Process 6

The compound (If) or a salt thereof can be prepared by reacting the compound (Ie) or a salt thereof with amidating agent.
Suitable salts of the compounds (Ie) and (If) can be referred to the ones as exemplified for the compound (I).
The reaction of this process can be carried out according to a similar manner to that of Process 1, and so the reaction conditions can be referred to the explanation therein.

Process 7

The compound (Ia) or a salt thereof can be prepared by reacting the compound (VII) or a salt thereof with amidating agent.
Suitable salt of the compound (VII) can be referred to the ones as exemplified for the compound (I).
The reaction of this process can be carried out according to the manner disclosed in Examples or a similar manner thereto.
It is to be noted the compound (I) or a salt thereof can be prepared by the methods other than aforesaid Processes 1 to 7, for examples, by the other methods disclosed in Examples in this specification.
Among the starting compounds, there are some novel compounds, and they can be prepared by the methods, for examples, disclosed in Preparations in this specification.

Biological Property of the Compound (I)

In order to show the utility of the object compound (I), the biological test data on phospholipase $A_2$ assay of the representative compound of the compound (I) is shown in the following.

Test on the inhibitory effect against phospholipase $A_2$ ($PLA_2$)

[I] Test Method

The substrate, [$^{14}$C]phosphatidylcholine (L-3-phosphatidylcholine, 1-stearyl-2-[1-$^{14}$C]arachidonyl, was dried under $N_2$ gas and suspended in deionized water with a probe sonicater. The standard reaction mixture in a total volume of 800 μl contained Tris-HCl buffer (10 mM, pH 7.4), $CaCl_2$ (5 mM) and 10 ng/ml enzyme, $PLA_2$ group I (from porcine pancreas, 700 units/mg, Boehringer Mannheim Yamanouchi Co., Ltd.). The reaction was started by addition of 5 μM [$^{14}$C]phosphatidylcholine (L-3-phosphatidylcholine, 1-stearyl-2-[1-$^{14}$C]arachidonyl to the enzyme solution. Following incubation at 37°C for 30 minutes, the reaction was terminated by addition of 2 ml of Dole's reagent[1)]. To determine the release of $^{14}$C-arachidonic acid from the phospholipid substrate, free fatty acid was extracted by the method of Natori et al[2)], and counted in 8 ml of Liquifluor (Du Pont, New England Nuclear). On the inhibition assay, inhibitor or vehicle was preincubated with enzyme at 37°C for 30 minutes. Each sample was dissolved in methanol (1.25% final). All data are the average of at least duplicate determinations corrected for none enzymatic hydrolysis. Data were expressed as percent inhibition.

1) Dole, V.P. & H. Meinertz: Microdetermination of long-chain fatty acid in plasma and tissues. J. Biol. Chem. 235: 2595-2599, 1960

2) Natori, Y; K. Karasawa, H. Arai, Y. Tamori-Natori & S. Nojima; Partial purification and properties of phospholipase $A_2$ from rat liver mitochondria. J. Biochem. 93; 631-637, 1983

[II] Test Compound

(3S)-3-[(2S)-5-Carboxy-2-(p-methoxybenzoylamino)-pentanoyl]oxyhexadecanamide (the compound of Example 9)

[III] Test Result

Percent inhibition

| Dose | % |
|---|---|
| 83 ng/ml | 87.1 |

It was confirmed that the same test compound also inhibited $PLA_2$ group II.

The pharmaceutical composition of the present invention can be used in the form of a pharmaceutical preparation, for example, in solid, semisolid or liquid form, which contains the object compound (I) or a pharmaceutically acceptable salt thereof, as an active ingredient in admixture with an organic or inorganic carrier or excipient suitable for rectal, pulmonary (nasal or buccal inhalation), nasal, ocular, external (topical), oral or parenteral (including subcutaneous, intravenous, intramuscular and intra-articular) administrations or insufflation.

The active ingredient may be compounded, for example, with the usual non-toxic, pharmaceutically acceptable carriers for tablets, pellets, troches, capsules, suppositories, creams, ointments, aerosols, powders for insufflation, solutions, emulsions, suspensions, and any other form suitable for use. And, if necessary, in addition, auxiliary, stabilizing, thickening and coloring agents and perfumes may be used.

The object compound (I) or a pharmaceutically acceptable salt thereof is/are included in the pharmaceutical composition in an amount sufficient to produce the desired effect upon the process or condition of the diseases.

The pharmaceutical composition of the present invention can be manufactured by the conventional method in this field of the art. If necessary, the technique generally used in this field of the art for improving the bioavailability of a drug can be applied to the pharmaceutical composition of the present invention.

For applying the composition to a human being or an animal, it is preferable to apply it by intravenous (including i.v. infusion), intramuscular, pulmonary, or oral administration, or insufflation including aerosols from metered dose inhalator, nebulizer or dry powder inhalator.

While the dosage of therapeutically effective amount of the object compound (I) varies from and also depends upon the age and condition of each individual patient to be treated, in the case of intravenous administration, a daily dose of 0.001-100 mg of the object compound (I) per kg weight of a human being or an animal, in the case of intramuscular administration, a daily dose of 0.001-100 mg of the object compound (I) per kg weight of a human being of an animal, in case of oral administration, a daily dose of 0.001-200 mg of the object compound (I) per kg weight of a human being or an animal is generally given for the prevention and/or the treatment of aforesaid diseases in a human being or an animal.

The following preparations and examples are given only for the purpose of illustrating the present invention in more detail.

Preparation 1

To a solution of (5)-methyl 3-hydroxyhexadecanoate (10.0 g) in methanol (200 ml) was added aqueous 1N sodium hydroxide (77 ml). The mixture was refluxed for one hour and cooled to 0°C. The resulting precipitate was collected by filtration, and the solid was dissolved in a mixture of ethyl acetate (250 ml) and 1N hydrochloric acid (200 ml). The organic layer was washed with brine (200 ml), dried over magnesium sulfate, and concentrated under reduced pressure. The resulting solid was dissolved in tetrahydrofuran (100 ml). To this solution were added 1-hydroxybenzotriazole (7.56 g) and 1-ethyl-3-(3-dimethylaminopropyl)carbodiimide hydrochloride (10.7 g). After the mixture was stirred for one hour, 28% ammonium hydroxide (5 ml) was added to the mixture and this was stirred at room temperature overnight. Then this mixture was poured into a mixture of ethyl acetate (300 ml) and 0.1N hydrochloric acid (250 ml). The resulting crystalline solid was collected by filtration, washed with ethyl acetate, and recrystallized from 2-propanol (25 ml) to obtain (S)-3-hydroxyhexadecanamide (5.79 g).

NMR (DMSO-$d_6$, δ) : 0.84 (3H, t, J=7Hz), 1.40-1.14 (24H, m), 2.11 (2H, d, J=6Hz), 3.75 (1H, m), 4.58 (1H, d, J=5Hz), 6.88 (1H, s, br), 7.24 (1H, s, br)

Example 1

To a suspended solution of (S)-3-hydroxyhexadecanamide (3.45 g) and (S)-5-benzyloxycarbonyl-2-(tertiarybutoxycarbonylamino)pentanoic acid (4.50 g) in dichloromethane (150 ml) were added benzotriazol-1-yloxy-tris (dimethylamino)phosphonium hexafluorophosphate (5.66 g) and 4-dimethylaminopyridine (3.13 g). The mixture was stirred overnight. The precipitates were filtered off, and the filtrate was concentrated under reduced pressure. The residue was dissolved in ethyl acetate (150 ml) and washed with 1N hydrochloric acid (100 ml x 3), water (100 ml), saturated sodium bicarbonate (100 ml x 2), and brine (100 ml). Then, the solution was dried over magnesium sulfate, and concentrated under reduced pressure. The residue was chromatographed on a silica gel column to give (3S)-3-[(2S)-5-benzyloxycarbonyl-2-(tertiarybutoxycarbonylamino)-pentanoyl]oxyhexadecanamide (3.85 g).

NMR (DMSO-$d_6$, δ) : 0.85 (3H, t, J=7Hz), 1.05-1.32 (24H, m), 1.37 (9H, s), 1.41-1.70 (4H, m), 2.31 (4H, m), 3.84 (1H, br s), 5.07 (3H, m), 6.84 (1H, br s), 7.22 (1H, d, J=8Hz), 7.35 (5H, s)

Example 2

A solution of (3S)-3-[(2S)-5-benzyloxycarbonyl-2-(tertiarybutoxycarbonylamino)pentanoyl]oxyhexadecanamide (100 mg) in trifluoroacetic acid (1 ml) was stirred at room temperature for 20 minutes. Then, the mixture was concentrated under reduced pressure, and dissolved in ethyl acetate (15 ml). This solution was washed with saturated sodium bicarbonate aqueous solution (15 ml x 2), dried over magnesium sulfate, and evaporated. The residue was dissolved in methanol (3 ml), and to this solution was added benzoic anhydride (40 mg). This mixture was allowed to stand at room temperature overnight. Then, the mixture was washed with saturated sodium bicarbonate aqueous solution, dried over magnesium sulfate, and concentrated under reduced pressure. The residue was chromatographed on a silica gel column to give (3S)-3-[(2S)-5-benzyloxy-carbonyl-2-(benzoylamino)pentanoyl]oxyhexadecanamide (76.8 mg).

NMR ($CDCl_3$, δ) : 0.88 (3H, t, J=7Hz), 1.18-1.39 (22H, m), 1.55-2.10 (6H, m), 2.40-2.50 (4H, m), 4.73 (1H, m), 5.10-5.22 (3H, m), 5.32 (1H, m), 5.81 (1H, br s), 5.96 (1H, br s), 6.94 (1H, d, J=7Hz), 7.36 (5H, s), 7.53 (1H, t, J=7Hz), 7.83 (2H, d, J=7Hz)

The following compounds (Examples 3 to 5) were obtained according to a similar manner to that of Example 2.

Example 3

(3S)-3-[(2S)-5-Benzyloxycarbonyl-2-{(1-naphthoyl)amino}pentanoyl]oxyhexadecanamide

NMR ($CDCl_3$-$CD_3OD$, δ) : 0.88 (3H, t, J=7Hz), 1.18-1.41 (22H, m), 1.62-2.14 (6H, m), 2.42-2.53 (4H, m), 4.83 (1H, m), 5.11 (2H, s), 5.28-5.41 (2H, m), 5.93 (1H, s, br), 6.66 (1H, d, J=8Hz), 7.33 (5H, s, br), 7.35-7.60 (3H, m), 7.67 (1H, d, J=8Hz), 7.88 (1H, m), 7.95 (1H, d, J=8Hz), 8.34 (1H, m)

Example 4

(3S)-3-[(2S)-5-Benzyloxycarbonyl-2-(p-methoxybenzoylamino)pentanoyl]oxyhexadecanamide

NMR ($CDCl_3$, δ) : 0.88 (3H, t, J=7Hz), 1.13-1.37 (22H, m), 1.57-2.08 (6H, m), 2.38-2.50 (4H, m), 3.86 (3H, s), 4.70 (1H, m), 5.12 (2H, s), 5.33 (2H, m), 5.98 (1H, s, br), 6.85 (1H, d, J=8Hz), 6.94 (2H, d, J=8Hz), 7.28-7.41 (5H, m), 7.78 (2H, d, J=8Hz)

Example 5

(3S)-3-[(2S)-5-Benzyloxycarbonyl-2-{(2-quinolyl)carbonylamino}pentanoyl]oxyhexadecanamide

NMR ($CDCl_3$, δ) : 0.87 (3H, t, J=7Hz), 1.15-1.58 (22H, m), 1.57-2.17 (6H, m), 2.40-2.53 (4H, m), 4.80 (1H, m), 5.11 (2H, s), 5.32 (1H, m), 5.36 (1H, s, br), 5.96 (1H, s, br), 7.34 (5H, m), 7.64 (1H, dd, J=7Hz, 8Hz), 7.79 (1H, dd, J=7Hz, 8Hz), 7.89 (1H, d, J=8Hz), 8.17 (1H, d, J=8Hz), 8.26 (1H, d, J=8Hz), 8.32 (1H, d, J=8Hz), 8.86 (1H, d, J=8Hz)

Example 6

A solution of (3S)-3-[(2S)-5-benzyloxycarbonyl-2-(tertiarybutoxycarbonylamino)pentanoyl]oxyhexadecanamide (100 mg) in methanol (2 ml) was hydrogenated over 10% palladium on carbon (20 mg) under atmospheric pressure of hydrogen for 3.5 hours at room temperature. Then, the catalyst was filtered off with celite and the filtrate was concentrated under reduced pressure. The residue was recrystallized from a mixture of ethyl acetate (0.5 ml) and n-hexane (1 ml) to give (3S)-3-[(2S)-5-carboxy-2-(tertiarybutoxycarbonylamino)-pentanoyl]oxyhexadecanamide (40 mg).

NMR (DMSO-$d_6$, δ) : 0.85 (3H, t, J=7Hz), 1.1-1.7 (37H, m), 2.16 (2H, m), 2.30 (2H, dd, J=5Hz, 4Hz), 3.82 (1H, m),

5.09 (1H, m), 6.84 (1H, br s), 7.21 (1H, d, J=7.5Hz), 7.35 (1H, br s)

The following compounds (Examples 7 to 10) were obtained according to a similar manner to that of Example 6.

Example 7

(3S)-3-[(2S)-5-Carboxy-2-(benzoylamino)-pentanoyl]oxyhexadecanamide

NMR ($CDCl_3$-$CD_3OD$, δ) : 0.82 (3H, t, J=6Hz), 1.14-1.33 (22H, m), 1.51-2.00 (6H, m), 2.30 (2H, m), 2.44 (2H, d, J=6Hz), 4.66 (1H, m), 5.25 (1H, m), 7.32 (2H, d, J=7Hz), 7.48 (1H, d, J=6Hz), 7.80 (2H, d, J=7Hz)

Example 8

(3S)-3-[(2S)-5-Carboxy-2-{(1-naphthoyl)amino}pentanoyl]oxyhexadecanamide

NMR ($CDCl_3$-$CD_3OD$, δ) : 0.84 (3H, t, J=7Hz), 1.15-1.40 (22H, m), 1.60-2.10 (6H, m), 2.30-2.53 (4H, m), 4.78 (1H, m), 5.29 (1H, m), 7.41-7.58 (4H, m), 7.65 (1H, dd, J=7Hz, 1Hz), 7.85 (1H, m), 7.92 (1H, d, J=8Hz), 8.29 (1H, m)

Example 9

(3S)-3-[(2S)-5-Carboxy-2-(p-methoxybenzoylamino)-pentanoyl]oxyhexadecanamide

NMR ($CD_3OD$, δ) : 0.89 (3H, t, J=7Hz), 1.18-1.44 (22H, m), 1.57-2.04 (6H, m), 2.34 (2H, t, J=7Hz), 2.39-2.60 (2H, m), 3.84 (3H, s), 4.52 (1H, m), 5.27 (1H, m), 6.98 (2H, d, J=8Hz), 7.85 (2H, d, J=8Hz)

Example 10

(3S)-3-[(2S)-5-Carboxy-2-{(2-quinolyl)carbonylamino}-pentanoyl]oxyhexadecanamide

NMR ($CDCl_3$, δ) : 0.86 (3H, t, J=7Hz), 1.00-1.45 (22H, m), 1.50-2.20 (6H, m), 2.28-2.66 (4H, m), 4.85 (1H, m), 5.38 (1H, m), 6.37 (1H, s, br), 7.62 (1H, t, J=8Hz), 7.77 (1H, t, J=8Hz), 7.87 (1H, d, J=8Hz), 8.25 (1H, d, J=8Hz), 8.31 (1H, d, J=8Hz), 8.84 (1H, d, J=8Hz)

Preparation 2

(S)-N-Benzyl-3-hydroxyhexadecanamide was obtained according to a similar manner to that of Preparation 1.

NMR ($CDCl_3$, δ) : 0.88 (1H, t, J=7Hz), 1.16-1.77 (28H, m), 2.30 (1H, dd, J=8Hz, 14Hz), 2.40 (1H, dd, J=3Hz, 14Hz), 4.02 (1H, m), 4.47 (2H, d, J=5Hz), 6.08 (1H, br s), 7.20-7.39 (5H, m)

Preparation 3

(5)-Methyl 3-hydroxyhexadecanoate (1.0 g) was dissolved in a solution of 40% methylamine in methanol (15 ml). This mixture was stirred at room temperature for 2 hours. The resulting crystalline solid was collected by filtration to give (S)-N-methyl-3-hydroxyhexadecanamide (0.71 g).

NMR ($DMSO-d_6$, δ) : 0.86 (3H, t, J=7Hz), 1.10-1.46 (24H, m), 2.12 (2H, d, J=7Hz), 2.55 (3H, d, J=5Hz), 3.77 (1H, m), 4.59 (1H, d, J=5Hz), 7.51 (1H, m)

Preparation 4

A solution of (3S)-3-[(2S)-5-benzyloxycarbonyl-2-(tertiarybutoxycarbonylamino)pentanoyl]oxyhexadecanamide (4.90 g) in 4N hydrogen chloride in ethyl acetate (10 ml) was stirred at room temperature for an hour, and then at 0°C for an additional one hour. This solution was diluted with diethyl ether (25 ml). The resulting precipitate was collected by filtration, and washed with diethyl ether (30 ml). This crystalline solid was dried over potassium hydroxide under vacuum to yield (3S)-3-[(2S)-5-benzyloxycarbonyl-2-aminopentanoyl)oxyhexadecanamide hydrochloride (4.35 g).

NMR ($CDCl_3$, δ) : 0.88 (3H, t, J=7Hz), 1.07-1.42 (22H, m), 1.45-2.21 (6H, m), 2.34-2.69 (4H, m), 4.07 (1H, m), 5.09 (2H, s), 5.30 (1H, m), 6.78 (1H, br s), 7.35 (5H, m)

Preparation 5

(3S)-3-[(2S)-5-Carboxy-2-aminopentanoyl]-oxyhexadecanamide was obtained according to a similar manner to that of Example 6.

NMR ($CDCl_3$-$CD_3OD$ (20:1), δ) : 0.87 (3H, t, J=7Hz), 1.05-1.41 (22H, m), 1.47-2.21 (6H, m), 2.28-2.76 (4H, m), 4.08 (1H, m), 5.33 (1H, m)

Preparation 6

To a solution of (R)-3-hydroxyhexadecanamide (100 mg) and pyridine (210 µl) in dichloromethane (10 ml) was added methanesulfonyl chloride (144 µl). This mixture was stirred at room temperature overnight, and the resulting precipitate was filtered off. The filtrate was concentrated, and dissolved in ethyl acetate (20 ml). This solution was washed with 1N-hydrochloric acid (20 ml x 3), saturated sodium carbonate (20 ml x 2), and brine (20 ml). After drying over $MgSO_4$ and concentration, the residue was chromatographed on a silica gel, eluting with a mixture of dichloromethane and methanol (50:1) to give (R)-3-methanesulfonyloxyhexadecanamide (40 mg).

NMR ($CDCl_3$, δ) : 0.87 (3H, t, J=7Hz), 1.35-1.50 (22H, m), 1.81 (2H, m), 2.62 (2H, d, J=7Hz), 3.04 (3H, s), 5.04 (1H, m), 5.45 (1H, br s), 5.68 (1H, br s)

Preparation 7

A solution of (R)-3-methanesulfonyloxyhexadecanamide (32 mg) and sodium azide (12 mg) in N,N-dimethylformamide (1 ml) was heated at 60°C for 2 hours. The cooled mixture was partitioned between ethyl acetate (20 ml) and water (10 ml). The organic phase was washed with water (10 ml), and brine (10 ml), dried, and concentrated under vacuum, then the residue was recrystallized at -20°C from a mixture of ethyl acetate (0.2 ml) and n-hexane (2 ml) to give (S)-3-azidohexadecanamide (14 mg).

NMR ($CDCl_3$, δ) : 0.88 (3H, t, J=7Hz), 1.18-1.64 (24H, m), 2.34 (1H, dd, J=15Hz, 8Hz), 2.42 (1H, dd, J=15Hz, 6Hz), 3.85 (1H, m), 5.40 (1H, br s), 5.62 (1H, br s)

Preparation 8

A mixture of (S)-3-azidohexadecanamide (0.17 g) and 10% palladium on carbon (20 mg) was hydrogenated at atmospheric pressure for 5 hours. The catalyst was filtered off with celite, and the filtrate was concentrated under reduced pressure. The residue was recrystallized from ethyl acetate (3 ml) to give (S)-3-aminohexadecanamide (106 mg).

NMR (DMSO-$d_6$, δ) : 0.85 (3H, t, J=7Hz), 1.13-1.52 (24H, m), 1.94 (1H, dd, J=14Hz, 8Hz), 2.08 (1H, dd, J=14Hz, 5Hz), 2.89 (1H, m), 6.72 (1H, br s), 7.36 (1H, br s)

The following compounds (Preparations 9 and 10) were obtained according to a similar manner to that of Preparation 1.

Preparation 9

3-Hydroxydecanamide

NMR (DMSO-$d_6$, δ) : 0.86 (3H, t, J=7Hz), 1.14-1.48 (12H, m), 2.11 (2H, d, J=7Hz), 3.75 (1H, m), 4.57 (1H, d, J=5Hz), 6.77 (1H, br s), 7.24 (1H, br s)

Preparation 10

(R)-3-Hydroxyhexadecanamide

NMR (DMSO-$d_6$, δ) : 0.84 (3H, t, J=7Hz), 1.40-1.14 (24H, m), 2.11 (2H, d, J=6Hz), 3.75 (1H, m), 4.58 (1H, d, J=5Hz), 6.88 (1H, br s), 7.26 (1H, br s)

Preparation 11

(S)-Methyl 3-hydroxyhexadecanoate and benzyl bromide were reacted according to a similar manner to that of Preparation 1 to give (S)-benzyl 3-hydroxyhexadecanoate.

NMR ($CDCl_3$, δ) : 0.87 (3H, t, J=7Hz), 1.16-1.58 (24H, m), 2.46 (1H, dd, J=16Hz, 8Hz), 2.56 (1H, dd, J=16Hz, 3Hz), 2.84 (1H, d, J=4Hz), 4.02 (1H, m), 5.15 (2H, s), 7.36 (5H, m)

Preparation 12

(S)-Methyl 3-hydroxyhexadecanoate and 2,2,2-trichloroethyl alcohol were reacted according to a similar manner to that of Preparation 1 to give (S)-(2,2,2-trichloroethyl) 3-hydroxyhexadecanoate.

NMR ($CDCl_3$, δ) : 0.87 (3H, t, J=7Hz), 1.17-1.62 (24H, m), 2.53 (1H, d, J=5Hz), 2.58 (1H, dd, J=16Hz, 8Hz), 2.67 (1H, dd, J=16Hz, 4Hz), 4.18 (1H, m), 4.76 (1H, d, J=12Hz), 4.80 (1H, d, J=12Hz)

The following compounds (Preparations 13 and 14) were obtained according to a similar manner to that of Preparation 3.

Preparation 13

2-Hydroxy-N-methylpentadecanamide

NMR ($CDCl_3$, δ) : 0.87 (3H, t, J=7Hz), 1.15-1.47 (22H, m), 1.63 (1H, m), 1.83 (1H, m), 2.36 (1H, d, J=SHz), 2.86 (3H, d, J=6Hz), 4.12 (1H, m), 6.43 (1H, br s)

Preparation 14

N-(N-Methylcarbamoylmethyl)tridecylamine

NMR (DMSO-$d_6$, δ) : 0.86 (3H, t, J=7Hz), 1.15-1.34 (22H, m), 2.03 (1H, br s), 2.41 (2H, t, J=7Hz), 2.40 (3H, d, J=5Hz), 3.02 (2H, s), 7.68 (1H, br s)

Preparation 15

A solution of methyl 3-(tridecylamino)propionate (0.77 g) in 10N-methanolic ammonia (7 ml) was allowed to

stand at room temperature for 7 days. The resultant crystal was collected by filtration and washed with a small portion of methanol to give 3-(tridecylamino)propionamide (0.16 g).

NMR ($CDCl_3$, δ) : 0.88 (3H, t, J=7Hz), 1.18-1.59 (22H, m), 2.36 (2H, t, J=7Hz), 2.61 (2H, t, J=8Hz), 2.87 (2H, t, J=7Hz), 5.30 (1H, br s), 7.79 (1H, br s)

<u>Preparation 16</u>

To an ice-cooled solution of methyl 3-oxododecanoate in methanol was added sodium borohydride (83 mg). After stirring for 20 minutes, IN-hydrochloric acid (2.2 ml) was added. After concentration, the residue was dissolved in ethyl acetate (30 ml) and 0.1N-hydrochloric acid (20 ml). The organic phase was washed with water (20 ml), saturated aqueous sodium bicarbonate (20 ml), and brine (20 ml), dried, and concentrated. The residue was dissolved in methanol (3 ml), and to this solution was added 1N-sodium hydroxide (2.4 ml). The mixture was refluxed for an hour, and diluted with water (30 ml). This aqueous solution was washed with dichloromethane (20 ml), acidified with 1N-hydrochloric acid (2.6 ml), and extracted with ethyl acetate (30 ml). This organic phase was washed with brine (20 ml), dried, and concentrated. The residue was recrystallized from a mixture of ethyl acetate (1 ml) and n-hexane (3 ml) to give 3-hydroxydodecanoic acid (0.27 g).

NMR (DMSO-$d_6$, δ) : 0.86 (3H, t, J=7Hz), 1.13-1.47 (16H, m), 2.22 (1H, dd, J=8Hz, 14Hz), 2.29 (1H, dd, J=7Hz, 14Hz), 3.79 (1H, m)

<u>Preparation 17</u>

3-Hydroxytetradecanoic acid was obtained according to a similar manner to that of <u>Preparation 16.</u>

NMR (DMSO-$d_6$, δ) : 0.86 (3H, t, J=7Hz), 1.12-1.48 (20H, m), 2.22 (1H, dd, J=8Hz, 15Hz), 2.28 (1H, dd, J=5Hz, 15Hz), 3.78 (1H, m)

<u>Preparation 18</u>

To a solution of 3-hydroxydodecanoic acid (0.26 g) in tetrahydrofuran (4 ml) was added 1-ethyl-3-(3-dimethylaminopropyl)carbodiimide hydrochloride (0.18 g) and 1-hydroxybenzotriazole (0.24 g). After stirring for 30 minutes, 28% ammonium hydroxide (0.17 ml) was added and the mixture was stirred at room temperature overnight. Then, the mixture was diluted with ethyl acetate (100 ml) and washed with 0.1N-hydrochloric acid (100 ml x 2), saturated sodium bicarbonate (50 ml), and brine (50 ml). After drying over magnesium sulfate and evaporation, the residue was recrystallized from isopropyl alcohol (1 ml) to give 3-hydroxydodecanamide (0.20 g).

NMR (DMSO-$d_6$, δ) : 0.86 (3H, t, J=7Hz), 1.13-1.47 (16H, m), 2.10 (2H, d, J=7Hz), 3.75 (1H, m), 4.57 (1H, d, J=5Hz), 6.77 (1H, br s), 7.25 (1H, br s)

<u>Preparation 19</u>

3-Hydroxytetradecanamide was obtained according to a similar manner to that of <u>Preparation 18.</u>

NMR (DMSO-$d_6$, δ) : 0.85 (3H, t, J=7Hz), 1.15-1.41 (20H, m), 2.11 (2H, d, J=7Hz), 3.74 (1H, m), 4.57 (1H, d, J=5Hz), 6.77 (1H, br s), 7.24 (1H, br s)

<u>Preparation 20</u>

To methanol (10 ml), cooled in an ice-brine bath, was added thionyl chloride (0.87 ml) dropwise. After stirring at 0°C for 10 minutes, 2-hydroxypentadecanoic acid (1.54 g) was added. The mixture was stirred at room temperature for 3 hours, and concentrated under vacuum. The residue was dissolved in ethyl acetate (60 ml), and washed with water (40 ml x 2), saturated aqueous sodium bicarbonate (30 ml), and brine (30 ml). The organic phase was dried, concentrated to dryness, and triturated in diisopropyl ether to give methyl 2-hydroxypentadecanoate (0.77 g).

NMR ($CDCl_3$, δ) : 0.88 (3H, t, J=7Hz), 1.16-1.42 (22H, m), 1.58-1.75 (2H, m), 2.67 (1H, d, J=7Hz), 3.78 (3H, s), 4.18 (1H, m)

Preparation 21

To an ice-cooled solution of tridecanal (1.0 g), methyl 3-aminopropionate hydrochloride (704 mg), and sodium acetate (414 mg) in methanol (20 ml) was added sodium cyanoborohydride (317 mg). After stirring for 45 minutes, the mixture was quenched with water (20 ml), concentrated, and extracted with ethyl acetate (40 ml). The organic phase was washed with brine, dried, and concentrated under reduced pressure. The residue was purified on silica gel, eluting with 5% methanol in chloroform to give methyl 3-tridecylaminopropionate (0.78 g).

NMR ($CDCl_3$, δ) : 0.88 (3H, t, J=7Hz), 1.18-1.41 (20H, m), 1.55 (2H, m), 2.62 (2H, t, J=7Hz), 2.69 (2H, t, J=8Hz), 2.96 (2H, t, J=7Hz), 3.71 (3H, s)

Preparation 22

Methyl 2-(tridecylamino)acetate was obtained according to a similar manner to that of Preparation 21.

NMR ($CDCl_3$, δ) : 0.88 (3H, t, J=7Hz), 1.14-1.63 (22H, m), 2.58 (2H, m), 3.40 (2H, s), 3.72 (3H, s)

Preparation 23

To an ice-cooled solution of tetradecanal (18.75 g) in diethyl ether (100 ml) was added acetic acid (5.66 ml) and potassium cyanide (6.64 g). The mixture was warmed to room temperature and stirred for 72 hours. The resulting solution was washed with water, and the aqueous phase was extracted with diethyl ether (50 ml). The combined organic phase was dried, and concentrated to dryness. The residue was purified by silica gel chromatography and triturated in diisopropyl ether (10 ml) to give 2-hydroxypentadecanenitrile (4.66 g).

NMR ($CDCl_3$, δ) : 0.88 (3H, t, J=7Hz), 1.16-1.42 (20H, m), 1.50 (2H, m), 1.86 (2H, m), 2.27 (1H, d, J=7Hz), 4.48 (1H, ddd, J=7Hz, 7Hz, 7Hz)

Preparation 24

2-Hydroxypentadecanenitrile (1.0 g) was suspended in concentrated hydrochloric acid (15 ml), and this suspension was vigorously stirred at room temperature for 24 hours. Then, this mixture was diluted with water (45 ml). The insoluble powder was collected by filtration, washed with water (10 ml x 3), and dried to give 2-hydroxypentadecanamide (1.06 g).

NMR (DMSO-$d_6$, δ) : 0.86 (3H, t, J=7Hz), 1.13-1.66 (24H, m), 3.75 (1H, m), 5.22 (1H, d, J=6Hz), 7.05 (1H, br s), 7.11 (1H, br s)

Preparation 25

2-Hydroxypentadecanenitrile (3.63 g) was suspended in concentrated hydrochloric acid (54 ml), and stirred for 66 hours. This mixture was stirred at 60°C for 6 hours, and refluxed for 95 minutes. Then, this was cooled to room temperature, and diluted with water (150 ml). The resulting precipitate was collected by filtration, and recrystallized from the mixture of ethyl acetate (4 ml) and n-hexane (16 ml) to give 2-hydroxypentadecanoic acid (2.30 g).

NMR (DMSO-$d_6$, δ) : 0.85 (3H, t, J=7Hz), 1.13-1.38 (22H, m), 1.41-1.66 (2H, m), 3.90 (1H, dd, J=8Hz, 5Hz)

Preparation 26

(3R)-[(2R)-5-Benzyloxycarbonyl-2-aminopentanoyl]oxyhexadecanamide hydrochloride (1.67 g) was obtained according to a similar manner to that of Preparation 4.

NMR (DMSO-$d_6$, δ) : 0.85 (3H, t, J=7Hz), 1.14-1.32 (22H, m), 1.50-1.85 (6H, m), 2.39 (4H, m), 4.01 (1H, m), 5.09 (2H, s), 5.22 (1H, m), 6.87 (1H, m), 7.35 (5H, m), 7.42 (1H, br s), 8.28 (2H, br s)

Preparation 27

(3S)-(2,2,2-Trichloroethyl) 3-[(2S)-5-benzyloxycarbonyl-2-(tertiarybutoxycarbonylamino)pentanoyl]oxyhexadecanoate (15.5 g) was dissolved with 4N-hydrogen chloride in ethyl acetate (30 ml), and this solution was stirred at room temperature for 40 minutes. After evaporation, the residue was dissolved in toluene (100 ml). To this solution was added triphosgene (5.67 g) and charcoal (100 mg), and this mixture was refluxed for 2 hours. Then, the solvent was removed by distillation, and the residue was crystallized in n-hexane (60 ml) at 4°C to give (3S)-2,2,2-trichloroethyl) 3-[(2S)-5-benzyloxycarbonyl-2-isocyanatopentanoyl]-oxyhexadecanoate (8.0 g).

NMR ($CDCl_3$, δ) : 0.88 (3H, t, J=7Hz), 1.15-1.39 (22H, m), 1.60-2.00 (6H, m), 2.39 (2H, t, J=7Hz), 4.36 (2H, m), 4.72 (1H, d, J=12Hz), 4.77 (1H, d, J=12Hz), 5.12 (2H, s), 5.34 (1H, m), 7.35 (5H, m)

Preparation 28

Isobutyl bromide (27.4 g) was added to magnesium (5.0 g) in tetrahydrofuran (100 ml). The mixture was stirred for 1 hour under nitrogen atmosphere. The mixture was added to a solution of 1,10-dibromodecane (50.0 g) and dilithium tetrachlorocuprate (1 m mole) in tetrahydrofuran (100 ml) at 0°C under nitrogen atmosphere. The mixture was stirred at 0°C for 1 hour and poured into a mixture of 7% hydrochloric acid (200 ml) and ethyl acetate (200 ml). The organic layer was washed with water, dried over magnesium sulfate and evaporated. The residue was distilled under reduced pressure to give a mixture of the product and 1,10-dibromodecane as a colorless oil (27.23 g) (bp.115-120°C/0.5 Torr). The mixture was chromatographed on a silica gel column eluting with n-hexane to give 1-bromo-12-methyltridecane as an oil (15.3 g).

NMR ($CDCl_3$, δ) : 3.41 (2H, t, J=7Hz), 1.86 (2H, m), 1.4-1.6 (1H, m), 1.1-1.4 (18H, m), 0.86 (6H, d, J=7Hz)

Preparation 29

Magnesium (1.5 g) was added to a solution of 1-bromo-12-methyltridecane (15.0 g) in tetrahydrofuran (200 ml). The mixture was refluxed for 10 minutes under nitrogen atmosphere and stirred for 1 hour at room temperature. A solid of carbon dioxide (50 g) was added to the mixture. To the mixture was added a mixture of 1% hydrochloric acid (300 ml) and ethyl acetate (150 ml). The organic layer was washed with water, dried over magnesium sulfate and evaporated. The residue was chromatographed on a silica gel column eluting with chloroform to give 13-methyltetradecanoic acid as an oil (7.54 g).

NMR ($CDCl_3$, δ) : 2.35 (2H, t, J=7Hz), 1.4-1.7 (3H, m), 1.1-1.4 (18H, m), 0.88 (6H, d, J=7Hz)

Preparation 30

To a solution of 13-methyltetradecanoic acid (7.45 g) in dichloromethane (50 ml) was added oxalyl chloride (3 ml) and N,N-dimethylformamide (0.05 ml). The mixture was stirred for 1 hour and evaporated. The residue was dissolved in n-hexane (50 ml) and insoluble salts was filtered off. The filtrate was evaporated and the residue was dissolved in dichloromethane (50 ml). The solution was added to a solution of 2,2-dimethyl-1,3-dioxane-4,6-dione (4.42 g) and pyridine (4.90 g) in dichloromethane (50 ml) at 0°C. The mixture was stirred at 0°C for 1 hour and at room temperature for 1 hour. The mixture was washed with diluted hydrochloric acid and water, dried over magnesium sulfate and evaporated. The residue was dissolved in benzyl alcohol (50 ml) and stirred at 100°C for 1 hour. The solvent was removed under reduced pressure. The residue was chromatographed on a silica gel column eluting with a mixture of n-hexane and ethyl acetate (10:1) to give benzyl 15-methyl-3-oxohexadecanoate of a colorless oil (6.40 g).

NMR ($CDCl_3$, δ) : 7.35 (5H, s), 5.18 (2H, s), 3.49 (2H, s), 2.50 (2H, t, J=7Hz), 1.4-1.7 (3H, m), 1.1-1.3 (18H, m), 0.86 (6H, d, J=7Hz)

Preparation 31

To a solution of benzyl 15-methyl-3-oxohexadecanoate (3.74 g) in 2-propanol (50 ml) was added sodium borohydride (0.38 g) at 0°C. The mixture was stirred at 0°C for 2 hours and poured into a mixture of ethyl acetate and diluted hydrochloric acid. The organic layer was washed with water, dried over magnesium sulfate and evaporated. The residue was chromatographed on a silica gel column eluting with a mixture of n-hexane and ethyl acetate (10:1) to give benzyl 3-hydroxy-15-methylhexadecanoate as a colorless oil (2.10 g).

NMR ($CDCl_3$, δ) : 7.38 (5H, s), 5.16 (2H, s), 4.03 (1H, m), 2.89 (1H, d, J=3Hz), 2.4-2.6 (2H, m), 1.4-1.6 (3H, m), 1.1-1.4 (20H, m), 0.88 (6H, d, J=7Hz)

Preparation 32

To a solution of benzyl 3-hydroxy-15-methylhexadecanoate (1.77 g) and N-tertiarybutoxycarbonyl-L-phenylalanine (1.25 g) in dichloromethane (50 ml) was added benzotriazol-1-yloxy-tris(dimethylamino)phosphonium hexafluorophosphate (2.08 g) and 4-dimethylaminopyridine (1.22 g). The mixture was stirred at room temperature for 14 hours and evaporated. The residue was poured into a mixture of ethyl acetate and diluted hydrochloric acid. The organic layer was washed with water, dried over magnesium sulfate and evaporated to give almost pure benzyl 3-(N-tertiary butoxycarbonyl-L-phenylalanyl)oxy-15-methylhexadecanoate, which can be used for next step without further purification (2.87 g).

NMR ($CDCl_3$, δ) : 7.1-7.4 (10H, m), 5.25 (1H, m), 5.12 (2H, s), 4.92 (1H, m), 4.50 (1H, m), 2.8-3.2 (2H, m), 2.5-2.7 (2H, m), 1.5-1.7 (3H, m), 1.40 (9H, s), 1.1-1.4 (20H, m), 0.88 (6H, d, J=7Hz)

Preparation 33

To a solution of benzyl 3-(N-tertiarybutoxycarbonyl-L-phenylalanyl)oxy-15-methylhexadecanoate (2.85 g) in methanol (50 ml) was added 10% palladium on active carbon (0.5 g) and water (5 ml). The mixture was stirred at room temperature under hydrogen atmosphere for 1 hour. The catalyst was filtered off and the solvent was removed under reduced pressure to give 3-(N-tertiarybutoxycarbonyl-L-phenylalanyl)oxy-15-methylhexadecanoic acid as an oil (2.31 g) .

NMR ($CDCl_3$, δ) : 7.1-7.4 (5H, m), 5.22 (1H, m), 5.00 (1H, d, J=7Hz), 4.52 (1H, m), 2.9-3.2 (2H, m), 2.5-2.7 (2H, m), 1.5-1.7 (3H, m), 1.40 (9H, s), 1.1-1.4 (20H, m), 0.88 (6H, d, J=7Hz)

Preparation 34

To a solution of 3-(N-tertiarybutoxycarbonyl-L-phenylalanyl)oxy-15-methylhexadecanoic acid (2.30 g) in tetrahydrofuran (30 ml) was added 1-hydroxybenzotriazole (0.58 g) and 1-ethyl-3-(3-dimethylaminopropyl) carbodiimide hydrochloride (0.82 g). The mixture was stirred at room temperature for 30 minutes. 28% Ammonia solution (0.5 ml) was added to the mixture and then the mixture was stirred at room temperature for 2 hours. The mixture was poured into a mixture of ethyl acetate and diluted hydrochloric acid. The organic layer was washed with water, dried over magnesium sulfate and evaporated. The residue was chromatographed on a silica gel column eluting with chloroform to give 3-(N-tertiarybutoxycarbonyl-L-phenylalanyl)oxy-15-methylhexadecanamide as white wax (1.41 g).

NMR ($CDCl_3$, δ) : 7.1-7.4 (5H, m), 4.9-5.3 (2H, m), 4.3-4.6 (1H, m), 2.9-3.2 (2H, m), 2.3-2.6 (2H, m), 1.4-1.7 (3H, m), 1.4 (9H, s), 1.1-1.3 (20H, m), 0.86 (6H, d, J=7Hz)

Preparation 35

To a solution of 3-(N-tertiarybutoxycarbonyl-L-phenylalanyl)oxy-15-methylhexadecanamide (1.40 g) and anisole (1.0 g) in dichloromethane (30 ml) was added trifluoroacetic acid (5 ml). The mixture was stirred at room temperature for 30 minutes and evaporated. The residue was dissolved in diethyl ether (50 ml) and washed with sodium bicarbonate aqueous solution and water, dried over magnesium sulfate and evaporated. The residue was chromatographed on a silica gel column eluting with a mixture of chloroform and methanol (10:1) to give the product as white powder.

(S)-15-methyl-3-(L-phenylalanyl)oxyhexadecanamide (0.60 g)

$[\alpha]_D^{23}$ - 1.2° (c 0.5, $CHCl_3$)

NMR ($CDCl_3$, δ) : 7.2-7.4 (5H, m), 5.60 (1H, br s), 5.25 (1H, br s), 5.18 (1H, m), 3.72 (1H, t, J=7Hz), 3.08 (1H, dd, J=15Hz, 6Hz), 2.85 (1H, dd, J=15Hz, 6Hz), 2.40 (2H, m), 1.4-1.7 (5H, m), 1.1-1.4 (20H, m), 0.86 (6H, d, J=7Hz)

(R)-15-methyl-3-(L-phenylalanyl)oxyhexadecanamide (0.50 g)

$[\alpha]_D^{23}$ + 8.7° (c 0.5, $CHCl_3$)

NMR ($CDCl_3$, δ) : 7.2-7.4 (5H, m), 5.79 (1H, br s), 5.42 (1H, br s), 5.18 (1H, m), 3.72 (1H, m), 3.08 (1H, dd, J=15Hz,

6Hz), 2.85 (1H, dd, J=15Hz, 6Hz), 2.48 (2H, d, J=6Hz), 1.4-1.7 (5H, m), 1.1-1.4 (20H, m), 0.86 (6H, d, J=7Hz)

Preparation 36

To a solution of (S)-15-methyl-3-(L-phenylalanyl)-oxyhexadecanamide (0.58 g) in methanol (30 ml) was added 1N-sodium hydroxide aqueous solution (3 ml). The mixture was stirred at room temperature for 30 minutes and poured into a mixture of ethyl acetate and diluted hydrochloric acid. The organic layer was washed with water, dried over magnesium sulfate and evaporated. The residue was crystallized from diisopropyl ether to give (S)-3-hydroxy- 15-methylhexadecanamide as white powder (0.20 g).

mp : 94-96°C

$[\alpha]_D^{23}$ + 17.26°(c 0.5, $CHCl_3$)

NMR ($CDCl_3$, δ) : 5.85 (1H, br s), 5.56 (1H, br s), 4.00 (1H, m), 3.32 (1H, br s), 2.2-2.5 (2H, m), 1.1-1.7 (21H, m), 0.86 (6H, d, J=7Hz)

Preparation 37

(S)-3-Hydroxy-15-methylhexadecanamide (0.20 g) was added to a solution of potassium hydroxide (0.1 g) in ethanol (10 ml). The mixture was refluxed for 15 hours, cooled and poured into a mixture of ethyl acetate and diluted hydrochloric acid. The organic layer was washed with water, dried over magnesium sulfate and evaporated. The residue was chromatographed on a silica gel column eluting with a mixture of chloroform and methanol (10:1) to give (S)-3-hydroxy-15-methylhexanoic acid as white powder (67 mg).

$[\alpha]_D^{25}$ + 11.18° (c 0.3, $CHCl_3$)

NMR ($CDCl_3$, δ) : 4.03 (1H, m), 2.4-2.7 (2H, m), 1.4-1.6 (3H, m), 1.1-1.4 (20H, m), 0.86 (6H, d, J=7Hz)

Preparation 38

To a solution of (S)-3-hydroxy-15-methylhexadecanamide (0.18 g) and 5-benzyloxycarbonyl-2-(L- tertiarybutoxycarbonylamino)pentanoic acid (0.22 g) in dichloromethane (10 ml) was added benzotriazol-1-yloxy-tris(dimethylamino)-phosphonium hexafluorophosphate (0.28 g) and 4-dimethylaminopyridine (0.16 g). The mixture was stirred at room temperature for 14 hours and evaporated. The residue was poured into a mixture of ethyl acetate and diluted hydrochloric acid. The organic layer was washed with water, dried over magnesium sulfate and evaporated. The residue was chromatographed on a silica gel column eluting with a mixture of chloroform and methanol (50:1) to give (3S)-3-[(2S)-5-benzyloxycarbonyl-2-(tertiarybutoxycarbonylamino)-pentanoyl]oxy-15-methylhexadecanamide as an oil (0.20 g).

NMR ($CDCl_3$, δ) : 7.35 (5H, s), 5.90 (1H, br s), 5.31 (1H, br s), 5.25 (1H, m), 5.12 (2H, s), 5.06 (1H, m), 4.23 (1H, m), 2.35-2.5 (4H, m), 1.5-1.9 (5H, m), 1.44 (9H, s), 1.1-1.4 (22H, m), 0.87 (6H, d, J=7Hz)

The following compounds (Examples 11 and 12) were obtained according to a similar manner to that of Example 1.

Example 11

(3S)-N-Methyl-3-[(2S)-5-benzyloxycarbonyl-2-(tertiarybutoxycarbonylamino)pentanoyl]oxyhexadecanamide

NMR ($CDCl_3$, δ) : 0.88 (3H, t, J=7Hz), 1.12-1.90 (37H, m), 2.74 (3H, d, J=5Hz), 4.21 (1H, m), 5.05 (1H, d, J=7Hz), 5.12 (2H, s), 5.23 (1H, m), 5.99 (1H, br s), 7.35 (5H, m)

Example 12

(3S)-N-Benzyl-3-[(2S)-5-benzyloxycarbonyl-2-(tertiarybutoxycarbonylamino)pentanoyl]oxyhexadecanamide

NMR ($CDCl_3$, δ) : 0.88 (3H, t, J=7Hz), 1.17-1.37 (22H, m), 1.43 (9H, s), 1.37-1.48 (2H, m), 1.50-1.82 (4H, m), 2.31 (2H, t, J=6Hz), 2.46 (2H, d, J=5Hz), 4.14 (1H, m), 4.39 (2H, m), 5.01 (1H, d, J=7Hz), 5.06 (2H, s), 5.27 (1H, m), 6.41 (1H, m), 7.22-7.41 (10H, m)

Example 13

(3S)-3-[(2S)-5-Benzyloxycarbonyl-2-(5-dimethylamino-1-naphthylsulfonylamino)pentanoyl]oxyhexadecanamide was obtained according to a similar manner to that of Example 2.

NMR ($CDCl_3$, δ) : 0.88 (3H, t, J=6Hz), 0.98-1.72 (28H, m), 2.11-2.26 (4H, m), 2.86 (6H, s), 3.83 (1H, m), 4.99 (1H, m), 5.05 (2H, s), 5.28 (1H, br s), 5.54 (1H, d, J=8Hz), 5.61 (1H, br s), 7.17 (1H, d, J=7Hz), 7.26-7.40 (5H, m), 7.45-7.61 (2H, m), 8.22 (1H, d, J=7Hz), 8.27 (1H, d, J=8Hz), 8.53 (1H, d, J=8Hz)

Example 14

To a solution of (3S)-3-[(2S)-5-benzyloxycarbonyl-2-aminopentanoyl]oxyhexadecanamide hydrochloride (2.17 g) in dichloromethane (8 ml) were added phenyl chlorocarbonate (0.65 ml) and triethylamine (1.68 ml). This mixture was stirred at room temperature overnight, and then poured into a mixture of ethyl acetate (80 ml) and 1N hydrochloric acid (80 ml). The organic layer was washed with water, saturated aqueous sodium bicarbonate, and brine successively. This solution was dried and evaporated to dryness. The residue was chromatographed on silica gel (200 cc) eluting with a mixture of dichloromethane and methanol (20:1) to give (3S)-3-[(2S)-5-benzyloxycarbonyl-2-(phenoxycarbonylamino)-pentanoyl]oxyhexadecanamide (1.73 g).

NMR ($CDCl_3$, δ) : 0.87 (3H, t, J=7Hz), 1.12-1.48 (22H, m), 1.54-2.03 (6H, m), 2.46-2.54 (4H, m), 4.38 (1H, m), 5.12 (2H, s), 5.30 (1H, m), 5.69 (1H, d, J=7Hz), 5.76 (1H, br s), 7.13 (2H, d, J=8Hz), 7.22 (1H, dd, J=7Hz, 7Hz), 7.29-7.52 (7H, m)

The following compounds (Examples 15 to 17) were obtained according to a similar manner to that of Example 14.

Example 15

(3S)-3-[(2S)-5-Carboxy-2-{(2E,4E)-hexadienoylamino}-pentanoyl]oxyhexadecanamide

NMR ($CD_3OD$, δ) : 0.88 (3H, t, J=7Hz), 1.07-1.45 (22H, m), 1.48-1.99 (6H, m), 1.83 (3H, t, J=6Hz), 2.22-2.60 (4H, m), 4.42 (1H, m), 5.25 (1H, m), 5.97 (1H, d, J=15Hz), 6.13 (1H, dt, J=15Hz, 6Hz), 6.23 (1H, dd, J=10Hz, 15Hz), 7.24 (1H, dd, J=10Hz, 15Hz)

Example 16

(3S)-3-[(2S)-5-Carboxy-2-cinnamoylaminopentanoyl]-oxyhexadecanamide

NMR ($CD_3OD$, δ) : 0.88 (3H, t, J=7Hz), 1.09-1.53 (22H, m), 1.53-2.03 (6H, m), 2.34 (2H, m), 2.49 (2H, m), 4.48 (1H, m), 5.39 (1H, m), 6.49 (1H, d, J=21Hz), 7.33-7.45 (3H, m), 7.49-7.62 (3H, m)

Example 17

(3S)-3-[(2S)-5-Carboxy-2-(crotonoylamino)pentanoyl]-oxyhexadecanamide

NMR ($CDCl_3$-$CD_3OD$ (20:1), δ) : 0.85 (3H, t, J=6Hz), 1.14-1.50 (22H, m), 1.50-1.78 (6H, m), 1.85 (3H, d, J=7Hz), 2.30 (2H, m), 2.46 (2H, d, J=6Hz), 4.55 (1H, m), 5.24 (1H, m), 5.87 (1H, dd, J=14Hz, 2Hz), 6.83 (1H, m)

Example 18

A solution of (3S)-3-[(2S)-5-benzyloxycarbonyl-2-(phenoxycarbonylamino)pentanoyl]oxyhexadecanamide (1.70 g) and 2-aminopyridine (2.56 g) in pyridine (10 ml) was stirred at 70°C for 64 hours, and then poured into a mixture of ethyl acetate (100 ml) and 1N hydrochloric acid (100 ml). The organic phase was washed with 1N hydrochloric acid (100 ml), 0.5N hydrochloric acid (100 ml), saturated aqueous sodium bicarbonate (100 ml), and brine (50 ml) successively, dried over magnesium sulfate, and evaporated to dryness. The residue was chromatographed on silica gel (300 cc) eluting with a mixture of dichloromethane and acetone (2:1 - 1:1) to give (3S)-3-[(2S)-5-benzyloxycarbonyl-2-{3-(2-pyridyl)ureido}pentanoyl]oxyhexadecanamide (1.14 g).

NMR ($CDCl_3$, δ) : 0.87 (3H, t, J=7Hz), 1.00-1.40 (22H, m), 1.48-2.12 (6H, m), 2.33-2.57 (4H, m), 4.54 (1H, m), 5.12 (2H, s), 5.22 (1H, m), 6.08 (1H, br s), 6.30 (1H, br s), 6.82 (1H, d, J=7Hz), 6.89 (1H, dd, J=5Hz, 5Hz), 7.27-7.42 (5H, m), 7.60 (1H, dd, J=5Hz, 7Hz), 8.19 (1H, d, J=5Hz), 8.48 (1H, s)

Example 19

(3S)-3-[(2S)-5-Benzyloxycarbonyl-2-(3-(2-pyridylmethyl)ureido}pentanoyl]oxyhexadecanamide was obtained according to a similar manner to that of Example 18.

NMR ($CDCl_3$, δ) : 0.88 (3H, t, J=7Hz), 1.15-1.38 (22H, m), 1.50-1.90 (6H, m), 2.33-2.52 (4H, m), 4.41 (1H, m), 4.47 (1H, m), 5.12 (2H, s), 5.21 (1H, m), 5.52 (1H, br s), 5.68 (1H, m), 6.13 (1H, br s), 7.20 (1H, dd, J=5Hz, 8Hz), 7.27 (1H, d, J=8Hz), 7.34 (5H, m), 7.67 (1H, t, J=8Hz), 8.52 (1H, d, J=5Hz)

The following compounds (Examples 20 to 25) were obtained according to a similar manner to that of Example 6.

Example 20

(3S)-3-[(2S)-5-Carboxy-2-(phenoxycarbonylamino)-pentanoyl]oxyhexadecanamide

NMR ($CDCl_3$, δ) : 0.88 (3H, t, J=7Hz), 1.06-1.43 (22H, m), 1.50-2.10 (6H, m), 2.27-2.69 (4H, m), 4.41 (1H, m), 5.34 (1H, br s), 5.90 (1H, d, J=8Hz), 6.12 (1H, br s), 7.09 (1H, br s), 7.12 (2H, d, J=8Hz), 7.21 (1H, t, J=8Hz), 7.36 (2H, t, J=8Hz)

Example 21

(3S)-3-[(2S)-5-Carboxy-2-{3-(2-pyridyl)ureido}pentanoyl]oxyhexadecanamide

NMR ($CDCl_3$-$CD_3OD$ (20:1), δ) : 0.88 (3H, t, J=7Hz), 1.13-1.43 (22H, m), 1.55-2.07 (6H, m), 2.37 (2H, m), 2.49 (2H, d, J=7Hz), 4.54 (1H, dd, J=6Hz, 7Hz), 5.26 (1H, m), 7.92 (1H, dd, J=5Hz, 7Hz), 7.03 (1H, d, J=8Hz), 7.62 (1H, dd, J=7Hz, 8Hz), 8.21 (1H, d, J=5Hz)

Example 22

(3S)-3-[(2S)-5-Carboxy-2-{3-(2-pyridylmethyl)ureido}pentanoyl]oxyhexadecanamide

NMR ($CDCl_3$-$CD_3OD$ (10:1), δ) : 0.88 (3H, t, J=7Hz), 1.07-1.40 (22H, m), 1.46-1.93 (6H, m), 2.05-2.56 (4H, m), 4.40 (1H, m), 4.45 (2H, s), 5.23 (1H, m), 7.22 (1H, dd, J=5Hz, 8Hz), 7.34 (1H, d, J=8Hz), 7.70 (1H, t, J=8Hz), 8.48 (1H, d, J=5Hz)

Example 23

(3S)-3-[(2S)-5-Carboxy-2-{(5-dimethylamino-1-naphthyl)sulfonylamino)pentanoyl]oxyhexadecanamide

NMR ($CDCl_3$, δ) : 0.89 (3H, t, J=7Hz), 0.95-1.70 (28H, m), 2.20-2.36 (4H, m), 2.87 (6H, s), 3.89 (1H, m), 5.03 (1H, m), 5.93 (1H, br s), 6.04 (1H, br s), 6.82 (1H, br s), 7.18 (1H, d, J=8Hz), 7.42-7.61 (2H, m), 8.21 (1H, d, J=7Hz), 8.28 (1H, d, J=8Hz), 8.53 (1H, d, J=8Hz)

Example 24

(3S)-N-Methyl-3-[(2S)-5-carboxy-2-(tertiarybutoxycarbonylamino)pentanoyl]oxyhexadecanamide

NMR ($CDCl_3$, δ) : 0.88 (3H, t, J=6Hz), 1.15-1.37 (22H, m), 1.44 (9H, s), 1.54-2.43 (6H, m), 2.29-2.51 (4H, m), 2.78 (3H, d, J=4Hz), 4.25 (1H, m), 5.14 (1H, d, J=7Hz), 5.24 (1H, m), 6.13 (1H, br s)

Example 25

(3S)-N-Benzyl-3-[(2S)-5-carboxy-2-(tertiarybutoxycarbonylamino)pentanoyl]oxyhexadecanamide

NMR ($CDCl_3$, δ) : 0.87 (3H, t, J=6Hz), 1.43 (9H, s), 1.15-1.83 (28H, m), 2.29 (2H, m), 2.48 (2H, d, J=7Hz), 4.16 (1H, m), 4.41 (2H, m), 5.06 (1H, d, J=8Hz), 5.26 (1H, m), 6.37 (1H, m), 7.16-7.39 (5H, m)

Example 26

To a solution of (3S)-3-[(2S)-5-benzyloxycarbonyl-2-aminopentanoyl]oxyhexadecanamide hydrochloride (1.79 g), 3-quinolinecarboxylic acid (630 mg) and 1-hydroxybenzotriazole (536 mg) in N,N-dimethylformamide (40 ml) was added 1-ethyl-3-(3-dimethylaminopropyl)carbodiimide (616 mg) at 0°C, and the mixture was stirred at room temperature for 2 hours. The solution was diluted with ethyl acetate (200 ml) and washed with 0.5N hydrochloric acid (200 ml), 10% aqueous citric acid (200 ml), saturated aqueous sodium bicarbonate (200 ml) and brine, successively. The organic layer was dried over magnesium sulfate and evaporated in vacuo to give (3S)-3-[(2S)-5-benzyloxycarbonyl-2-{(3-quinolyl)carbonylamino}pentanoyl] oxyhexadecanamide (2.16 g).

mp : 85-87°C

NMR ($CDCl_3$, δ) : 9.35 (1H, s), 8.62 (1H, s), 8.16 (1H, d, J=10Hz), 7.90 (1H, d, J=10Hz); 7.32 (1H, t, J=10Hz), 7.12 (1H, t, J=10Hz), 7.28-7.40 (5H, m), 5.92 (1H, br s), 5.46 (1H, br s), 5.35 (1H, m), 5.12 (2H, s), 4.30 (1H, m), 2.40-2.54 (4H, m), 1.6-2.1 (6H, m), 1.1-1.4 (22H, m), 0.88 (3H, t, J=7Hz)

FAB-MS : 660 (M+H)

Example 27

To a solution of (3S)-3-[(2S)-5-benzyloxycarbonyl-2-{(3-quinolyl)carbonylamino}pentanoyl]-oxyhexadecanamide (1.97 g) and anisole (9.73 ml) in dichloromethane (40 ml) was added dropwise aluminum chloride (3.98 g) in nitromethane (40 ml) at 0°C, and the mixture was stirred at room temperature for 2 hours. The solution was diluted with ethyl acetate (200 ml) and washed with 10% aqueous citric acid (200 ml), water (20 ml) and brine (200 ml), successively. The organic layer was dried over magnesium sulfate and evaporated in vacuo. The resulting crystalline was triturated with diethyl ether to give (3S)-3-[(2S)-5-carboxy-2-{(3-quinolyl)carbonylamino}pentanoyl] oxyhexadecanamide (2.16 g).

mp : 113-120°C

NMR (DMSO-$d_6$, δ) : 9.30 (1H, d, J=2Hz), 9.10 (1H, d, J=7Hz), 8.86 (1H, d, J=2Hz), 8.10 (1H, d, J=5Hz), 8.07 (1H, d, J=5Hz), 7.87 (1H, t, J=7Hz), 7.70 (1H, t, J=7Hz), 7.35 (1H, s), 6.84 (1H, s), 5.15 (1H, m), 4.42 (1H m), 2.2-2.45 (4H, m), 1.4-1.9 (6H, m), 1.1-1.35 (22H, m), 0.85 (3H, t, J=7Hz)

FAB-MS : 570 (M+H)

Example 28

To a solution of (3S)-3-[(2S)-5-benzyloxycarbonyl-2-aminopentanoyl]oxyhexadecanamide hydrochloride (0.20 g) and 2-pyridinecarbaldehyde (79.2 mg) in methanol (4 ml) was added sodium cyanoborohydride (46.4 mg) at room temperature and the mixture was stirred at the same temperature for 30 minutes. The solution was diluted with ethyl acetate (20 ml) and washed with saturated aqueous sodium bicarbonate (20 ml x 2) and brine (20 ml x 2), successively. The organic layer was dried over magnesium sulfate and concentrated in vacuo. The residue was purified by thin layered chromatography (10% methanol in chloroform) to give (3S)-3-[(2S)-5-benzyloxycarbonyl-2-(2-pyridylmethylamino)pentanoyl]-oxyhexadecanamide (0.25 g).

NMR ($CDCl_3$, δ) : 8.52 (1H, dd, J=5.2Hz), 7.64 (1H, dd, J=7.5Hz), 7.3-7.4 (6H, m), 6.16 (1H, br s), 5.38 (1H, br s), 5.26 (1H, m), 5.10 (2H, s), 3.85 (2H, ABq), 3.30 (1H, m), 2.50 (2H, d, J=7Hz), 2.38 (2H, m), 1.4-1.9 (6H, m), 1.2-1.4 (22H, m), 0.90 (3H, t, J=7Hz)

Example 29

(3S)-3-[(2S)-5-Benzyloxycarbonyl-2-(phenoxycarbonylamino)pentanoyl]oxyhexadecanenitrile was obtained according to a similar manner to that of Example 14.

NMR ($CDCl_3$, δ) : 7.31-7.44 (7H, m), 7.21 (1H, t, J=7Hz), 7.14 (2H, d, J=8Hz), 5.60 (1H, d, J=7Hz), 5.13 (2H, s), 5.06 (1H, m), 4.44 (1H, m), 2.74 (1H, dd, J=16Hz, 5Hz), 2.60 (1H, dd, J=16Hz, 6Hz), 2.45 (2H, m), 1.61-2.05 (6H, m), 1.15-1.41 (22H, m), 0.88 (3H, t, J=7Hz)

Example 30

(3R)-3-[(2R)-5-Benzyloxycarbonyl-2-{3-(2-pyridyl)ureido}pentanoyl]oxyhexadecanamide was obtained according to a similar manner to that of Example 18.

NMR ($CDCl_3$, δ) : 8.48 (1H, s), 8.19 (1H, d, J=5Hz), 7.60 (1H, dd, J=7Hz, 5Hz), 7.27-7.42 (5H, m), 6.89 (1H, dd, J=5Hz, 5Hz), 6.82 (1H, d, J=7Hz), 6.30 (1H, br s), 6.08 (1H, br s), 5.22 (1H, m), 5.12 (2H, s), 4.54 (1H, m), 2.33-2.57 (4H, m), 1.48-2.12 (6H, m), 1.00-1.40 (22H, m), 0.87 (3H, t, J=7Hz)

Example 31

To a solution of (3S)-(2,2,2-trichloroethyl) 3-[(2S)-5-benzyloxycarbonyl-2-{3-methyl-3-(2-pyridyl)ureido}pentanoyl]oxyhexadecanoate (240 mg) in aqueous 80% acetic acid was added zinc powder (203 mg). This mixture was stirred at room temperature for an hour. Then, the insoluble powder was filtered off with celite. After evaporation, the residue was dissolved in ethyl acetate (20 ml) and washed with water (20 ml x 2) and brine (20 ml). This solution was dried over magnesium sulfate and evaporated to dryness, and redissolved in tetrahydrofuran (2 ml). To this solution was added 1-ethyl-3-(3-dimethylaminopropyl)carbodiimide hydrochloride (50 mg) and 1-hydroxybenzotriazole (71 mg). After stirring for 30 minutes, 28% ammonium hydroxide (90 µl) was added and the mixture was stirred at room temperature overnight. Then, the mixture was diluted with ethyl acetate (20 ml) and washed with 1N-hydrochloric acid (20 ml x 2), saturated sodium bicarbonate (20 ml), and brine (20 ml). After drying over magnesium sulfate and evaporation, the residue was chromatographed on a silica gel to give (3S)-3-[(2S)-5-benzyloxycarbonyl-2-{3-methyl-3-(2-pyridyl)ureido}pentanoyl]oxyhexadecanamide (145 mg).

NMR ($CDCl_3$, δ) : 8.29 (1H, m), 7.72 (1H, m), 7.34 (5H, m), 6.92-7.03 (2H, m), 6.20 (1H, m), 5.17-5.28 (2H, m), 5.10 (2H, s), 4.52 (1H, m), 3.38 (3H, s), 2.38-2.52 (4H, m), 1.48-2.03 (6H, m), 1.14-1.36 (22H, m), 8.88 (3H, t, J=7Hz)

The following compounds (Examples 32 to 42) were obtained according to a similar manner to that of Example 31.

Example 32

(3S)-3-[(2S)-5-Benzyloxycarbonyl-2-(piperidinocarbonylamino)pentanoyl]oxyhexadecanamide

NMR ($CDCl_3$, δ) : 7.35 (5H, m), 6.21 (1H, br s), 5.28 (1H, br s), 5.22 (1H, br s), 5.11 (2H, s), 4.40 (1H, m), 3.34 (4H, m), 2.31-2.53 (4H, m), 1.42-1.95 (12H, m), 1.06-1.34 (22H, m), 0.87 (3H, t, J=7Hz)

Example 33

(3S)-3-[(2S)-5-Benzyloxycarbonyl-2-{4-(3,4-methylenedioxybenzyl)-1-piperazinylcarbonylamino}pentanoyl] oxyhexadecanamide

NMR ($CDCl_3$, δ) : 7.35 (5H, m), 6.86 (1H, s), 6.77 (1H, d, J=9Hz), 6.73 (1H, d, J=9Hz), 6.11 (1H, br s), 5.95 (2H, s), 5.25 (1H, br s), 5.23 (1H, m), 5.17 (1H, d, J=7Hz), 5.11 (2H, s), 4.39 (1H, m), 3.33-3.46 (6H, m), 2.32-2.50 (8H, m), 1.53-1.93 (6H, m), 1.14-1.37 (22H, m), 0.88 (3H, t, J=7Hz)

Example 34

(3S)-3-[(2S)-5-Benzyloxycarbonyl-2-(morpholinocarbonylamino)pentanoyl]oxyhexadecanamide

NMR ($CDCl_3$, δ) : 7.35 (5H, m), 6.01 (1H, br s), 5.19-5.34 (3H, m), 5.12 (2H, s), 4.42 (1H, m), 3.68 (4H, m), 3.37 (4H, m), 2.37-2.53 (4H, m), 1.50-1.95 (6H, m), 1.16-1.37 (22H, m), 0.88 (3H, t, J=7Hz)

Example 35

(3S)-3-[(2S)-5-Benzyloxycarbonyl-2-{3-((2S)-2-hydroxypropyl)ureido}pentanoyl]oxyhexadecanamide

NMR (DMSO-$d_6$, δ) : 7.35 (5H, m), 6.83 (1H, br s), 6.35 (1H, d, J=8Hz), 6.04 (1H, t, J=6Hz), 5.08 (2H, s), 5.08 (1H, m), 4.09 (1H, m), 3.57 (1H, m), 3.00 (1H, m), 2.85 (1H, m), 2.23-2.40 (4H, m), 1.36-1.71 (6H, m), 1.08-1.31 (22H, m), 0.98 (3H, d, J=7Hz), 0.84 (3H, t, J=7Hz)

Example 36

(3S)-3-[(2S)-5-Benzyloxycarbonyl-2-(3-morpholinoureido)pentanoyl]oxyhexadecanamide

NMR ($CDCl_3$, δ) : 7.35 (5H, m), 6.53 (1H, d, J=8Hz), 5.95 (1H, br s), 5.32 (1H, br s), 5.23 (1H, m), 5.11 (2H, s), 4.42 (1H, m), 3.58-3.94 (4H, m), 2.35-3.11 (8H, m), 1.52-2.00 (6H, m), 1.16-1.38 (22H, m), 0.88 (3H, t, J=7Hz)

Example 37

(3S)-3-[(2S)-5-Benzyloxycarbonyl-2-{3-(5-chloro-2-pyridyl)ureido}pentanoyl]oxyhexadecanamide

NMR ($CDCl_3$, δ) : 9.51 (1H, d, J=7Hz), 8.85 (1H, br s), 8.16 (1H, d, J=2Hz), 7.55 (1H, dd, J=8Hz, 2Hz), 7.34 (5H, m), 6.85 (1H, d, J=8Hz), 6.30 (1H, br s), 6.24 (1H, br s), 5.20 (1H, m), 5.12 (2H, s), 4.51 (1H, m), 2.47-2.57 (2H, m), 2.44 (2H, t, J=7Hz), 1.52-2.06 (6H, m), 1.10-1.36 (22H, m), 0.88 (3H, t, J=7Hz)

Example 38

(3S)-3-[(2S)-5-Benzyloxycarbonyl-2-{3-(5-methyl-2-pyridyl)ureido}pentanoyl]oxyhexadecanamide

NMR ($CDCl_3$, δ) : 8.02 (1H, d, J=2Hz), 7.89 (1H, br s), 7.42 (1H, dd, J=8Hz, 2Hz), 7.35 (5H, m), 6.67 (1H, d, J=8Hz), 6.29 (1H, br s), 5.86 (1H, br s), 5.20 (1H, m), 5.12 (2H, s), 4.53 (1H, m), 2.39-2.52 (4H, m), 2.25 (3H, s), 1.48-2.08 (6H, m), 1.12-1.37 (22H, m), 0.87 (3H, t, J=7Hz)

Example 39

(3S)-3-[(2S)-5-Benzyloxycarbonyl-2-{3-(2-quinolyl)ureido}pentanoyl]oxyhexadecanamide

NMR (DMSO-$d_6$, δ) : 9.99 (1H, s), 8.24 (1H, d, J=8Hz), 7.84 (1H, d, J=8Hz), 7.75 (1H, d, J=8Hz), 7.65 (1H, dd, J=8Hz, 7Hz), 7.43 (1H, dd, J=8Hz, 7Hz), 7.37 (1H, br s), 7.30 (5H, m), 6.86 (1H, br s), 5.18 (1H, m), 5.08 (2H, s), 4.39 (1H, m), 2.36-2.49 (4H, m), 1.47-1.97 (6H, m), 1.12-1.43 (22H, m), 0.85 (3H, t, J=7Hz)

Example 40

(3S)-3-[(2S)-5-Benzyloxycarbonyl-2-{3-(3-quinolyl)ureido}pentanoyl]oxyhexadecanamide

NMR (DMSO-$d_6$, δ) : 9.12 (1H, br s), 8.75 (1H, d, J=2Hz), 8.46 (1H, d, J=2Hz), 7.90 (1H, d, J=8Hz), 7.82 (1H, d, J=8Hz), 7.57 (1H, dd, J=8Hz, 6Hz), 7.52 (1H, dd, J=8Hz, 6Hz), 7.35 (5H, m), 6.85 (2H, m), 5.16 (1H, m), 5.10 (2H, s), 4.23 (1H, m), 2.27-2.49 (4H, m), 1.42-1.85 (6H, m), 0.98-1.37 (22H, m), 0.84 (3H, t, J=7Hz)

Example 41

(3S)-3-[(2S)-5-Benzyloxycarbonyl-2-{3-(2-benzothiazolyl)ureido}pentanoyl]oxyhexadecanamide

NMR ($CDCl_3$, δ) : 7.70 (1H, d, J=8Hz), 7.65 (1H, d, J=8Hz), 7.19-7.40 (7H, m), 6.52 (1H, br s), 6.10 (1H, br s), 5.37 (1H, m), 5.07 (2H, s), 4.53 (1H, m), 2.45-2.59 (2H, m), 2.40 (2H, t, J=7Hz), 1.49-2.05 (6H, m), 1.10-1.37 (22H, m), 0.86 (3H, t, J=7Hz)

Example 42

(3S)-3-[(2S)-5-Benzyloxycarbonyl-2-{3-(5-chloro-2-benzoxazolyl)ureido}pentanoyl]oxyhexadecanamide

NMR ($CDCl_3$, δ) : 9.21 (1H, br s), 9.11 (1H, d, J=7Hz), 7.52 (1H, d, J=2Hz), 7.28-7.37 (6H, m), 7.18 (1H, dd, J=8Hz, 2Hz), 6.22 (1H, br s), 6.12 (1H, br s), 5.25 (1H, m), 5.12 (2H, s), 4.53 (1H, m), 2.54 (2H, m), 2.47 (2H, t, J=7Hz),

1.60-2.06 (6H, m), 1.16-1.37 (22H, m), 0.87 (3H, t, J=7Hz)

Example 43

A solution of (3S)-(2,2,2-trichloroethyl) 3-[(2S)-5-benzyloxycarbonyl-2-isocyanatopentanoyl]oxyhexadecanoate (0.33 g) and 2-(methylamino)pyridine (113 mg) in dichloromethane (5 ml) was stirred at room temperature overnight. The mixture was poured into ethyl acetate (20 ml), and washed with aqueous IN-hydrochloric acid (20 ml x 2), saturated sodium bicarbonate (20 ml), and brine (20 ml). Then, the solution was dried over magnesium sulfate, and evaporated to dryness. The residue was chromatographed on a silica gel to give (3S)-(2,2,2-trichloroethyl) 3-[(2S)-5-benzyloxycarbonyl-2-(3-methyl-3-(2-pyridyl)ureido}pentanoyl]oxyhexadecanoate (0.25 g).

NMR ($CDCl_3$, δ) : 8.28 (1H, m), 7.70 (1H, m), 7.34 (5H, m), 6.91-7.01 (2H, m), 5.30 (1H, m), 5.11 (2H, s), 4.76 (1H, d, J=12Hz), 4.70 (1H, d, J=12Hz), 4.58 (1H, m), 3.38 (3H, s), 2.78 (1H, dd, J=16Hz, 7Hz), 2.71 (1H, dd, J=16Hz, 6Hz), 2.51 (2H, t, J=7Hz), 1.51-1.99 (6H, m), 1.12-1.38 (22H, m), 0.87 (3H, t, J=7Hz)

The following compounds (Examples 44 to 54) were obtained according to a similar manner to that of Example 43.

Example 44

(3S)-(2,2,2-Trichloroethyl) 3-[(2S)-5-benzyloxycarbonyl-2-{3-((2S)-2-hydroxypropyl)ureido}pentanoyl] oxyhexadecanoate

NMR ($CDCl_3$, δ) : 7.37 (5H, m), 5.29 (1H, m), 5.12 (2H, s), 4.77 (1H, d, J=12Hz), 4.71 (1H, d, J=12Hz), 4.43 (1H, m), 3.91 (1H, m), 3.31 (1H, m), 3.05 (1H, m), 2.74 (2H, m), 2.38 (2H, m), 1.45-1.90 (6H, m), 1.20-1.37 (22H, m), 1.18 (3H, d, J=7Hz), 0.88 (3H, t, J=7Hz)

Example 45

(3S)-(2,2,2-Trichloroethyl) 3-[(2S)-5-benzyloxycarbonyl-2-{3-(5-chloro-2-pyridyl)ureido}pentanoyl]oxyhexadecanoate

NMR ($CDCl_3$, δ) : 9.48 (1H, d, J=7Hz), 8.20 (1H, d, J=2Hz), 7.77 (1H, s), 7.55 (1H, dd, J=8Hz), 7.34 (5H, m), 6.75 (1H, d, J=8Hz), 5.32 (1H, m), 5.11 (2H, s), 4.76 (1H, d, J=12Hz), 4.70 (1H, d, J=12Hz), 4.62 (1H, m), 2.75 (2H, m), 2.42 (2H, t, J=7Hz), 1.61-2.07 (6H, m), 1.14-1.40 (22H, m), 0.88 (3H, t, J=7Hz)

Example 46

(3S)-(2,2,2-Trichloroethyl)3-[(2S)-5-benzyloxycarbonyl-2-{3-(5-methyl-2-pyridyl)ureido}pentanoyl]oxyhexadecanoate

NMR ($CDCl_3$, δ) : 8.04 (1H, d, J=2Hz), 7.40 (1H, dd, J=8Hz, 2Hz), 7.33 (5H, m), 6.98 (1H, s), 6.56 (1H, d, J=8Hz), 5.31 (1H, m), 5.10 (2H, s), 4.75 (1H, d, J=12Hz), 4.70 (1H, d, J=12Hz), 4.62 (1H, m), 2.78 (1H, dd, J=16Hz, 7Hz), 2.21 (1H, dd, J=16Hz, 6Hz), 2.42 (2H, t, J=7Hz), 2.25 (3H, s), 1.60-2.02 (6H, m), 1.14-1.40 (22H, m), 0.88 (3H, t, J=7Hz)

Example 47

(3S)-(2,2,2-Trichloroethyl) 3-[(2S)-5-benzyloxycarbonyl-2-{3-(2-quinolyl)ureido}pentanoyl]oxyhexadecanoate

NMR ($CDCl_3$, δ) : 8.03 (1H, d, J=8Hz), 7.88 (1H, d, J=8Hz), 7.70 (1H, d, J=8Hz), 7.59-7.68 (2H, m), 7.42 (1H, dd, J=7Hz, 8Hz), 7.30 (5H, m), 6.80 (1H, d, J=8Hz), 5.36 (1H, m), 5.10 (2H, s), 4.76 (1H, d, J=12Hz), 4.71 (1H, d,

J=12Hz), 4.69 (1H, m), 2.80 (1H, dd, J=16Hz, 7Hz), 2.73 (1H, dd, J=16Hz, 6Hz), 2.46 (2H, t, J=7Hz), 1.60-2.09 (6H, m), 1.09-1.40 (22H, m), 0.86 (3H, t, J=7Hz)

Example 48

(3S)-(2,2,2-Trichloroethyl) 3-[(2S)-5-benzyloxycarbonyl-2-{3-(3-quinolyl)ureido}pentanoyl]oxyhexadecanoate

NMR ($CDCl_3$, δ) : 9.14 (1H, br s), 8.75 (1H, d, J=3Hz), 8.44 (1H, d, J=3Hz), 7.89 (1H, d, J=8Hz), 7.80 (1H, d, J=8Hz), 7.46-7.60 (2H, m), 7.35 (5H, m), 6.91 (1H, d, J=8Hz), 5.20 (1H, m), 5.10 (2H, s), 4.86 (1H, d, J=12Hz), 4.81 (1H, d, J=12Hz), 4.21 (1H, m), 2.87 (1H, dd, J=16Hz, 6Hz), 2.76 (1H, dd, J=16Hz, 7Hz), 2.41 (2H, t, J=7Hz), 1.50-1.84 (6H, m), 1.02-1.38 (22H, m), 0.84 (3H, t, J=7Hz)

Example 49

(3S)-(2,2,2-Trichloroethyl)3-[(2S)-5-benzyloxycarbonyl-2-{3-(2-benzothiazolyl)ureido}pentanoyl]oxyhexadecanoate

NMR ($CDCl_3$, δ) : 7.75 (1H, d, J=8Hz), 7.70 (1H, d, J=8Hz) 7.27-7.42 (7H, m), 5.34 (1H, m), 5.19 (2H, s), 4.75 (1H, d, J=12Hz), 4.70 (1H, d, J=12Hz), 4.52 (1H, m), 2.75 (2H, m), 2.42 (2H, t, J=7Hz), 1.50-2.06 (6H, m), 1.14-1.40 (22H, m), 0.87 (3H, t, J=7Hz)

Example 50

(3S)-(2,2,2-Trichloroethyl) 3-[(2S)-5-benzyloxycarbonyl-2-{3-(5-chloro-2-benzoxazolyl)ureido}pentanoyl]-oxyhexadecanoate

NMR ($CDCl_3$, δ) : 9.06 (1H, d, J=8Hz), 8.10 (1H, s), 7.53 (1H, d, J=2Hz), 7.27-7.38 (6H, m), 7.18 (1H, dd, J=8Hz, 2Hz), 5.35 (1H, m), 5.11 (2H, s), 4.75 (1H, d, J=12Hz), 4.70 (1H, d, J=12Hz), 4.61 (1H, m), 2.77 (2H, m), 2.43 (2H, t, J=7Hz), 1.59-2.09 (6H, m), 1.15-1.44 (22H, m), 0.87 (3H, t, J=7Hz)

Example 51

(3S)-(2,2,2-Trichloroethyl) 3-[(2S)-5-benzyloxycarbonyl-2-(3-morpholinoureido)pentanoyl]oxyhexadecanoate

NMR ($CDCl_3$, δ) : 7.34 (5H, m), 6.50 (1H, d, J=8Hz), 5.28 (1H, m), 5.16 (1H, br s), 5.10 (2H, s), 4.77 (1H, d, J=12Hz), 4.70 (1H, d, J=12Hz), 4.47 (1H, m), 3.60-3.92 (4H, m), 2.48-3.13 (4H, m), 2.38 (2H, m), 1.58-1.94 (6H, m), 1.16-1.40 (22H, m), 0.87 (3H, t, J=7Hz)

Example 52

(3S)-(2,2,2-Trichloroethyl) 3-[(2S)-5-benzyloxycarbonyl-2-(piperidinocarbonylamino)pentanoyl]oxyhexadecanoate

NMR ($CDCl_3$, δ) : 7.35 (5H, m), 5.29 (1H, m), 5.11 (2H, s), 5.02 (1H, d, J=7Hz), 4.77 (1H, d, J=12Hz), 4.70 (1H, d, J=12Hz), 4.49 (1H, m), 3.33 (4H, m), 2.77 (1H, dd, J=15Hz, 7Hz), 2.70 (1H, dd, J=15Hz, 6Hz), 2.39 (2H, t, J=7Hz), 1.48-1.96 (12H, m), 1.15-1.43 (22H, m), 0.88 (3H, t, J=7Hz)

Example 53

(3S)-(2,2,2-Trichloroethyl) 3-[(2S)-5-benzyloxycarbonyl-2-{4-(3,4-methylenedioxybenzyl-1-piperazinylcarbonylamino}-pentanoyl]oxyhexadecanoate

NMR ($CDCl_3$, δ) : 7.34 (5H, m), 6.85 (1H, s), 6.76 (1H, d, J=9Hz), 6.73 (1H, d, J=9Hz), 5.95 (2H, s), 5.28 (1H, m), 5.10 (1H, s), 5.04 (1H, d, J=8Hz), 4.77 (1H, d, J=12Hz), 4.70 (1H, d, J=12Hz), 3.32-3.46 (6H, m), 2.73 (2H, m), 2.31-2.46 (6H, m), 1.55-1.92 (6H, m), 1.13-1.41 (22H, m), 0.88 (3H, t, J=7Hz)

Example 54

(3S)-2,2,2-Trichloroethyl) 3-[(2S)-5-benzyloxycarbonyl-2-(morpholinocarbonylamino)pentanoyl]oxyhexadecanoate

NMR ($CDCl_3$, δ) : 7.35 (5H, m), 5.29 (1H, m), 5.21 (2H, s), 5.08 (1H, d, J=7Hz), 4.77 (1H, d, J=12Hz), 4.70 (1H, d, J=12Hz), 4.47 (1H, m), 3.69 (4H, m), 3.37 (4H, m), 2.73 (2H, m), 2.38 (2H, m), 1.50-1.90 (6H, m), 1.15-1.27 (22H, m), 0.88 (3H, t, J=7Hz)

Example 55

(3R)-3-[(2R)-5-Benzyloxycarbonyl-2-(phenoxycarbonylamino)pentanoyl]oxyhexadecanamide was obtained according to a similar manner to that of Example 14.

NMR ($CDCl_3$, δ) : 7.29-7.52 (7H, m), 7.22 (1H, dd, J=7Hz, 7Hz), 7.13 (2H, d, J=8Hz), 5.76 (1H, br s), 5.69 (1H, d, J=7Hz), 5.30 (1H, m), 5.12 (2H, s), 4.38 (1H, m), 1.54-2.03 (6H, m), 1.12-1.48 (22H, m), 0.87 (3H, t, J=7Hz)

The following compounds (Examples 56 to 74) were obtained according to a similar manner to that of Example 1.

Example 56

(3S)-3-[6-Benzyloxycarbonyl-2-(tert-butoxycarbonylamino)hexanoyl]oxyhexadecanamide

NMR ($CDCl_3$, δ) : 7.32 (5H, m), 6.32 & 5.96 (1H, br s), 5.58 & 5.50 (1H, br s), 4.95-5.30 (1H, m), 5.10 (2H, s), 4.0-4.3 (1H, m), 2.3-2.6 (4H, m), 1.6-1.9 (8H, m), 1.40 & 1.45 (9H, s), 1.2-1.4 (22H, m), 0.88 (3H, t, J=7Hz)

Example 57

2-[(2S)-5-Benzyloxycarbonyl-2-(tertiarybutoxycarbonylamino)pentanoyl]oxypentadecanamide

NMR ($CDCl_3$, δ) : 7.36 (5H, m), 7.01· 6.96 (sum 1H, br s), 5.26 (1H, br s), 5.18 (1H, m), 5.12 (2H, s), 5.04 (1H, m), 4.23 4.11 (sum 1H, m), 2.43 (2H, m), 1.64-1.98 (4H, m), 1.46 · 1.41 (sum 9H, s), 1.14-1.38 (24H, m), 0.87 (3H, t, J=7Hz)

Example 58

3-[(2S)-5-Benzyloxycarbonyl-2-(tertiarybutoxycarbonylamino)pentanoyl]oxydecanamide

NMR ($CDCl_3$, δ) : 7.35 (5H, m), 6.28 · 5.94 (sum 1H, br s), 5.40 (1H, br s), 5.25 5.15 (sum 1H, m), 5.11 (2H, s), 5.06 (1H, m), 4.23 4.13 (sum 1H, m), 2.30-2.51 (4H, m), 1.55-1.93 (6H, m), 1.43 · 1.42 (sum 9H, s), 1.15-1.38 (10H, m), 0.87 (3H, t, J=7Hz)

Example 59

3-[(2S)-5-Benzyloxycarbonyl-2-(tertiarybutoxycarbonylamino)pentanoyl]oxydodecanamide

NMR ($CDCl_3$, δ) : 7.36 (5H, m), 6.28 5.93 (sum 1H, m), 5.27 (1H, m), 5.11 (2H, s), 4.95-5.12 (1H, m), 4.23 4.13 (sum 1H, m), 2.32-2.51 (4H, m), 1.49-1.95 (6H, m), 1.46 · 1.45 (sum 9H, s), 1.08-1.40 (14H, m), 0.88 (3H, t, J=7Hz)

Example 60

3-[(2S)-5-Benzyloxycarbonyl-2-(tertiarybutoxycarbonylamino)pentanoyl]oxytetradecanamide

NMR ($CDCl_3$, δ) : 7.35 (5H, m), 6.27 5.92 (sum 1H, br s), 5.27 (1H, br s), 5.24 (1H, m), 5.12 (2H, s), 4.98-5.17 (1H, m), 4.23 4.12 (sum 1H, m), 2.32-2.50 (4H, m), 1.54-1.92 (6H, m), 1.43 · 1.41 (sum 9H, s), 1.13-1.38 (18H, m), 0.88 (3H, t, J=7Hz)

Example 61

(3S)-3-[(2S)-5-Benzyloxycarbonyl-2-(tertiarybutoxycarbonylamino)pentanoyl]oxybutyramide

NMR ($CDCl_3$, δ) : 7.36 (5H, m), 5.81 (1H, br s), 5.31 (2H, m), 5.12 (2H, s), 5.06 (1H, d, J=7Hz), 4.23 (1H, m), 2.35-2.55 (4H, m), 1.62-1.89 (4H, m), 1.44 (9H, s), 1.34 (3H, t, J=7Hz)

Example 62

(3R)-3-[(2R)-5-Benzyloxycarbonyl-2-(tertiarybutoxycarbonylamino)pentanoyl]oxyhexadecanamide

NMR (DMSO-$d_6$, δ) : 7.35 (5H, m), 7.22 (1H, d, J=8Hz), 6.84 (1H, br s), 5.07 (3H, m), 3.84 (1H, br s), 2.31 (4H, m), 1.41-1.70 (4H, m), 1.37 (9H, s), 1.05-1.32 (24H, m), 0.85 (3H, t, J=7Hz)

Example 63

(3R)-3-[(2S)-5-Benzyloxycarbonyl-2-(tertiarybutoxycarbonylamino)pentanoyl]oxyhexadecanamide

NMR ($CDCl_3$, δ) : 7.35 (5H, m), 6.28 (1H, br s), 5.32 (1H, br s), 5.13 (1H, m), 5.11 (1H, s), 5.02 (1H, d, J=7Hz), 4.12 (1H, m), 2.35-2.62 (4H, m), 1.57-1.91 (6H, m), 1.43 (9H, s), 1.18-1.37 (22H, m), 0.88 (3H, t, J=7Hz)

Example 64

(3S)-3-[(2)-5-Benzyloxycarbonyl-2-(tertiarybutoxycarbonylamino)pentanoyl]oxyhexadecanamide

NMR ($CDCl_3$, δ) : 7.35 (5H, m), 6.28 (1H, br s), 5.32 (1H, br s), 5.13 (1H, m), 5.11 (1H, s), 5.02 (1H, d, J=7Hz), 4.12 (1H, m), 2.35-2.62 (4H, m), 1.57-1.91 (6H, m), 1.43 (9H, s), 1.18-1.37 (22H, m), 0.88 (3H, t, J=7Hz)

Example 65

2-[(2S)-5-Benzyloxycarbonyl-2-(tertiarybutoxycarbonylamino)pentanoyl]oxy-N-methylpentadecanamide

NMR ($CDCl_3$, δ) : 7.35 (5H, m), 6.98 (1H, br s), 5.20 (1H, m), 5.13 5.11 (sum 2H, s), 5.06 5.01 (sum 1H, d, J=7Hz), 4.21·4.08 (sum 1H, m), 2.79 2.78 (sum 3H, d, J=5Hz), 2.42 (2H, m), 1.66-1.98 (6H, m), 1.46 · 1.43 (sum 9H, s), 1.15-1.37 (22H, m), 0.87 (3H, t, J=7Hz)

Example 66

(3S)-Benzyl 3-[(2S)-5-benzyloxycarbonyl-2-(tertiarybutoxycarbonylamino)pentanoyl)oxyhexadecanoate

NMR ($CDCl_3$, δ) : 7.35 (10H, m), 5.27 (1H, m), 5.11 (2H, s), 5.09 (2H, s), 4.99 (1H, d, J=8Hz), 4.22 (1H, m), 2.53-2.69 (2H, m), 2.33 (2H, m), 1.48-1.81 (6H, m), 1.43 (9H, s), 1.16-1.35 (22H, m), 0.88 (3H, t, J=7Hz)

Example 67

Methyl 2-[(2S)-5-benzyloxycarbonyl-2-(tertiarybutoxycarbonylamino)pentanoyl]oxypentadecanoate

NMR ($CDCl_3$, δ) : 7.36 (5H, m), 5.12 (2H, s), 5.03 (2H, m), 4.38 (1H, m), 3.75 · 3.71 (3H, s), 2.43 (2H, m), 1.63-2.05 (6H, m), 1.44 (9H, s), 1.16-1.48 (22H, m), 0.89 (3H, t, J=7Hz)

Example 68

(3S)-Methyl 3-[(2S)-5-benzyloxycarbonyl-2-(tertiarybutoxycarbonylamino)pentanoyl]oxyhexadecanoate

NMR ($CDCl_3$, δ) : 7.35 (5H, m), 5.29 (1H, m), 5.11 (2H, s), 5.01 (1H, m), 4.25 (1H, m), 3.67 (3H, s), 2.73 (2H, m), 2.48 (2H, ddd, J=6Hz, 6Hz, 3Hz), 1.52-1.88 (6H, m), 1.44 (9H, s), 1.16-1.48 (22H, m), 0.88 (3H, t, J=7Hz)

Example 69

(3S)-(2,2,2-Trichloroethyl)3-[(2S)-5-benzyloxycarbonyl-2-(tertiarybutoxycarbonylamino)pentanoyl]oxyhexadecanoate

NMR ($CDCl_3$, δ) : 7.35 (5H, m), 5.29 (1H, m), 5.11 (2H, s), 5.01 (1H, m), 4.76 (1H, d, J=12Hz), 4.70 (1H, d, J=12Hz), 4.25 (1H, m), 2.73 (2H, m), 2.48 (2H, ddd, J=6Hz, 6Hz, 3Hz), 1.52-1.88 (6H, m), 1.44 (9H, s), 1.16-1.48 (22H, m), 0.88 (3H, t, J=7Hz)

Example 70

(3S)-3-[(2S)-5-Benzyloxycarbonyl-2-(tertiarybutoxycarbonylamino)pentanoyl]aminohexadecanamide

NMR ($CDCl_3$, δ) : 7.35 (5H, m), 6.77 (1H, d, J=8Hz), 5.95 (1H, br s), 5.32 (1H, br s), 5.11 (2H, s), 5.06 (1H, d, J=7Hz), 4.15 (1H, m), 4.01 (1H, m), 2.32-2.52 (4H, m), 1.49-1.92 (6H, m), 1.43 (9H, s), 1.06-1.36 (22H, m), 0.88 (3H, t, J=7Hz)

Example 71

N-[(S)-5-Benzyloxycarbonyl-2-(tertiarybutoxycarbonylamino)pentanoyl]-N-(2-carbamoylethyl) tridecylamine

NMR ($CDCl_3$, δ) : 7.36 (5H, m), 6.64 · 6.20 (sum 1H, br s), 5.24 (2H, m), 5.12 (2H, s), 4.54 (1H, m), 3.86 · 3.62 (sum 2H, m), 3.50 · 3.30 (sum 2H, m), 2.33-2.53 (4H, m), 1.50-1.77 (4H, m), 1.43 (9H, s), 1.14-1.36 (22H, m), 0.90 (3H, t, J=7Hz)

Example 72

N-[(S)-5-Benzyloxycarbonyl-2-(tertiarybutoxycarbonylamino)pentanoyl]-N-(N-methylcarbamoylmethyl)tridecylamine

NMR ($CDCl_3$, δ) : 7.35 (5H, m), 6.64 (1H, br s), 5.17 · 5.09 (1H, d, J=7Hz), 5.11 (2H, s), 4.45 (1H, m), 4.29 4.07 (sum 1H, d, J=16Hz), 3.84 · 3.75 (sum 1H, d, J=16Hz), 3.50 (1H, m), 3.25 · 3.11 (sum 1H, m), 2.84 2.77 (sum 3H, d, J=5Hz), 2.40 (2H, m), 1.50-1.85 (4H, m), 1.42 (9H, s), 1.12-1.35 (22H, m), 0.87 (3H, t, J=7Hz)

The following compounds (Examples 73 to 93) were obtained according to a similar manner to that of Example 26.

Example 73

(3S)-3-[(2S)-5-Benzyloxycarbonyl-2-(2-biphenylcarbonylamino)pentanoyl]oxyhexadecanamide

NMR (DMSO-$d_6$, δ) : 8.62 (1H, d, J=10Hz), 7.2-7.6 (10H, m), 6.82 (1H, br s), 5.10 (2H, s), 4.18 (2H, s), 4.18 (1H, m), 2.2-2.35 (4H, m), 1.35-1.8 (6H, m), 1.2-1.35 (22H, m), 0.84 (3H, t, J=7Hz)·

FAB-MS : 685 [M+H]

Example 74

(3S)-3-[(2S)-5-Benzyloxycarbonyl-2-(N-tert-butoxycarbonyl-L-prolylamino)pentanoyl]oxyhexadecanamide

NMR ($CDCl_3$, δ) : 7.35 (5H, m), 6.36 (1H, br s), 5.44 (1H, br s), 5.15 (1H, m), 5.10 (2H, s), 4.50 (1H, m), 4.26 (1H, br s), 3.46 (2H, br s), 2.35-2.5 (4H, m), 1.5-1.95 (10H, m), 1.45 (9H, s), 1.2-1.35 (22H, m), 0.88 (3H, t, J=7Hz)

Example 75

(3S)-3-[(2S)-5-Benzyloxycarbonyl-2-(N-tert-butoxycarbonyl-D-prolylamino)pentanoyl]oxyhexadecanamide

NMR ($CDCl_3$, δ) : 7.35 (5H, m), 6.36 (1H, br s), 5.44 (1H, br s), 5.15 (1H, m), 5.10 (2H, s), 4.50 (1H, m), 4.26 (1H, br s), 3.46 (2H, br s), 2.35-2.5 (4H, m), 1.5-1.95 (10H, m), 1.45 (9H, s), 1.2-1.35 (22H, m), 0.88 (3H, t, J=7Hz)

Example 76

(3S)-3-[(2S)-5-Benzyloxycarbonyl-2-(N-tert-butoxycarbonyl-2-piperidylcarbonylamino)pentanoyl]-oxyhexadecanamide

NMR ($CDCl_3$, δ) : 7.34 (5H, m), 6.75 & 6.65 (1H, br s), 5.95 & 5.40 (1H, br s), 5.14 (1H, m), 5.10 (2H, s), 4.74 (1H, br), 4.53 (1H, m), 2.90 (1H, br), 2.2-2.5 (5H, m), 1.46 & 1.44 (9H, s), 1.35-2.0 (12H, m), 1.2-1.35 (22H, m), 0.88 (3H, t, J=7Hz)

Example 77

(3S)-3-[(2S)-5-Benzyloxycarbonyl-2-(N-tert-butoxycarbonyl-4-piperidylcarbonylamino)pentanoyl]-oxyhexadecanamide

NMR (DMSO-$d_6$, δ) : 8.14 (1H, d, J=10Kz), 7.34 (6H, m), 6.82 (1H, br s), 5.08 (2H, s), 5.08 (1H, m), 4.12 (1H, m), 3.92 (2H, d, J=10Hz), 2.1-2.8 (7H, m), 1.4-1.7 (10H, m), 1.35 (9H, s), 1.1-1.3 (22H, m), 0.84 (3H, t, J=7Hz)

Example 78

(3S)-3-[(2S)-5-Benzyloxycarbonyl-2-(6-methyl-2-pyridylcarbonylamino)pentanoyl]oxyhexadecanamide

NMR ($CDCl_3$, δ) : 8.60 (1H, d, J=10Hz), 7.94 (1H, d, J=10Hz), 7.72 (1H, t, J=10Hz), 7.35 (5H, m), 7.30 (1H, d, J=10Hz), 5.96 (1H, br s), 5.32 (2H, m), 5.12 (2H, s), 4.72 (1H, m), 2.62 (3H, s), 2.4-2.55 (4H, m), 1.1-2.1 (6H, m), 1.2-1.4 (22H, m), 0.88 (3H, t, J=7Hz)

FAB-MS : 624 [M+H]

Example 79

(3S)-3-[(2S)-5-Benzyloxycarbonyl-2-(nicotinoylamino)pentanoyl]oxyhexadecanamide

NMR ($CDCl_3$, δ) : 9.11 (1H, m), 8.76 (1H, m), 8.17 (1H, d, J=8Hz), 7.42 (1H, dd, J=8Hz, 5Hz), 7.22 (1H, d, J=7Hz), 5.87 (1H, br s), 5.45 (1H, br s), 5.32 (1H, m), 5.12 (2H, s), 4.72 (1H, m), 2.49-2.52 (4H, m), 1.57-2.09 (6H, m), 1.16-1.39 (22H, m), 0.87 (3H, t, J=7Hz)

Example 80

(3S)-3-[(2S)-5-Benzyloxycarbonyl-2-(isonicotinoylamino)pentanoyl]oxyhexadecanamide

NMR ($CDCl_3$, δ) : 8.76 (2H, d, J=6Hz), 7.67 (2H, d, J=6Hz), 7.35 (5H, m), 7.19 (1H, J=7Hz), 5.82 (1H, br s), 5.40 (1H, br s), 5.33 (1H, m), 5.12 (2H, s), 4.70 (1H, m), 2.32-2.52 (4H, m), 1.50-2.08 (6H, m), 1.08-1.35 (22H, m), 0.87 (3H, t, J=7Hz)

Example 81

(3S)-3-[(2S)-5-Benzyloxycarbonyl-2-(2-pyridylcarbonylamino)pentanoyl]oxyhexadecanamide

NMR ($CDCl_3$, δ) : 8.58 (1H, d, J=6Hz), 8.53 (1H, d, J=9Hz), 8.15 (1H, t, J=8Hz), 7.45 (1H, dd, J=8Hz, 6Hz), 7.34 (5H, m), 5.93 (1H, m), 5.28 (1H, m), 5.11 (2H, s), 4.72 (1H, m), 2.38-2.52 (4H, m), 1.49-2.12 (6H, m), 1.12-1.39 (22H, m), 0.87 (3H, m)

Example 82

(3S)-3-[(2S)-5-Benzyloxycarbonyl-2-(2-pyrazinylcarbonylamino)pentanoyl]oxyhexadecanamide

NMR ($CDCl_3$, δ) : 9.37 (1H, s), 8.77 (1H, d, J=4Hz), 8.55 (1H, d, J=4Hz), 8.33 (1H, d, J=10Kz), 7.36 (5H, m), 5.80 (1H, br s), 5.36 (1H, br s), 5.33 (1H, m), 5.10 (2H, s), 4.76 (1H, m), 2.38-2.52 (4H, m), 1.6-2.1 (6H, m), 1.2-1.4 (22H, m), 0.88 (3H, t, J=7Hz)

FAB-MS : 611 [M+H]

Example 83

(3S)-3-[(2S)-5-Benzyloxycarbonyl-2-{(5-quinolyl)carbonylamino}pentanoyl]oxyhexadecanamide

NMR ($DMSO-d_6$, δ) : 9.02 (1H, d, J=10Hz), 8.94 (1H, m), 8.64 (1H, d, J=10Hz), 8.12 (1H, d, J=10Hz), 7.75 (2H, ABq), 7.56 (1H, m), 7.38 (1H, br s), 7.35 (5H, m), 6.86 (1H, br s), 5.18 (1H, m), 5.08 (2H, s), 4.44 (1H, m), 2.3-2.5 (4H, m), 1.5-1.9 (6H, m), 1.1-1.4 (22H, m), 0.82 (3H, t, J=7Hz)

Example 84

(3R)-3-[(2R)-5-Benzyloxycarbonyl-2-{(3-quinolyl)carbonylamino}pentanoyl]oxyhexadecanamide

NMR ($CDCl_3$, δ) : 9.35 (1H, d, J=1.5Hz), 8.64 (1H, d, J=1.5Hz), 8.16 (1H, d, J=8Hz), 7.92 (1H, d, J=8Hz), 7.83 (1H, t, J=8Hz), 7.63 (1H, t, J=8Hz), 7.35 (5H, m), 5.95 (1H, br s), 5.50 (1H, br s), 5.35 (1H, m), 5.12 (2H, s), 4.78 (1H, m), 2.47 (4H, m), 1.57-2.12 (6H, m), 1.15-1.38 (22H, m), 0.87 (3H, t, J=7Hz)

Example 85

(3S)-3-[(2S)-5-Benzyloxycarbonyl-2-{(1-isoquinolyl)carbonylamino}pentanoyl]oxyhexadecanamide

NMR (DMSO-$d_6$, δ) : 9.10 (1H, d, J=10Hz), 8.85 (1H, d, J=10Hz), 8.54 (1H, d, J=7Hz), 8.04 (2H, m), 7.82 (1H, t, J=7Hz), 7.70 (1H, t, J=7Hz), 7.38 (1H, br s), 7.32 (5H, m), 6.86 (1H, br s), 5.18 (1H, m), 5.08 (2H, s), 4.45 (1H, m), 2.2-2.45 (4H, m), 1.45-1.95 (6H, m), 1.1-1.3 (22H, m), 0.84 (3H, t, J=7Hz)

FAB-MS : 660 [M+H]

Example 86

(3S)-3-[(2S)-5-Benzyloxycarbonyl-2-{(3-isoquinolyl)carbonylamino}pentanoyl]oxyhexadecanamide

NMR ($CDCl_3$, δ) : 9.20 (1H, s), 8.74 (1H, d, J=10Hz), 8.05 (1H, d, J=10Hz), 7.98 (1H, d, J=10Hz), 7.76 (2H, m), 7.34 (5H, m), 6.08 (1H, br s), 5.53 (1H, br s), 5.30 (1H, m), 5.12 (2H, s), 4.82 (1H, m), 2.3-2.6 (4H, m), 1.1-2.0 (6H, m), 1.1-1.4 (22H, m), 0.88 (3H, t, J=7Hz)

Example 87

(3S)-3-[(2S)-5-Benzyloxycarbonyl-2-{(2-quinoxalinyl)carbonylamino}pentanoyl]oxyhexadecanamide

NMR (DMSO-$d_6$, δ) : 9.45 (1H, s), 9.18 (1H, d, J=10Hz), 8.22 (2H, m), 8.00 (2H, m), 7.35 (1H, br s), 7.25-7.35 (5H, m), 6.85 (1H, br s), 5.15 (1H, m), 5.08 (2H, s), 4.50 (1H, m), 2.25-2.45 (2H, m), 1.90 (2H, m), 1.65 (2H, m), 1.50 (2H, m), 1.0-1.3 (22H, m), 0.84 (3H, t, J=7Hz)

Example 88

(3S)-3-[(2S)-5-Benzyloxycarbonyl-2-{((6S)-5-tert-butoxycarbonyl-3-tert-butoxycarbonyl-4,5,6,7-tetrahydro-3H-imidazo[4,5-c]pyridin-6-yl)carbonylamino}pentanoyl]-oxyhexadecanamide

NMR ($CDCl_3$, δ) : 8.00 (1H, s), 7.34 (5H, m), 6.70 (1H, br s), 5.85 (1H, br s), 5.30 (1H, br s), 5.20 (1H, m), 5.10 (2H, s), 4.42 (2H, br s), 3.3 (1H, m), 2.90 (1H, m), 2.2-2.5 (4H, m), 1.60 (9H, s), 1.50 (9H, s), 1.4-1.9 (8H, m), 1.15-1.35 (22H, m), 0.88 (3H, t, J=7Hz)

Example 89

(3S)-3-[(2S)-5-Benzyloxycarbonyl-2-{((6R)-5-tert-butoxycarbonyl-3-tert-butoxycarbonyl-4,5,6,7-tetrahydro-3H-imidazo[4,5-c]pyridin-6-yl)carbonylamino}pentanoyl]-oxyhexadecanamide

NMR ($CDCl_3$, δ) : 8.00 (1H, s), 7.34 (5H, m), 6.70 (1H, br s), 5.85 (1H, br s), 5.30 (1H, br s), 5.20 (1H, m), 5.10 (2H, s), 4.42 (2H, br s), 3.3 (1H, m), 2.90 (2H, m), 2.2-2.5 (4H, m), 1.60 (9H, s), 1.50 (9H, s), 1.4-1.9 (8H, m), 1.15-1.35 (22H, m), 0.88 (3H, t, J=7Hz)

Example 90

N-[(S)-5-Benzyloxycarbonyl-2-{(2-quinolyl)carbonyl-amino}pentanoyl]-N-(2-carbamoylethyl)tridecylamine

NMR ($CDCl_3$, δ) : 8.89 · 8.84 (sum 1H, d, J=8Hz), 8.31 (1H, d, J=8Hz), 8.26 (1H, d, J=8Hz), 8.17 (1H, d, J=8Hz), 7.88 (1H, d, J=8Hz), 7.78 (1H, m), 7.63 (1H, m), 7.34 (5H, m), 6.78 (1H, br s), 6.17 (1H, br s), 5.11 (1H, m), 5.10 (2H, s), 3.85 3.68 (sum 2H, m), 3.52 3.41 (sum 2H, m), 2.37-2.63 (4H, m), 1.62-2.00 (4H, m), 1.16-1.38 (22H, m), 0.88 (3H, t, J=7Hz)

Example 91

(3S)-3-[(2S)-5-Benzyloxycarbonyl-2-(5-imidazolylmethylcarbonylamino)pentanoyl]oxyhexadecanamide

NMR ($CDCl_3$, δ) : 7.60 (1H, s), 7.35 (5H, m), 6.90 (1H, s), 6.30 (1H, br s), 5.65 (1H, br s), 5.20 (1H, m), 5.10 (2H, s), 4.50 (1H, m), 3.57 (2H, ABq), 2.3-2.5 (4H, m), 1.5-1.9 (6H, m), 1.1-1.4 (22H, m), 0.88 (3H, t, J=7Hz)

Example 92

(3S)-3-[(2S)-5-Benzyloxycarbonyl-2-(2-pyridylmethylcarbonylamino)pentanoyl]oxyhexadecanamide

NMR ($CDCl_3$, δ) : 8.56 (1H, d, J=5Hz), 8.00 (1H, d, J=10Hz), 7.66 (1H, dd, J=10Hz, 5Hz), 7.35 (5H, m), 7.25 (1H, d, J=10Hz), 7.20 (1H, dd, J=10Kz, 5Hz), 6.00 (1H, br s), 5.36 (1H, br s), 5.20 (1H, m), 5.10 (2H, s), 4.50 (1H, m), 3.75 (2H, s), 2.3-2.45 (4H, m), 1.5-2.0 (6H, m), 1.1-1.35 (22H, m), 0.88 (3H, t, J=7Hz)

FAB-MS : 624 [M+H]

Example 93

(3S)-3-[(2S)-5-Benzyloxycarbonyl-2-(8-quinolylsulfonylamino)pentanoyl]oxyhexadecanamide

NMR (DMSO-$d_6$, δ) : 9.04 (1H, d, J=5Hz), 8.50 (1H, d, J=10Hz), 8.26 (2H, m), 7.65 (2H, q, J=10Hz), 7.35 (5H, m), 7.22 (1H, br s), 6.72 (1H, br s), 5.12 (2H, s), 4.72 (1H, m), 4.15 (1H, m), 2.0-2.2 (4H, m), 1.4-1.8 (6H, m), 0.9-1.3 (22H, m), 1.88 (3H, t, J=7Hz)

The following compounds (Examples 94 to 97) were obtained according to a similar manner to that of Preparation 4.

Example 94

(3S)-3-[(2S)-5-Carboxy-2-{(2-piperidyl)carbonylamino}-pentanoyl]oxyhexadecanamide hydrochloride [from (3S)-3-[(2S)-5-carboxy-2-{(N-t-butoxycarbonyl-2-piperidyl)carbonylamino}-pentanoyl]oxyhexadecanamide]

MMR ($CD_3OD$, δ) : 5.28 (1H, m), 4.40 (1H, m), 3.85 (1H, d, J=12Hz), 3.42 (1H, d, J=12Hz), 3.06 (1H, t, J=12Hz), 2.2-2.6 (5H, m), 1.6-2.0 (12H, m), 1.2-1.4 (22H, m), 0.90 (3H, t, J=7Hz)

Example 95

(3S)-3-[(2S)-5-Carboxy-2-{(4-piperidyl)carbonylamino}-pentanoyl]oxyhexadecanamide hydrochloride [from (3S)-3-[[2S)-5-carboxy-2-{(N-t-butoxycarbonyl-4-piperidyl)carbonylamino}pentanoyl]oxyhexadecanamide]

NMR (DMSO-$d_6$, δ) : 8.14 (1H, d, J=10Hz), 7.34 (1H, br s), 6.82 (1H, br s), 5.08 (1H, m), 4.12 (1H, m), 3.92 (2H, d, J=10Kz), 2.1-2.8 (7H, m), 1.4-1.7 (10H, m), 1.35 (9H, s), 1.1-1.3 (22H, m), 0.84 (3H, t, J=7Hz)

FAB-MS : 648 [M+Na]

Example 96

(3S)-3-[(2S)-5-Carboxy-2-{((6R)-4,5,6,7-tetrahydro-3H-imidazo[4,5-c]pyridin-6-yl)carbonylamino}pentanoyl]-oxyhexadecanamide dihydrochloride [from (3S)-3-[(2S)-5-carboxy-2-{((6R)-3-t-butoxycarbonyl-5-t-buoxycarbonyl4,5,6,7-tetrahydro-3H-imidazo [4,5-c]pyridin-6-yl)carbonylamino}pentanoyl]oxyhexadecanamide]

NMR ($CD_3OD$, δ) : 8.98 (1H, s), 5.30 (1H, m), 4.4-4.6 (3H, m), 3.08 (1H, dd, J=15Hz, 5Hz), 3.17 (1H, dd, J=15Hz, 10Hz), 2.52 (2H, m), 2.36 (2H, t, J=7Hz), 1.4-2.0 (6H, m), 1.2-1.4 (22H, m), 0.90 (3H, t, J=7Hz)

Example 97

(3S)-3-[(2S)-5-Carboxy-2-{((6S)-4,5,6,7-tetrahydro-3H-imidazo[4,5-c]pyridin-6-yl)carbonylamino}pentanoyl]-oxyhexadecanamide dihydrochloride [from (3S)-3-[(2S)-5-carboxy-2-{((6S)-3-t-butoxycarbonyl-5-t-butoxycarbonyl-4,5,6,7-tetrahydro-3H-imidazo[4,5-c]pyridin-6-yl)carbonylamino)pentanoyl]oxyhexadecanamide]

NMR ($CD_3OD$, δ) : 8.98 (1H, s), 5.30 (1H, m), 4.4-4.6 (3H, m), 3.08 (1H, dd, J=15Hz, 5Hz), 3.17 (1H, dd, J=15Hz, 10Hz), 2.52 (2H, m), 2.36 (2H, t, J=7Hz), 1.4-2.0 (6H, m), 1.2-1.4 (22H, m), 0.90 (3H, t, J=7Hz)

The following compounds (Examples 98 to 143) were obtained according to a similar manner to that of Example 6.

Example 98

(3S)-3-[6-Carboxy-2-(tert-butoxycarbonylamino)hexanoyl]-oxyhexadecanamide

NMR ($CDCl_3$, δ) : 6.32 & 5.96 (1H, br s), 5.58 & 5.50 (1H, br s), 4.95-5.30 (1H, m), 4.0-4.3 (1H, m), 2.3-2.6 (4H, m), 1.6-1.9 (8H, m), 1.40 & 1.45 (9H, s), 1.2-1.4 (22H, m), 0.88 (3H, t, J=7Hz)

Example 99

2-[(2S)-5-Carboxy-2-(tertiarybutoxycarbonylamino)-pentanoyl]oxypentadecanamide

MMR ($CDCl_3$, δ) : 7.04 6.30 (1H, br s), 5.33 · 5.21 (1H, m), 5.25 5.05 (1H, d, J=8Hz), 4.44 · 4.19 (1H, m), 2.43 (2H, m), 1.64-1.98 (4H, m), 1.46 · 1.43 (9H, s), 1.12-1.37 (24H, m), 0.88 (3H t, J=7Hz)

Example 100

3-[(2S)-5-Carboxy-2-(tertiarybutoxycarbonylamino)-pentanoyl]oxydecanamide

NMR (DMSO-$d_6$, δ) : 7.36 (1H, br s), 7.22 (1H, d, J=7Hz), 6.85 (1H, br s), 5.08 (1H, m), 3.83 (1H, m), 2.07-2.45 (4H, m), 1.38 (9H, s), 1.14-1.86 (28H, m), 0.85 (3H, t, J=7Hz)

Example 101

3-[(2S)-5-Carboxy-2-(tertiarybutoxycarbonylamino)-pentanoyl]oxydodecanamide.

NMR ($CDCl_3$, δ) : 7.05 6.41 · 6.22 (total 2H, br s), 5.10-5.37 (2H, m), 4.27 · 4.18 (sum 1H, m), 2.26-2.62 (4H, m), 1.50-1.94 (6H, m), 1.44 (9H, s), 1.08-1.39 (14H, m), 0.88 (3H, t, J=7Hz)

Example 102

3-[(2S)-5-Carboxy-2-(tertiarybutoxycarbonylamino)-pentanoyl]oxytetradecanamide

NMR ($CDCl_3$, δ) : 7.07 · 6.44 · 6.23 (total 2H, br s), 5.10-5.39 (2H, m), 4.28 · 4.18 (sum 1H, m), 2.25-2.60 (4H, m), 1.50-1.92 (6H, m), 1.44 (9H, s), 1.10-1.39 (18H, m), 0.88 (3H, t, J=7Hz)

Example 103

(3S)-3-[(2S)-5-Carboxy-2-(tertiarybutoxycarbonylamino)-pentanoyl]oxybutyramide

NMR ($CDCl_3$-$CD_3OD$(20:1), δ) : 5.27 (1H, m), 4.18 (1H, m), 2.18-2.55 (4H, m), 1.49-1.85 (4H, m), 1.41 (9H, s), 1.31 (3H, d, J=7Hz)

Example 104

(3R)-3-[(2R)-5-Carboxy-2-(tertiarybutoxycarbonylamino)-pentanoyl]oxyhexadecanamide

NMR (DMSO-$d_6$, δ) : 7.35 (1H, br s), 7.21 (1H, d, J=7.5Hz), 6.84 (1H, br s), 5.09 (1H, m), 3.82 (1H, m), 2.30 (2H, dd, J=5Hz, 4Hz), 2.16 (2H, m), 1.10-1.70 (37H, m), 0.85 (3H, t, J=7Hz)

Example 105

(3R)-3-[(2S)-5-Carboxy-2-(tertiarybutoxycarbonylamino)-pentanoyl]oxyhexadecanamide

NMR ($CDCl_3$, δ) : 6.45 (1H, br s), 6.30 (1H, br s), 5.19 (2H, m), 4.18 (1H, m), 2.29-2.64 (4H, m), 1.16-1.92 (6H, m), 1.45 (9H, s), 1.18-1.38 (22H, m), 0.89 (3H, t, J=7Hz)

Example 106

(3S)-3-[(2R)-5-Carboxy-2-(tertiarybutoxycarbonylamino)-pentanoyl]oxyhexadecanamide

NMR ($CDCl_3$, δ) : 6.45 (1H, br s), 6.30 (1H, br s), 5.19 (2H, m), 4.18 (1H, m), 2.29-2.64 (4H, m), 1.16-1.92 (6H, m), 1.45 (9H, s), 1.18-1.38 (22H, m), 0.89 (3H, t, J=7Hz)

Example 107

2-[(2S)-5-Carboxy-2-(tertiarybutoxycarbonylamino)-pentanoyl]oxy-N-methylpentadecanamide

NMR ($CDCl_3$, δ) : 5.20 (1H, m), 5.07 (1H, m), 4.22 · 4.12 (sum 1H, m), 2.79 (3H, d, J=5Hz), 2.42 (2H, m), 1.70-2.00 (4H, m), 1.46 · 1.44 (9H, s), 1.16-1.35 (24H, m), 0.87 (3H, t, J=7Hz)

Example 108

(3S)-3-[(2S)-5-Carboxy-2-(tertiarybutoxycarbonylamino)-pentanoyl]oxyhexadecanoic acid

NMR ($CDCl_3$, δ) : 5.20-5.40 (2H, m), 4.30 (1H, m), 2.63 (2H, m), 2.35 (2H, m), 1.53-1.92 (6H, m), 1.45 (9H, s), 1.16-1.36 (22H, m), 0.88 (3H, t, J=7Hz)

Example 109

Methyl 2-[(2S)-5-Carboxy-2-(tertiarybutoxycarbonylamino)pentanoyl]oxypentadecanoate

NMR ($CDCl_3$, δ) : 5.05 (2H, m), 4.39 (1H, m), 3.74 (3H, s), 2.42 (2H, m), 1.65-2.03 (6H, m), 1.44 (9H, s), 1.13-1.40 (22H, m), 0.88 (3H, t, J=7Hz)

Example 110

(3S)-Methyl 3-[(2S)-5-carboxy-2-(tertiarybutoxycarbonylamino)pentanoyl]oxyhexadecanoate

NMR ($CDCl_3$, δ) : 5.26 (1H, m), 5.10 (1H, d, J=7Hz), 4.27 (1H, m), 3.67 (3H, s), 2.59 (2H, m), 2.39 (2H, m), 1.53-1.90 (6H, m), 1.45 (9H, s), 1.15-1.37 (22H, m), 0.88 (3H, t, J=7Hz)

Example 111

(3S)-3-[(2S)-5-Carboxy-2-(tertiarybutoxycarbonylamino)-pentanoyl]aminohexadecanamide

NMR (DMSO-$d_6$, δ) : 7.63 (1H, d, J=8Hz), 7.25 (1H, br s), 6.82 (1H, d, J=8Hz), 6.77 (1H, br s), 3.98 (1H, m), 3.78 (1H, m), 2.15 (4H, m), 1.36 (9H, s), 1.07-1.65 (28H, m), 0.85 (3H, t, J=7Hz)

Example 112

N-[(2S)-5-Carboxy-2-(tertiarybutoxycarbonylamino)-pentanoyl]-N-(2-carbamoylethyl)tridecylamine

NMR ($CDCl_3$, δ) : 6.84 6.64 (1H, br s), 6.58 · 6.27 (1H, br s), 5.54 · 5.51 (1H, d, J=7Hz), 4.60 (1H, m), 3.96 (1H, m), 3.12-3.73 (3H, m), 2.23-2.60 (4H, m), 1.50-1.80 (4H, m), 1.12-1.33 (22H, m), 0.88 (3H, t, J=7Hz)

Example 113

(3S)-3-[(2S)-5-Carboxy-2-(2-biphenylcarbonylamino)-pentanoyl]oxyhexadecanamide hydrochloride

NMR (DNSO-$d_6$, δ) : 8.65 (1H, d, J=10Hz), 7.25-7.55 (10H, m), 6.85 (1H, br s), 5.12 (1H, m), 4.18 (1H, m), 2.1-2.4 (4H, m), 1.3-1.9 (6H, m), 1.15-1.3 (22H, m), 0.84 (3H, t, J=7Hz)

Example 114

(3R)-3-[(2R)-5-Carboxy-2-(p-methoxybenzoylamino)-pentanoyl]oxyhexadecanamide

NMR ($CD_3OD$, δ) : 7.85 (2H, d, J=8Hz), 6.98 (2H, d, J=8Hz), 5.27 (1H, m), 4.52 (1H, m), 3.84 (3H, s), 2.39-2.60 (2H, m), 2.34 (2H, t, J=7Hz), 1.57-2.04 (6H, m), 1.18-1.44 (22H, m), 0.89 (3H, t, J=7Hz)

Example 115

(3S)-N-Methyl-3-[(2S)-5-carboxy-2-(p-methoxybenzoylamino)pentanoyl]oxyhexadecanamide

NMR ($CDCl_3$, δ) : 7.79 (2H, d, J=8Hz), 7.08 (1H, d, J=7Hz) , 6.91 (2H, d, J=8Hz), 6.28 (1H, m), 5.30 (1H, m), 4.71 (1H, m), 3.84 (3H, m), 2.74 (2H, m), 2.32-2.52 (2H, m), 1.52-2.07 (6H, m), 1.13-1.36 (22H, m), 0.89 (3H, t, J=7Hz)

Example 116

(3S)-3-[(2S)-5-Carboxy-2-(N-tert-butoxycarbonyl-L-prolylamino)pentanoyl]oxyhexadecanamide

NMR ($CDCl_3$, δ) : 6.36 (1H, br s), 5.44 (1H, br s), 5.15 (1H, m), 4.50 (1H, m), 4.26 (1H, br s), 3.46 (2H, br s), 2.35-2.5 (4H, m), 1.5-1.95 (10H, m), 1.45 (9H, s), 1.2-1.35 (22H, m), 0.88 (3H, t, J=7Hz)

Example 117

(3S)-3-[(2S)-5-Carboxy-2-(N-tert-butoxycarbonyl-D-prolylamino)pentanoyl]oxyhexadecanamide

NMR ($CDCl_3$, δ) : 6.36 (1H, br s), 5.44 (1H, br s), 5.15 (1H, m), 4.50 (1H, m), 4.26 (1H, br s), 3.46 (2H, br s), 2.35-2.5 (4H, m), 1.5-1.95 (10H, m), 1.45 (9H, s), 1.2-1.35 (22H, m), 0.88 (3H, t, J=7Hz)

Example 118

(3S)-3-[(25)-5-Carboxy-2-{(1-tert-butoxycarbonyl-2-piperidyl)carbonylamino}pentanoyl]oxyhexadecanamide

NMR ($CDCl_3$, δ) : 7.10 (1H, br s), 6.80 (1H, d, J=10Hz), 5.28 (1H, m), 4.76 (1H, br), 4.55 (1H, m), 2.90 (1H, br), 2.2-2.6 (5H, m), 1.50 (9H, s), 1.35-2.0 (12H, m), 1.2-1.35 (22H, m), 0.88 (3H, t, J=7Hz)

Example 119

(3S)-3-[(2S)-5-Carboxy-2-{(1-tert-butoxycarbonyl-4-piperidyl)carbonylamino}pentanoyl]oxyhexadecanamide

NMR (DMSO-$d_6$, δ) : 8.14 (1H, d, J=10Kz), 7.34 (1H, br s), 6.82 (1H, br s), 5.08 (1H, m), 4.12 (1H, m), 3.92 (2H, d, J=10Hz), 2.1-2.8 (7H, m), 1.4-1.7 (10H, m), 1.35 (9H, s), 1.1-1.3 (22H, m), 0.84 (3H, t, J=7Hz)

FAB-MS : 648 [M+Na]

Example 120

(3S)-3-[(2S)-5-Carboxy-2-(piperidinocarbonylamino)-pentanoyl]oxyhexadecanamide

NMR (DMSO-$d_6$, δ) : 7.34 (1H, br s), 6.83 (1H, br s), 6.57 (1H, d, J=7Hz), 5.05 (1H, m), 3.96 (1H, m), 3.27 (4H, m), 2.33 (1H, dd, J=15Hz, 7Hz), 2.26 (1H, dd, J=15Hz, 7Hz), 2.16 (2H, t, J=7Hz), 1.13-1.73 (34H, m), 0.86 (3H, t, J=7Hz)

Example 121

(3S)-3-[(2S)-5-Carboxy-2-{(4-(3,4-methylenedioxybenzyl)-1-piperazinyl}carbonylamino}pentanoyl] oxyhexadecanamide

NMR (DMSO-$d_6$, δ) : 7.35 (1H, s), 6.85 (3H, m), 6.74 (2H, d, J=8Hz), 5.98 (2H, s), 5.05 (1H, m), 3.94 (1H, m), 3.37 (6H, m), 2.05-2.42 (8H, m), 1.40-1.75 (6H, m), 1.06-1.36 (22H, m), 0.84 (3H, t, J=7Hz)

Example 122

(3S)-3-[(2S)-5-Carboxy-2-(morpholinocarbonylamino)-pentanoyl]oxyhexadecanamide

NMR (DMSO-$d_6$, δ) : 7.33 (1H, br s), 6.83 (1H, br s), 6.73 (1H, d, J=7Hz), 5.05 (1H, m), 3.97 (1H, m), 3.52 (4H, m), 3.27 (4H, m), 2.32 (1H, dd, J=15Hz, 8Hz), 2.26 (1H, dd, J=15Hz, 7Hz), 2.16 (2H, t, J=7Hz), 1.40-1.75 (6H, m), 1.12-1.35 (22H, m), 0.84 (3H, t, J=7Hz)

Example 123

(3S)-3-[(2S)-5-Carboxy-2-(nicotinoylamino)pentanoyl]-oxyhexadecanamide

NMR (DMSO-$d_6$, δ) : 9.02 (1H, m), 8.96 (1H, m), 8.73 (1H, m), 8.22 (1H, m), 7.52 (1H, dd, J=8Hz, 5Hz), 7.36 (1H, br s), 6.85 (1H, br s), 5.12 (1H, m), 4.36 (1H, m), 2.34 (2H, t, J=7Hz), 2.23 (2H, t, J=7Hz), 1.43-1.92 (6H, m),

1.09-1.36 (22H, m), 0.85 (3H, t, J=7Hz)

Example 124

(3S)-3-[(2S)-5-Carboxy-2-isonicotinoylamino)-pentanoyl]oxyhexadecanamide

NMR (DMSO-$d_6$, δ) : 8.72 (2H, d, J=6Hz), 7.87 (2H, d, J=6Hz), 7.37 (1H, br s), 6.86 (1H, br s), 5.11 (1H, m), 4.25 (1H, m), 2.32 (2H, m), 2.11 (2H, t, J=7Hz), 1.78 (2H, m), 1.43-1.65 (4H, m), 1.10-1.38 (22H, m), 0.85 (3H, t, J=7Hz)

Example 125

(3S)-3-[(2S)-5-Carboxy-2-{(2-pyridyl)carbonylamino}-pentanoyl]oxyhexadecanamide

NMR ($CDCl_3$, δ) : 8.60 (2H, m), 8.15 (1H, d, J=9Hz), 7.84 (1H, t, J=8Hz), 7.43 (1H, dd, J=8Hz, 6Hz), 5.46 (1H, m), 4.77 (1H, m), 2.19-2.75 (4H, m), 1.50-2.12 (6H, m), 1.08-1.36 (22H, m), 0.87 (3H, m)

Example 126

(3S)-3-[(2S)-5-Carboxy-2-{(2-pyrazinyl)carbonylamino}-pentanoyl]oxyhexadecanamide

NMR (DMSO-$d_6$, δ) : 9.20 (1H, s), 9.04 (1H, br s), 8.90 (1H, s), 8.76 (1H, s), 7.42 (1H, br s), 6.88 (1H, br s), 5.12 (1H, m), 4.40 (1H, m), 1.9-2.4 (4H, m), 1.4-1.9 (6H, m), 1.2-1.4 (22H, m), 0.88 (3H, t, J=7Hz)

FAB-MS : 543 [M+Na]

Example 127

(3S)-3-[(2S)-5-Carboxy-2-{(5-quinolyl)carbonylamino}-pentanoyl]oxyhexadecanamide

NMR (DMSO-$d_6$, δ) : 9.02 (1H, d, J=10Hz), 8.94 (1H, m), 8.64 (1H, d, J=10Hz), 8.12 (1H, d, J=10Hz), 7.75 (2H, ABq), 7.56 (1H, m), 7.38 (1H, br s), 6.86 (1H, br s), 5.18 (1H, m), 4.44 (1H, m), 2.3-2.5 (4H, m), 1.5-1.9 (6H, m), 1.1-1.4 (22H, m), 0.82 (3H, t, J=7Hz)

Example 128

(3S)-3-[(2S)-5-Carboxy-2-{(1-isoquinolyl)carbonylamino}-pentanoyl]oxyhexadecanamide

NMR (DMSO-$d_6$, δ) : 9.10 (1H, d, J=10Hz), 8.85 (1H, d, J=10Hz), 8.54 (1H, d, J=7Hz), 8.04 (2H, m), 7.82 (1H, t, J=7Hz), 7.70 (1H, t, J=7Hz), 7.38 (1H, br s), 6.86 (1H, br s), 5.18 (1H, m), 4.45 (1H, m), 2.2-2.45 (4H, m), 1.45-1.95 (6H, m), 1.1-1.3 (22H, m), 0.84 (3H, t, J=7Hz)

FAB-MS : 592 [M+Na]

Example 129

(3S)-3-[(2S)-5-Carboxy-2-{(3-isoquinolyl)carbonylamino}-pentanoyl]oxyhexadecanamide

NMR (DMSO-$d_6$, δ) : 9.42 (1H, s), 8.97 (1H, d, J=10Hz), 8.56 (1H, s), 8.1-8.4 (2H, m), 7.7-7.9 (2H, m), 5.15 (1H, m), 4.50 (1H, m), 2.2-2.4 (4H, m), 1.4-2.0 (6H, m), 1.1-1.4 (22H, m), 0.83 (3H, t, J=7Hz)

FAB-MS : 570 [M+H]

Example 130

(3S)-3-[(2S)-5-Carboxy-2-{(2-quinoxalinyl) carbonylamino}pentanoyl]oxyhexadecanamide

NMR (DMSO-$d_6$, δ) : 9.45 (1H, s), 9.18 (1H, d, J=10Hz), 8.22 (2H, m), 8.00 (2H, m), 7.35 (1H, br s), 6.85 (1H, br s), 5.15 (1H, m), 4.50 (1H, m), 2.25-2.45 (2H, m), 1.90 (2H, m), 1.65 (2H, m), 1.50 (2H, m), 1.0-1.3 (22H, m), 0.84 (3H, t, J=7Hz)

Example 131

(3S)-3-[(2S)-5-Carboxy-2-{((6S)-5-tert-butoxycarbonyl-3-tert-butoxycarbonyl-4,5, 6, 7-tetrahydro-3H-imidazo [4,5-c]-pyridin-6-yl)carbonylamino}pentanoyl]oxyhexadecanamide

NMR ($CDCl_3$, δ) : 8.00 (1H, s), 6.70 (1H, br s), 5.85 (1H, br s), 5.30 (1H, br s), 5.20 (1H, m), 4.42 (2H, br s), 3.3 (1H, m), 2.90 (2H, m), 2.2-2.5 (4H, m), 1.60 (9H, s), 1.50 (9H, s), 1.4-1.9 (8H, m), 1.15-1.35 (22H, m), 0.88 (3H, t, J=7Hz)

Example 132

(3S)-3-[(2S)-5-Carboxy-2-{((6R)-5-tert-butoxycarbonyl-3-tert-butoxycarbonyl-4,5,6,7-tetrahydro-3H-imidazo[4,5-c]-pyridin-6-yl)carbonylamino}pentanoyl]oxyhexadecanamide

NMR ($CDCl_3$, δ) : 8.00 (1H, s), 6.70 (1H, br s), 5.85 (1H, br s), 5.30 (1H, br s), 5.20 (1H, m), 4.42 (2H, br s), 3.3 (1H, m), 2.90 (2H, m), 2.2-2.5 (4H, m), 1.60 (9H, s), 1.50 (9H, s), 1.4-1.9 (8H, m), 1.15-1.35 (22H, m), 0.88 (3H, t, J=7Hz)

Example 133

(3S)-3-[(2S)-5-Carboxy-2-{(2-pyridyl)-methylamino}pentanoyl]oxyhexadecanamide

NMR (DMSO-$d_6$, δ) : 8.45 (1H, d, J=5Hz), 7.72 (1H, dd, J=7.5Hz), 7.40 (1H, d, J=10Hz), 7.38 (1H, br s), 6.84 (1H, br s), 5.16 (1H, m), 3.72 (2H, ABq), 3.14 (1H, br), 2.35 (2H, m), 2.18 (2H, br), 1.45-1.65 (6H, m), 1.15-1.35 (22H, m), 0.88 (3H, t, J=7Hz)

Example 134

(3S)-3-[(2S)-5-Carboxy-2-{(5-imidazolyl)-methylcarbonylamino}pentanoyl]oxyhexadecanamide

NMR (DMSO-$d_6$, δ) : 8.33 (1H, d, J=10Hz), 7.52 (1H, s), 7.36 (1H, br s), 6.83 (2H, s), 5.08 (1H, m), 4.16 (1H, m), 3.40 (2H, s), 2.1-2.4 (4H, m), 1.4-1.7 (6H, m), 1.15-1.35 (22H, m), 0.84 (3H, t, J=7Hz)

Example 135

(3S)-3-[(2S)-5-Carboxy-2-{(2-pyridyl)-methylcarbonylamino}pentanoyl]oxyhexadecanamide

NMR ($CDCl_3$, δ) : 8.48 (1H, d, J=5Hz), 8.07 (1H, d, J=10Hz), 7.69 (1H, t, J=7Hz), 7.40 (1H, d, J=7Hz), 7.25 (1H, t, J=7Hz), 6.50 (1H, br s), 6.15 (1H, br s), 5.25 (1H, m), 4.52 (1H, m), 2.35-2.5 (4H, m), 2.30 (2H, t, J=7Hz), 1.5-1.9 (6H, m), 1.2-1.35 (22H, m), 0.86 (3H, t, J=7Hz)

FAB-MS : 534 [M+H]

Example 136

(3S)-3-[(2S)-5-Carboxy-2-(phenoxycarbonylamino)-pentanoyl]oxyhexadecanenitrile

NMR ($CDCl_3$, δ) : 7.06-7.41 (5H, m), 5.92 (1H, m), 5.06 (1H, m), 4.43 (1H, m), 2.32-2.82 (4H, m), 1.56-2.12 (6H, m), 1.04-1.42 (22H, m), 0.88 (3H, t, J=7Hz)

Example 137

(3S)-3-[(2S)-5-Carboxy-2-{3-((2S)-2-hydroxypropyl)-uzeldo}pentanoyl]oxyhexadecanamide

NMR (DMSO-$d_6$, δ) : 7.35 (1H, br s), 6.83 (1H, br s), 6.34 (1H, d, J=8Hz), 6.05 (1H, t, J=6Hz), 5.07 (1H, m), 4.07 (1H, m), 3.56 (1H, m), 2.98 (1H, m), 2.85 (1H, m), 2.34 (1H, dd, J=15Hz, 7Hz), 2.27 (1H, dd, J=15Hz, 6Hz), 2.18 (2H, t, J=7Hz), 1.37-1.70 (6H, m), 1.10-1.32 (22H, m), 0.99 (3H, d, J=7Hz), 0.85 (3H, t, J=7Hz)

Example 138

(3S)-3-[(2S)-5-Carboxy-2-(3-morpholinoureido)-pentanoyl]oxyhexadecanamide

NMR (DMSO-$d_6$, δ) : 7.45 (1H, br s), 7.38 (1H, br s), 6.86 (1H, br s), 6.58 (1H, d, J=9Hz), 5.08 (1H, m), 4.10 (1H, m), 3.64 (4H, m), 2.68 (4H, m), 2.32 (2H, d, J=7Hz), 2.18 (2H, t, J=7Hz), 1.06-1.80 (28H, m), 0.85 (3H, t, J=7Hz)

Example 139

(3R)-3-[(2R)-5-Carboxy-2-{3-(2-pyridyl)ureido}pentanoyl]oxyhexadecanamide

NMR ($CDCl_3$-$CD_3OD$(20:1), δ) : 8.21 (1H, d, J=5Hz), 7.62 (1H, dd, J=8Hz, 7Hz), 7.03 (1H, d, J=8Hz), 6.92 (1H, dd, J=7Hz, 5Hz), 5.26 (1H, m), 4.54 (1H, dd, J=7Hz, 6Hz), 2.49 (2H, d, J=7Hz), 2.37 (2H, m), 1.55-2.07 (6H, m), 1.13-1.43 (22H, m), 0.88 (3H, t, J=7Hz)

Example 140

(3S)-3-[(2S)-5-Carboxy-2-{3-methyl-3-(2-pyridyl)ureido}pentanoyl]oxyhexadecanamide

NMR ($CDCl_3$, δ) : 8.33 (1H, m), 7.71 (1H, m), 6.98 (2H, m), 6.81 (1H, br s), 6.27 (1H, br s), 5.28 (1H, m), 4.60 (1H, m), 3.39 (3H, s), 2.28-2.60 (4H, m), 1.53-2.07 (6H, m), 1.12-1.38 (22H, m), 0.88 (3H, t, J=7Hz)

Example 141

(3S)-3-[(2S)-5-Carboxy-2-{3-(2-quinolyl)ureido}-pentanoyl]oxyhexadecanamide

NMR ($CDCl_3$, δ) : 9.97 (1H, s), 8.23 (1H, d, J=8Hz), 7.84 (1H, d, J=7Hz), 7.78 (1H, d, J=8Hz), 7.66 (1H, dd, J=8Hz, 7Hz), 7.37-7.47 (2H, m), 7.27 (1H, d, J=8Hz), 6.87 (1H, br s), 5.16 (1H, m), 4.36 (1H, m), 2.39 (1H, dd, J=15Hz, 8Hz), 2.33 (1H, dd, J=15Hz, 6Hz), 2.26 (2H, t, J=7Hz), 1.45-1.94 (6H, m), 1.09-1.39 (22H, m), 0.84 (3H, t, J=7Hz)

Example 142

(3S)-3-[(2S)-5-Carboxy-2-{3-(3-quinolyl)ureido}-pentanoyl]oxyhexadecanamide

NMR (DMSO-$d_6$, δ) : 9.15 (1H, br s), 8.74 (1H, d, J=2Hz), 8.45 (1H, d, J=2Hz), 7.89 (1H, d, J=8Hz), 7.82 (1H, d, J=8Hz), 7.47-7.60 (2H, m), 7.38 (1H, br s), 6.81-6.90 (2H, m), 5.15 (1H, m), 4.21 (1H, m), 2.18-2.39 (4H, m), 1.43-1.82 (6H, m), 1.02-1.34 (22H, m), 0.84 (3H, t, J=7Hz)

Example 143

(3S)-3-[(2S)-5-Carboxy-2-{(8-quinolyl)sulfonylamino}-pentanoyl]oxyhexadecanamide

NMR (DMSO-$d_6$, δ) : 9.04 (1H, d, J=5Hz), 8.50 (1H, d, J=10Hz), 8.26 (2H, m), 7.65 (2H, q, J=10Hz), 7.22 (1H, br s), 6.72 (1H, br s), 4.72 (1H, m), 4.15 (1H, m), 2.0-2.2 (4H, m), 1.4-1.8 (6H, m), 0.9-1.3 (22H, m), 1.88 (3H, t, J=7Hz)

The following compounds (Examples 144 and 145) were obtained according to a similar manner to that of Example 2.

Example 144

(3R)-3-[(2R)-5-Benzyloxycarbonyl-2-(p-methoxybenzoylamino)pentanoyl]oxyhexadecanamide

NMR ($CDCl_3$, δ) : 7.78 (2H, d, J=8Hz), 7.28-7.41 (5H, m), 6.94 (2H, d, J=8Hz), 6.85 (1H, d, J=8Hz) 5.98 (1H, br s), 5.33 (2H, m), 5.12 (2H, s), 4.70 (1H, m), 3.86 (3H, s), 2.38-2.50 (4H, m), 1.57-2.08 (6H, m), 1.13-1.37 (22H, m), 0.88 (3H, t, J=7Hz)

Example 145

(3S)-N-Methyl-3-[(2S)-5-benzyloxycarbonyl-2-(p-methoxybenzoylamino)pentanoyl]oxyhexadecanamide

NMR ($CDCl_3$, δ) : 7.81 (2H, d, J=8Hz), 7.35 (5H, m), 6.94 (2H, d, J=8Hz), 6.85 (1H, d, J=7Hz), 6.04 (1H, m), 5.28 (1H,

m), 5.13 (2H, s), 4.68 (1H, m), 3.86 (3H, s), 2.70 (3H, d, J=5Hz), 2.35-2.51 (4H, m), 1.53-2.06 (6H, m), 1.15-1.37 (22H, m), 0.88 (3H, t, J=7Hz)

The following compounds (Examples 146 to 153) were obtained according to a similar manner to that of Example 27.

Example 146

(3S)-3-[(2S)-5-Carboxy-2-{(6-methyl-2-pyridyl)carbonylamino}pentanoyl]oxyhexadecanamide

NMR ($CDCl_3$, δ) : 8.70 (1H, d, J=10Hz), 7.96 (1H, d, J=10Hz), 7.70 (1H, t, J=10Hz), 7.30 (1H, d, J=10Hz), 7.20 (1H, br s), 6.26 (1H, br s), 5.35 (1H, m), 4.80 (1H, m), 2.60 (3H, s), 2.3-2.55 (4H, m), 1.6-2.2 (6H, m), 1.2-1.4 (22H, m), 0.88 (3H, t, J=7Hz)

FAB-MS : 534 [M+H]

Example 147

(3R)-3-[(2R)-5-Carboxy-2-{(3-quinolyl(carbonylamino}-pentanoyl]oxyhexadecanamide

NMR (DMSO-$d_6$, δ) : 9.29 (1H, d, J=1.5Hz), 9.10 (1H, d, J=8Hz), 8.87 (1H, d, J=1.5Hz), 8.10 (1H, d, J=8Hz), 8.09 (1H, d, J=8Hz), 7.88 (1H, t, J=8Hz), 7.70 (1H, t, J=8Hz), 7.35 (1H, br s), 6.85 (1H, br s), 5.15 (1H, m), 4.42 (1H, m), 2.19-2.45 (4H, m), 1.45-1.98 (6H, m), 1.07-1.40 (22H, m), 0.84 (3H, t, J=7Hz)

Example 148

N-[(S)-5-Carboxy-2-{(2-quinolyl) carbonylamino}pentanoyl]-N-(2-carbamoylethyl)tridecylamine

NMR ($CDCl_3$, δ) : 9.02 · 8.95 (1H, d, J=8Hz), 8.31 (1H, d, J=8Hz), 8.26 · 8.23 (sum 1H, d, J=8Hz), 8.18 (1H, d, J=8Hz), 7.88 (1H, d, J=8Hz), 7.78 (1H, m), 7.62 (1H, m), 7.04 · 6.84 (sum 1H, br s), 6.71 · 6.54 (sum 1H, m), 5.22 (1H, m), 4.09 (1H, m), 3.77 · 3.66 (sum 2H, m), 3.34 3.15 (sum 2H, m), 2.67 (1H, m), 2.30-2.56 (3H, m), 1.49-2.08 (4H, m), 1.11-1.36 (22H, m), 0.88 (3H, t, J=7Hz)

Example 149

N-[(S)-5-Carboxy-2-{(2-quinolyl) carbonylamino}pentanoyl]-N-(N-methylcarbamoylmethyl)tridecylamine

NMR ($CDCl_3$, δ) : 8.89 · 8.83 (sum 1H, d, J=7Hz), 8.31 · 8.29 (sum 1H, d, J=8Hz), 8.23 · 8.21 (sum 1H d, J=8Hz), 8.15 (1H, d, J=8Hz), 7.87 (1H, m), 7.77 (1H, m), 7.62 (1H, m), 6.71 (1H, m), 5.07 · 4.84 (sum 1H, m), 4.16 (1H, d, J=16Hz), 3.96 (1H, d, J=16Hz), 3.62 (1H, m), 3.47 · 3.22 (1H, m), 2.87 2.80 (sum 3H, d, J=5Hz), 2.45 (2H, m), 1.50-2.07 (4H, m), 1.14-1.38 (22H, m), 0.88 (3H, m)

Example 150

(3S)-3-[(2S)-5-Carboxy-2-{3-(5-chloro-2-pyridyl)ureido}pentanoyl]oxyhexadecanamide

NMR (DMSO-$d_6$, δ) : 9.37 (1H, br s), 8.23 (1H, d, J=2Hz), 7.86 (1H, d, J=7Hz), 7.78 (1H, dd, J=8Hz, 2Hz), 7.59 (1H, d, J=8Hz), 7.35 (1H, br s), 6.84 (1H, br s), 5.13 (1H, m), 4.22 (1H, m), 2.37 (1H, dd, J=15Hz, 8Hz), 2.31 (1H, dd,

J=15Hz, 6Hz), 2.24 (2H, t, J=7Hz), 1.42-1.83 (6H, m), 1.11-1.36 (22H, m), 0.85 (3H, t, J=7Hz)

Example 151

(3S)-3-[(2S)-5-Carboxy-2-{3-(5-methyl-2-pyridyl)ureido}pentanoyl]oxyhexadecanamide

NMR (DMSO-$d_6$, δ) : 9.18 (1H, br s), 8.47 (1H, br s), 8.02 (1H, d, J=2Hz), 7.50 (1H, dd, J=8Hz, 2Hz), 7.35 (1H, br s), 7.27 (1H, d, J=8Hz), 6.83 (1H, br s), 5.13 (1H, m), 4.25 (1H, m), 2.37 (1H, dd, J=15Hz, 7Hz), 2.31 (1H, dd, J=15Hz, 6Hz), 2.25 (2H, t, J=7Hz), 2.19 (3H, s), 1.43-1.85 (6H, m), 1.12-1.34 (22H, m), 0.85 (3H, t, J=7Hz)

Example 152

(3S)-3-[(2S)-5-Carboxy-2-{3-(2-benzothiazolyl)ureido}-pentanoyl]oxyhexadecanamide

NMR (DMSO-$d_6$, δ) : 7.85 (1H, d, J=8Hz), 7.63 (1H, d, J=8Hz), 7.36 (2H, m), 7.21 (2H, m), 6.85 (1H, br s), 5.15 (1H, m), 4.26 (1H, m), 2.38 (1H, dd, J=15Hz, 8Hz), 2.33 (1H, dd, J=15Hz, 6Hz), 2.25 (2H, t, J=7Hz), 1.45-1.85 (6H, m), 1.09-1.34 (22H, m), 0.84 (3H, t, J=7Hz)

Example 153

(3S)-3-[(2S)-5-Carboxy-2-{3-(5-chloro-2-benzoxazolyl)ureido}pentanoyl]oxyhexadecanamide

NMR (DMSO-$d_6$, δ) : 8.68 (1H, m), 7.62 (2H, m), 7.37 (1H, br s), 7.26 (1H, dd, J=8Hz, 2Hz), 6.85 (1H, br s), 5.16 (1H, m), 4.34 (1H, m), 2.32-2.42 (2H, m), 2.27 (2H, t, J=7Hz), 1.47-1.88 (6H, m), 1.11-1.36 (22H, m), 0.84 (3H, t, J=7Hz)

Example 154

To a solution of (3S)-3-[(2S)-5-carboxy-2-{(3-quinolyl)-carbonylamino}pentanoyl]oxyhexadecanamide (100 mg) in methanol (10 ml) was added 2M solution of trimethylsilyldiazomethane in hexane (2 ml) at room temperature. After being stirred at the same temperature for 2 days, the solvent was removed under reduced pressure and the resulting solid was triturated with ethyl ether to give (3S)-3-[(2S)-5-methoxycarbonyl-2-{(3-quinolyl)carbonylamino}-pentanoyl]oxyhexadecanamide (68.4 mg).

NMR (DMSO-$d_6$, δ) : 9.42 (1H, d, J=5Hz), 9.14 (1H, d, J=10Hz), 9.05 (1H, m), 8.18 (2H, t, J=7Hz), 7.95 (1H, t, J=7Hz), 7.75 (1H, t, J=7Hz), 7.44 (1H, br s), 6.85 (1H, br s), 5.15 (1H, m), 4.42 (1H, m), 3.58 (3H, s), 2.22-2.40 (4H, m), 1.4-1.95 (6H, m), 1.1-1.4 (22H, m), 0.82 (3H, t, J=7Hz)

Example 155

(3S)-3-[(2S)-5-Carboxy-2-{(3-quinolyl)carbonylamino}-pentanoyl]oxyhexadecanamide (100 mg) was dissolved in 4N-hydrogen chloride in 1,4-dioxane (5 ml) at room temperature. After being stirred at the same temperature for 10 minutes, the solvent was removed under reduced pressure and the resulting solid was triturated with ethyl ether to give (3S)-3-[(2S)-5-carboxy-2-{(3-quinolyl)carbonylamino}pentanoyl]-oxyhexadecanamide hydrochloride (100 mg).

NMR (DMSO-$d_6$, δ) : 9.42 (1H, d, J=5Hz), 9.14 (1H, d, J=10Hz), 9.05 (1H, m), 8.18 (2H, t, J=7Hz), 7.95 (1H, t, J=7Hz), 7.75 (1H, t, J=7Hz), 7.44 (1H, br s), 6.85 (1H, br s), 5.15 (1H, m), 4.42 (1H, m), 2.22-2.40 (4H, m), 1.4-1.95 (6H, m), 1.1-1.4 (22H, m), 0.82 (3H, t, J=7Hz)

Example 156

To a solution of (3S)-3-[(2S)-5-carboxy-2-{(3-quinolyl)carbonylamino}pentanoyl]oxyhexadecanamide (100

mg) in 1,4-dioxane (5 ml) was added 1M aqueous sulfuric acid (0.18 ml) at room temperature. After being stirred at the same temperature for 10 minutes, the solvent was removed under reduced pressure and the resulting solid was triturated with ethyl ether to give (3S)-3-[(2S)-5-carboxy-2-{(3-quinolyl)carbonylamino}pentanoyl]oxyhexadecanamide sulfate (100 mg).

NMR (DMSO-$d_6$, δ) : 9.42 (1H, d, J=5Hz), 9.14 (1H, d, J=10Hz), 9.05 (1H, m), 8.18 (1H, d, J=7Hz), 8.14 (1H, d, J=7Hz), 7.95 (1H, t, J=7Hz), 7.75 (1H, t, J=7Hz), 7.44 (1H, br s), 6.85 (1H, br s), 5.15 (1H, m), 4.42 (1H, m), 2.22-2.40 (4H, m), 1.4-1.95 (6H, m), 1.1-1.4 (22H, m), 0.82 (3H, t, J=7Hz)

Example 157

To a solution of (3S)-3-[(2S)-5-carboxy-2-{(3-quinolyl)carbonylamino}pentanoyl]oxyhexadecanamide (100 mg) in 1,4-dioxane (5 ml) was added IN aqueous sodium hydroxide (0.18 ml) at room temperature. After being stirred at the same temperature for 10 minutes, the solvent was removed under reduced pressure and the resulting solid was triturated with ethyl ether to give sodium (3S)-3-[(2S)-5-carboxy-2-{(3-quinolyl)carbonylamino}pentanoyl]oxyhexadecanamide (100 mg).

NMR (DMSO-$d_6$, δ): 9.42 (1H, d, J=5Hz), 9.05 (1H, m), 8.08 (2H, d, J=7Hz), 7.86 (1H, t, J=7Hz), 7.68 (1H, t, J=7Hz), 7.44 (1H, br s), 6.90 (1H, br s), 5.12 (1H, m), 4.25 (1H, m), 2.22-2.40 (2H, m), 2.00 (2H, m), 1.83 (2H, m), 1.45-1.65 (4H, m), 1.1-1.4 (22H, m), 0.82 (3H, t, J=7Hz)

Example 158

To a solution of (3S)-3-[(2S)-5-benzyloxycarbonyl-2-(tertiarybutoxycarbonylamino)pentanoyl]oxy-15-methylhexadecanamide (0.13 g) and anisole (0.2 g) in dichloromethane (10 ml) was added trifluoroacetic acid (2 ml). The mixture was stirred at room temperature for 30 minutes and evaporated. The residue was dissolved in ethyl acetate (10 ml) and washed with water, sodium bicarbonate aqueous solution and water. The solution was dried over magnesium sulfate. (S)-3-hydroxy-15-methylhexadecanoic acid (60 mg), l-hydroxybenzotriazole (30 mg) and 1-ethyl-3-(3-dimethylaminopropyl)carbodiimide hydrochloride (42 mg) were added to the solution. The mixture was stirred at room temperature for 6 hours and poured into a mixture of ethyl acetate and diluted hydrochloric acid. The organic layer was washed with water, dried over magnesium sulfate and evaporated. The residue was chromatographed on a silica gel column eluting with a mixture of chloroform and methanol (50:1) to give (3S)-3-[(2S)-5-benzyloxycarbonyl-2-{(3S)-3-hydroxy-15-methylhexadecanoylamino}pentanoyl]oxy-15-methylhexadecanamide as an oil (0.10 g).

NMR ($CDCl_3$, δ) : 7.35 (5H, s), 6.55 (1H, d, J=7Hz), 5.82 (1H, br s), 5.40 (1H, br s), 5.26 (1H, m), 5.10 (2H, s), 4.53 (1H, m), 3.98 (1H, m), 3.52 (1H, d, J=3Hz), 2.3-2.5 (6H, m), 1.1-1.9 (50H, m), 0.87 (12H, d, J=7Hz)

Example 159

To a solution of (3S)-3-[(2S)-5-benzyloxycarbonyl-2-{(3S)-3-hydroxy-15-methylhexadecanoylamino}pentanoyl]oxy15-methylhexadecanamide (80 mg) in a mixture of 1,4-dioxane (10 ml) and methanol (10 ml) were added 10% palladium on active carbon (50 mg) and water (1 ml). The mixture was stirred at room temperature under hydrogen atmosphere for 1 hour. The catalyst was filtered off and the solvent was removed under reduced pressure to give (3S)-3-[(2S)-5-carboxy-2-{(3S)-3-hydroxy-15-methylhexadecanoylamino}-pentanoyl]oxy-15-methylhexadecanamide as white powder (56 mg).

NMR ($CD_3OD$, δ) : 5.26 (1H, m), 4.38 (1H, m), 3.95 (1H, m), 2.3-2.6 (6H, m), 1.1-1.9 (50H, m), 0.89 (12H, d, J=7Hz)

## Claims

1. A fatty acid derivative represented by the following formula :

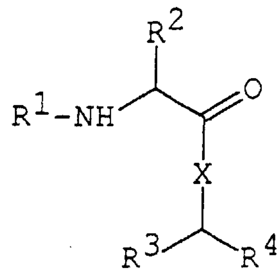

wherein

$R^1$ is acyl group or ($C_1$-$C_6$) aliphatic hydrocarbon group which may have one or more suitable substituent(s),
$R^2$ is acyl ($C_1$-$C_6$) alkyl,
$R^3$ is ($C_1$-$C_6$)alkyl or ($C_7$-$C_{20}$) alkyl,
$R^4$ is acyl group, acyl ($C_1$-$C_6$)alkyl, or cyano ($C_1$-$C_6$) alkyl, and
X is -O- or

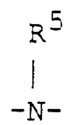

[wherein $R^5$ is hydrogen or acyl($C_1$-$C_6$) alkyl],

and a pharmaceutically acceptable salt thereof.

**2.** A derivative of claim 1, wherein

$R^1$ is protected carboxy; ($C_3$-$C_6$) alkenoyl; aryl($C_1$-$C_6$) alkanoyl; aryl ($C_3$-$C_6$) alkenoyl; arylsulfonyl which may have one or more suitable substituent(s) ;
heterocyclic ($C_1$-$C_6$) alkanoyl which may have one or more suitable substituent(s);
heterocyclicsulfonyl; amidated carboxy; ($C_7$-$C_{20}$) alkanoyl which may have one or more suitable substituent (s) ;
($C_1$-$C_6$)alkyl which may have one or more suitable substituent(s);
($C_2$-$C_6$) alkenyl which may have one or more suitable substituent(s);
or ($C_2$-$C_6$) alkynyl which may have one or more suitable substituent(s),
$R^2$ is carboxy($C_1$-$C_6$) alkyl or protected carboxy ($C_1$-$C_6$) alkyl,
$R^4$ is protected carboxy; amidated carboxy; carboxy ($C_1$-$C_6$) alkyl; protected carboxy ($C_1$-$C_6$) alkyl; amidated carboxy ($C_1$-$C_6$)alkyl; or cyano ($C_1$-$C_6$) alkyl,
X is -O- or

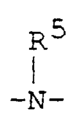

[wherein $R^5$ is hydrogen or amidated carboxy ($C_1$-$C_6$) alkyl].

**3.** A derivative of claim 2, wherein

$R^1$ is ($C_1$-$C_6$) alkoxycarbonyl; ($C_6$-$C_{10}$)aryloxycarbonyl; ($C_3$-$C_6$)alkenoyl; ($C_6$-$C_{10}$)aryl ($C_1$-$C_6$)alkanoyl which may have 1 to 3 ($C_1$-$C_6$)alkoxy or ($C_6$-$C_{10}$)aryl; ($C_6$-$C_{10}$)aryl ($C_3$-$C_6$) alkenoyl; ($C_6$-$C_{10}$)arylsulfonyl which may have 1 to 3 di ($C_1$-$C_6$) alkylamino;

heterocyclic ($C_1$-$C_6$) alkanoyl, wherein heterocyclic moiety is unsaturated 3 to 8-membered heteromonocyclic group containing 1 to 4 nitrogen atom(s), which may have 1 to 3 ($C_1$-$C_6$) alkyl;
heterocyclic ($C_1$-$C_6$) alkanoyl, wherein heterocyclic moiety is unsaturated condensed heterocyclic group containing 1 to 4 nitrogen atom(s), which may have 1 to 3 ($C_1$-$C_6$) alkoxycarbonyl;
heterocyclic ($C_1$-$C_6$) alkanoyl, wherein heterocyclic moiety is saturated 3 to 8-membered heteromonocyclic group containing 1 to 4 nitrogen atom(s), which may have 1 to 3 ($C_1$-$C_6$) alkoxycarbonyl or ($C_6$-$C_{10}$)aryl ($C_1$-$C_6$) alkyl which may have ($C_1$-$C_6$) alkylenedioxy;
heterocyclic ($C_1$-$C_6$)alkanoyl, wherein heterocyclic moiety is saturated 3 to 8-membered heteromonocyclic group containing 1 to 2 oxygen atom(s) and 1 to 3 nitrogen atom(s) ;
heterocyclicsulfonyl, wherein heterocyclic moiety is unsaturated condensed heterocyclic group containing 1 to 4 nitrogen atom(s);
N-heterocyclic-carbamoyl, wherein heterocyclic moiety is unsaturated 3 to 8-membered heteromonocyclic group containing 1 to 4 nitrogen atom(s), which may have 1 to 3 ($C_1$-$C_6$) alkyl or halogen;
N-heterocyclic-carbamoyl, wherein heterocyclic moiety is unsaturated condensed heterocyclic group containing 1 to 4 nitrogen atoms;
N-heterocyclic-carbamoyl, wherein heterocyclic moiety is unsaturated condensed heterocyclic group containing 1 to 2 oxygen atom(s) and 1 to 3 nitrogen atom(s), which may have 1 to 3 halogen;
N-heterocyclic-carbamoyl, wherein heterocyclic moiety is unsaturated condensed heterocyclic group containing 1 to 2 sulfur atom(s) and 1 to 3 nitrogen atom(s);
N-heterocyclic-carbamoyl, wherein heterocyclic moiety is saturated 3 to 8-membered heteromonocyclic group containing 1 to 2 oxygen atom(s) and 1 to 3 nitrogen atom(s);
N- ($C_1$-$C_6$)alkyl-N-heterocyclic-carbamoyl wherein heterocyclic moiety is unsaturated 3 to 8-membered heteromonocyclic group containing 1 to 4 nitrogen atom(s);
N-heterocyclic ($C_1$-$C_6$) alkylcarbamoyl, wherein heterocyclic moiety is unsaturated 3 to 8-membered heteromonocyclic group containing 1 to 4 nitrogen atom(s);
N-.($C_1$-$C_6$)alkylcarbamoyl which may have 1 to 3 hydroxy;
($C_7$-$C_{20}$) alkanoyl which may have 1 to 3 hydroxy; or ($C_1$-$C_6$)alkyl which may have 1 to 3 unsaturated 3 to 8-membered heteromonocyclic group containing 1 to 4 nitrogen atom(s) ;

$R^2$ is carboxy($C_1$-$C_6$)alkyl; ($C_1$-$C_6$)alkoxycarbonyl ($C_1$-$C_6$) - alkyl or ($C_6$-$C_{10}$) aryl ($C_1$-$C_6$) alkoxycarbonyl;
$R^4$ is ($C_1$-$C_6$)alkoxycarbonyl; carbamoyl; N-($C_1$-$C_6$) alkylcarbamoyl; carboxy ($C_1$-$C_6$) alkyl; ($C_1$-$C_6$) alkoxycarbonyl ($C_1$-$C_6$) alkyl;
halo ($C_1$-$C_6$) alkoxycarbonyl ($C_1$-$C_6$) alkyl; ($C_6$-$C_{10}$) aryl ($C_1$-$C_6$) alkoxycarbonyl ($C_1$-$C_6$) alkyl; carbamoyl ($C_1$-$C_6$) alkyl;
N-($C_1$-$C_6$) alkylcarbamoyl ($C_1$-$C_6$) alkyl;
N- ($C_6$-$C_{10}$) aryl ($C_1$-$C_6$) alkylcarbamoyl ($C_1$-$C_6$) alkyl;
or cyano ($C_1$-$C_6$)alkyl; and
X is -O- or

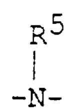

[wherein $R^5$ is hydrogen; carbamoyl ($C_1$-$C_6$) alkyl; or N- ($C_1$-$C_6$)alkylcarbamoyl ($C_1$-$C_6$) alkyl].

**4.** A derivative of claim 3, wherein

$R^3$ is ($C_7$-$C_{20}$) alkyl,
$R^4$ is ($C_1$-$C_6$) alkoxycarbonyl; carbamoyl;
N-($C_1$-$C_6$) alkylcarbamoyl; carboxy ($C_1$-$C_6$) alkyl; ($C_1$-$C_6$) alkoxycarbonyl ($C_1$-$C_6$) alkyl;
halo ($C_1$-$C_6$) alkoxycarbonyl ($C_1$-$C_6$) alkyl;
phenyl ($C_1$-$C_6$) alkoxycarbonyl ($C_1$-$C_6$) alkyl; carbamoyl ($C_1$-$C_6$) alkyl;
N-lower alkylcarbamoyl ($C_1$-$C_6$) alkyl; or N-phenyl ($C_1$-$C_6$) alkylcarbamoyl ($C_1$-$C_6$) alkyl;
and
X is -O-.

**5.** A derivative of claim 4, wherein

$R^1$ is ($C_1$-$C_6$) alkoxycarbonyl; phenoxycarbonyl; ($C_3$-$C_6$) alkenoyl; phenyl ($C_1$-$C_6$) alkanoyl which may have 1 to 3 ($C_1$-$C_6$)alkoxy or phenyl;
naphthyl ($C_1$-$C_6$) alkanoyl; phenyl ($C_3$-$C_6$) alkenoyl;
N-($C_1$-$C_6$) alkylcarbamoyl which may have 1 to 3 hydroxy; ($C_7$-$C_{20}$) alkanoyl which may have 1 to 3 hydroxy; or naphthylsulfonyl which may have 1 to 3 di ($C_1$-$C_6$) alkylamino;
$R^2$ is carboxy ($C_1$-$C_6$) alkyl,
$R^4$ is carbamoyl ($C_1$-$C_6$) alkyl.

**6.** A derivative of claim 4, wherein

$R^1$ is heterocyclic ($C_1$-$C_6$) alkanoyl, wherein heterocyclic moiety is unsaturated 3 to 8-membered heteromonocyclic group containing 1 to 4 nitrogen atom(s), which may have 1 to 3 ($C_1$-$C_6$) alkyl;
heterocyclic ($C_1$-$C_6$) alkanoyl, wherein heterocyclic moiety is unsaturated condensed heterocyclic group containing 1 to 4 nitrogen atom(s), which may have 1 to 3 ($C_1$-$C_6$) alkoxycarbonyl;
heterocyclic ($C_1$-$C_6$) alkanoyl, wherein heterocyclic moiety is saturated 3 to 8-membered heteromonocyclic group containing 1 to 4 nitrogen atom(s), which may have 1 to 3 ($C_1$-$C_6$) alkoxycarbonyl or ($C_6$-$C_{10}$)aryl ($C_1$-$C_6$) alkyl which may have ($C_1$-$C_6$) alkylenedioxy;
heterocyclic ($C_1$-$C_6$) alkanoyl, wherein heterocyclic moiety is saturated 3 to 8-membered heteromonocyclic group containing 1 to 2 oxygen atom(s) and 1 to 3 nitrogen atom(s);
heterocyclicsulfonyl, wherein heterocyclic moiety is unsaturated condensed heterocyclic group containing 1 to 4 nitrogen atom(s);
N-heterocyclic-carbamoyl, wherein heterocyclic moiety is unsaturated 3 to 8-membered heteromonocyclic group containing 1 to 4 nitrogen atom(s), which may have 1 to 3 ($C_1$-$C_6$) alkyl or halogen;
N-heterocyclic-carbamoyl, wherein heterocyclic moiety is unsaturated condensed heterocyclic group containing 1 to 4 nitrogen atoms;
N-heterocyclic-carbamoyl, wherein heterocyclic moiety is unsaturated condensed heterocyclic group containing 1 to 2 oxygen atom(s) and 1 to 3 nitrogen atom(s), which may have 1 to 3 halogen;
N-heterocyclic-carbamoyl, wherein heterocyclic moiety is unsaturated condensed heterocyclic group containing 1 to 2 sulfur atom(s) and 1 to 3 nitrogen atom(s);
N-heterocyclic-carbamoyl, wherein heterocyclic moiety is saturated 3 to 8-membered heteromonocyclic group containing 1 to 2 oxygen atom(s) and 1 to 3 nitrogen atom(s);
N-($C_1$-$C_6$) alkyl-N-heterocyclic-carbamoyl wherein heterocyclic moiety is unsaturated 3 to 8-membered heteromonocyclic group containing 1 to 4 nitrogen atom(s); or
N-heterocyclic ($C_1$-$C_6$) alkylcarbamoyl, wherein heterocyclic moiety is unsaturated 3 to 8-membered heteromonocyclic group containing 1 to 4 nitrogen atom(s);
$R^2$ is carboxy ($C_1$-$C_6$) alkyl, and
$R^4$ is carbamoyl ($C_1$-$C_6$) alkyl.

**7.** A process for the preparation of the fatty acid derivative of claim 1 or a salt thereof, which comprises

1) reacting the compound of the formula :

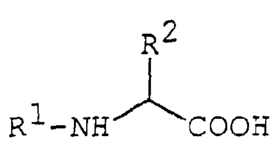

wherein $R^1$ and $R^2$ are each as defined in claim 1, or a reactive derivative at the carboxy group or a salt thereof, with the compound of the formula :

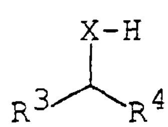

wherein $R^3$, $R^4$ and X are each as defined in claim 1, or a salt thereof,

2) reacting the compound of the formula :

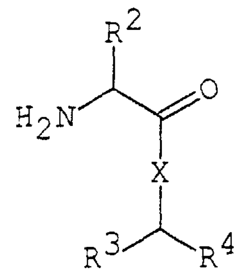

wherein $R^2$, $R^3$, $R^4$ and X are each as defined in claim 1, or a reactive derivative at the amino group or a salt thereof, with the compound of the formula :

$$R^1_a\text{-OH}$$

wherein $R^1_a$ is acyl group,
or a reactive derivative at the acyl group or a salt thereof, to give the compound of the formula :

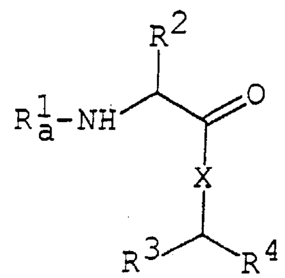

wherein $R^2$, $R^3$, $R^4$ and X are each as defined in claim 1,
and
$R^1_a$ is as defined above,
or a salt thereof,

3) reacting the compound of the formula :

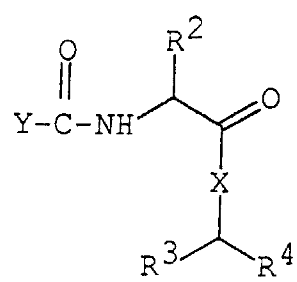

wherein $R^2$, $R^3$, $R^4$ and X are each as defined in claim 1
and
Y is a leaving group, or a salt thereof, with amidating agent, to give the compound of the formula :

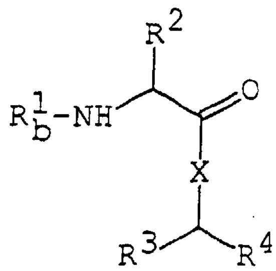

wherein $R^2$, $R^3$, $R^4$ and X are each as defined in claim 1, and $R_b^1$ is amidated carboxy, or a salt thereof,

4) subjecting the compound of the formula :

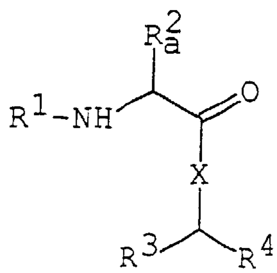

wherein $R^1$, $R^3$, $R^4$ and X are each as defined in claim 1, and $R_a^2$ is protected carboxy ($C_3$-$C_6$) alkyl, or a salt thereof, to elimination reaction of carboxy protective group, to give the compound of the formula :

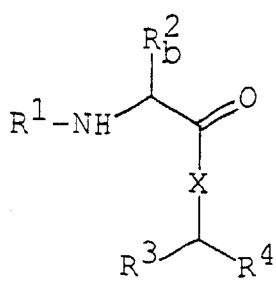

wherein $R^1$, $R^3$, $R^4$ and X are each as defined in claim 1, and $R_b^2$ is carboxy ($C_1$-$C_6$)alkyl, or a salt thereof,

5) reacting the compound of the formula :

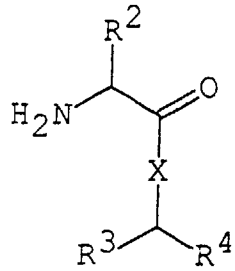

wherein $R^2$, $R^3$, $R^4$ and X are each as defined in claim 1, or a reactive derivative at the amino group or a salt thereof, with ($C_1$-$C_6$) aliphatic hydrocarbon compound substituted with oxo which may have one or more suitable substituent(s) in the presence of a reducing agent, to give the compound of the formula :

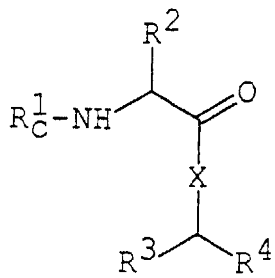

wherein $R^2$, $R^3$, $R^4$ and X are each as defined above, and

$R^1_c$ is ($C_1$-$C_6$) aliphatic hydrocarbon group which may have one or more suitable substituent(s), or a salt thereof,

6) reacting the compound of the formula :

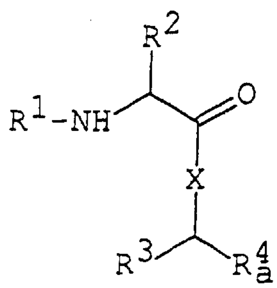

wherein $R^1$, $R^2$, $R^3$ and X are each as defined in claim 1,

and

$R^4_a$ is carboxy or carboxy ($C_1$-$C_6$) alkyl,

or a salt thereof, with amidating agent, to give the compound of the formula :

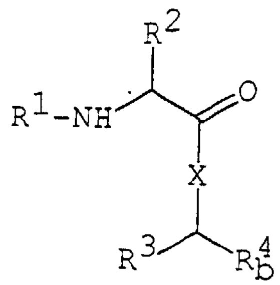

wherein $R^1$, $R^2$, $R^3$ and X are each as defined in claim 1,
and
$R_b^4$ is amidated carboxy or amidated carboxy ($C_1$-$C_6$) alkyl,
or a salt thereof, or

7) reacting the compound of the formula :

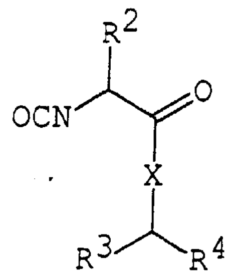

wherein $R^2$, $R^3$, $R^4$ and X are each as defined in claim 1, or a salt thereof, with amidating agent, to give the compound of the formula :

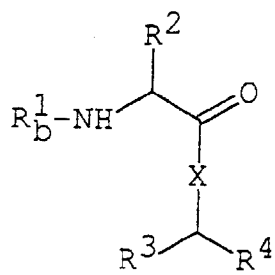

wherein $R^2$, $R^3$, $R^4$ and X are each as defined in claim 1, or a salt thereof.

**8.** A pharmaceutical composition which comprises, as an active ingredient, a fatty acid derivative of claim 1 or a pharmaceutically acceptable salt thereof in admixture with pharmaceutically acceptable carriers or excipients.

**9.** Use of a fatty acid derivative of claim 1 or a pharmaceutically acceptable salt thereof for the manufacture of a medicament.

**10.** A fatty acid derivative of claim 1 or a pharmaceutically acceptable salt thereof for use as a medicament.

**11.** Use of a fatty acid derivative of claim 1 or a pharmaceutically acceptable salt thereof for the manufacture of a medicament for the prevention and/or the treatment of pancreatitis, hepatitis, chronic renal failure, shock, arthritis ,

respiratory disease, heart disease, allergic disease, thrombosis, arteriosclerosis, pain, autoimmune disease, dermal disease, inflammatory bowel disease, ophthalmic disease, nasal diseases, gout, trauma induced inflammation or liver diseases.

## Patentansprüche

**1.** Fettsäurederivat, dargestellt durch die folgende Formel:

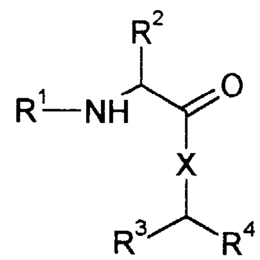

worin

$R^1$ eine Acylgruppe oder eine aliphatische $(C_1\text{-}C_6)$-Kohlenwasserstoffgruppe ist, die einen oder mehrere geeignete Substituent(en) aufweisen kann,
$R^2$ Acyl$(C_1\text{-}C_6)$alkyl ist,
$R^3$ $(C_1\text{-}C_6)$Alkyl oder $(C_7\text{-}C_{20})$Alkyl ist,
$R^4$ eine Acylgruppe, Acyl$(C_1\text{-}C_6)$alkyl oder Cyano-$(C_1\text{-}C_6)$alkyl ist und
X -O- oder

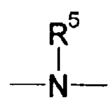

ist,
[worin $R^5$ Wasserstoff oder Acyl$(C_1\text{-}C_6)$alkyl ist,
und pharmazeutisch verträgliche Salze davon.

**2.** Derivat nach Anspruch 1, worin

$R^1$ geschütztes Carboxy; $(C_3\text{-}C_6)$Alkenoyl; Acryl$(C_1\text{-}C_6)$-alkanoyl; Aryl$(C_3\text{-}C_6)$alkenoyl; Arylsulfonyl, das einen oder mehrere geeignete Substituent(en) aufweisen kann;
Heterocyclyl$(C_1\text{-}C_6)$alkanoyl, das einen oder mehrere geeignete Substituent(en) aufweisen kann;
Heterocyclylsulfonyl; amidiertes Carboxy;
$(C_7\text{-}C_{20})$Alkanoyl, das einen oder mehrere geeignete Substituent(en) aufweisen kann;
$(C_1\text{-}C_6)$Alkyl, das einen oder mehrere geeignete Substituent(en) aufweisen kann;
$(C_2\text{-}C_6)$Alkenyl, das einen oder mehrere geeignete Substituent(en) aufweisen kann;
oder $(C_2\text{-}C_6)$Alkinyl ist, das einen oder mehrere geeignete Substituent(en) aufweisen kann,
$R^2$ Carboxy$(C_1\text{-}C_6)$alkyl oder geschütztes Carboxy$(C_1\text{-}C_6)$-alkyl ist,
$R^4$ geschütztes Carboxy; amidiertes Carboxy; Carboxy-$(C_1\text{-}C_6)$alkyl; geschütztes Carboxy$(C_1\text{-}C_6)$alkyl; amidiertes Carboxy$(C_1\text{-}C_6)$alkyl; amidiertes Carboxy$(C_1\text{-}C_6)$-alkyl; oder Cyano$(C_1\text{-}C_6)$alkyl ist,
X -O- oder

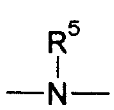

ist

[worin $R^5$ Wasserstoff oder amidiertes Carboxy($C_1$-$C_6$)-alkyl ist].

**3.** Derivat nach Anspruch 2, worin

$R^1$ ($C_1$-$C_6$)Alkoxycarbonyl; ($C_6$-$C_{10}$)Aryloxycarbonyl; ($C_3$-$C_6$)-Alkenoyl; ($C_6$-$C_{10}$)Aryl($C_1$-$C_6$)alkanoyl, das 1 bis 3 ($C_1$-$C_6$)Alkoxy oder ($C_6$-$C_{10}$)Aryl aufweisen kann;
($C_6$-$C_{10}$)Aryl($C_3$-$C_6$)alkenoyl;
($C_6$-$C_{10}$)Arylsulfonyl, das 1 bis 3 Di($C_1$-$C_6$)alkylamino aufweisen kann;
Heterocyclyl($C_1$-$C_6$)alkanoyl, worin der heterocyclische Anteil eine ungesättigte 3- bis 8-gliedrige heteromonocyclische Gruppe ist, die 1 bis 4 Stickstoffatom(e) enthält, die 1 bis 3 ($C_1$-$C_6$)Alkyl aufweisen kann;
Heterocyclyl($C_1$-$C_6$)alkanoyl, worin der heterocyclische Anteil eine ungesättigte kondensierte heterocyclische Gruppe ist, die 1 bis 4 Stickstoffatom(e) enthält, die 1 bis 3 ($C_1$-$C_6$)Alkoxycarbonyl aufweisen kann;
Heterocyclyl($C_1$-$C_6$)alkanoyl, worin der heterocyclische Anteil eine gesättigte 3- bis 8-gliedrige heteromonocyclische Gruppe ist, die 1 bis 4 Stickstoffatom(e) enthält, die 1 bis 3 ($C_1$-$C_6$)Alkoxycarbonyl oder ($C_6$-$C_{10}$)-Aryl($C_1$-$C_6$) alkyl, das ($C_1$-$C_6$)Alkylendioxy aufweisen kann;
Heterocyclyl($C_1$-$C_6$)alkanoyl, worin der heterocyclische Anteil eine gesättigte 3- bis 8-gliedrige heteromonocyclische Gruppe ist, die 1 bis 2 Sauerstoffatom(e) und 1 bis 3 Stickstoffatom(e) enthält;
Heterocyclylsulfonyl, worin der heterocyclische Anteil eine ungesättigte kondensierte heterocyclische Gruppe ist, die 1 bis 4 Stickstoffatom(e) enthält;
N-Heterocyclyl-carbamoyl, worin der heterocyclische Anteil eine ungesättigte 3- bis 8-gliedrige heteromonocyclische Gruppe ist, die 1 bis 4 Stickstoffatom(e) enthält, die 1 bis 3 ($C_1$-$C_6$)Alkyl oder Halogen aufweisen kann;
N-Heterocyclyl-carbamoyl, worin der heterocyclische Anteil eine ungesättigte kondensierte heterocyclische Gruppe ist, die 1 bis 4 Stickstoffatom(e) enthält;
N-Heterocyclyl-carbamoyl, worin der heterocyclische Anteil eine ungesättigte kondensierte heterocyclische Gruppe ist, die 1 bis 2 Sauerstoffatom(e) und 1 bis 3 Stickstoffatom(e) enthält, die 1 bis 3 Halogene aufweisen kann;
N-Heterocyclyl-carbamoyl, worin der heterocyclische Anteil eine ungesättigte kondensierte heterocyclische Gruppe ist, die 1 bis 2 Schwefelatom(e) und 1 bis 3 Stickstoffatom(e) aufweist;
N-Heterocyclyl-carbamoyl, worin der heterocyclische Anteil eine gesättigte 3- bis 8-gliedrige heteromonocyclische Gruppe ist, die 1 bis 2 Sauerstoffatom(e) und 1 bis 3 Stickstoffatom(e) enthält;
N-($C_1$-$C_6$)Alkyl-N-heterocyclycl-carbamoyl, worin der heterocyclische Anteil eine ungesättigte 3- bis 8-gliedrige heteromonocyclische Gruppe ist, die 1 bis 4 Stickstoffatom(e) enthält;
N-Heterocyclyl($C_1$-$C_6$)alkylcarbamoyl, worin der heterocyclische Anteil eine ungesättigte 3- bis 8-gliedrige heteromonocyclische Gruppe ist, die 1 bis 4 Stickstoffatom(e) enthält;
N-($C_1$-$C_6$)Alkylcarbamoyl, das 1 bis 3 Hydroxy aufweisen kann;
($C_7$-$C_{20}$)Alkanoyl, das 1 bis 3 Hydroxy aufweisen kann; oder ($C_1$-$C_6$)Alkyl ist, das 1 bis 3 ungesättigte oder 3-bis 8-gliedrige heteromonocyclische Gruppen, die 1 bis 4 Stickstoffatom(e) enthalten, aufweisen kann;

$R^2$ Carboxy($C_1$-$C_6$)alkyl; ($C_1$-$C_6$)Alkoxycarbonyl($C_1$-$C_6$)alkyl oder ($C_6$-$C_{10}$)Aryl($C_1$-$C_6$)alkoxycarbonyl ist;

$R^4$ ($C_1$-$C_6$)Alkoxycarbonyl; Carbamoyl; N-($C_1$-$C_6$)Alkylcarbamoyl; Carboxy($C_1$-$C_6$)alkyl; ($C_1$-$C_6$)Alkoxycarbonyl($C_1$-$C_6$)alkyl; Halogen($C_1$-$C_6$)alkoxycarbonyl($C_1$-$C_6$)alkyl; ($C_6$-$C_{10}$)Aryl($C_1$-$C_6$)alkoxycarbonyl($C_1$-$C_6$)alkyl; Carbamoyl($C_1$-$C_6$)alkyl; N-($C_1$-$C_6$)Alkylcarbamoyl($C_1$-$C_6$)alkyl; N-($C_6$-$C_{10}$)Aryl ($C_1$-$C_6$)alkylcarbamoyl ($C_1$-$C_6$)alkyl; oder Cyano($C_1$-$C_6$)alkyl ist und

X -O- oder

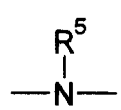

ist

[worin $R^5$ Wasserstoff; Carbamoyl($C_1$-$C_6$)Alkyl; oder N-($C_1$-$C_6$)Alkylcarbamoyl ($C_1$-$C_6$)alkyl ist].

**4.** Derivat nach Anspruch 3, worin

$R^3$ ($C_7$-$C_{20}$)Alkyl ist,

$R^4$ ($C_1$-$C_6$)Alkoxycarbonyl; Carbamoyl; N-($C_1$-$C_6$)Alkylcarbamoyl; Carboxy($C_1$-$C_6$) alkyl; ($C_1$-$C_6$)Alkoxycarbonyl ($C_1$-$C_6$)alkyl; Halogen($C_1$-$C_6$)alkoxycarbonyl ($C_1$-$C_6$)alkyl; Phenyl($C_1$-$C_6$)alkoxycarbonyl($C_1$-$C_6$) alkyl; Carbamoyl($C_1$-$C_6$)alkyl; N-Niederalkylcarbamoyl ($C_1$-$C_6$) alkyl; oder N-Phenyl($C_1$-$C_6$)alkylcarbamoyl($C_1$-$C_6$)alkyl ist;
und
X -O- ist.

**5.** Derivat nach Anspruch 4, worin

$R^1$ ($C_1$-$C_6$)Alkoxycarbonyl; Phenoxycarbonyl; ($C_3$-$C_6$)-Alkenoyl; Phenyl($C_1$-$C_6$)alkanoyl, das 1 bis 3 ($C_1$-$C_6$)-Alkoxy oder Phenyl aufweisen kann; Naphthyl($C_1$-$C_6$)-alkanoyl; Phenyl($C_3$-$C_6$)alkenoyl; N-($C_1$-$C_6$)alkylcarbamoyl, das 1 bis 3 Hydroxy aufweisen kann; oder Naphthylsulfonyl ist, das 1 bis 3 Di($C_1$-$C_6$)alkylamino aufweisen kann;
$R^2$ Carboxy($C_1$-$C_6$)alkyl ist,
$R^4$ Carbamoyl($C_1$-$C_6$)alkyl ist.

**6.** Derivat nach Anspruch 4, worin

$R^1$ Heterocyclyl($C_1$-$C_6$)alkanoyl ist, worin der heterocyclische Anteil eine ungesättigte 3- bis 8-gliedrige heteromonocyclische Gruppe ist, die 1 bis 4 Stickstoffatom(e) enthält, die 1 bis 3 ($C_1$-$C_6$)Alkyl aufweisen kann;
Heterocyclyl($C_1$-$C_6$)alkanoyl, worin der heterocyclische Anteil eine ungesättigte kondensierte heterocyclische Gruppe, die 1 bis 4 Stickstoffatom(e) enthält, die 1 bis 3 ($C_1$-$C_6$)Alkoxycarbonyl aufweisen kann;
Heterocyclyl($C_1$-$C_6$)alkanoyl, worin der heterocyclische Anteil eine gesättigte 3- bis 8-gliedrige heteromonocyclische Gruppe ist, die 1 bis 4 Stickstoffatom(e) enthält, die 1 bis 3 ($C_1$-$C_6$)Alkoxycarbonyl oder ($C_6$-$C_{10}$)Aryl($C_1$-$C_6$)alkyl, das ($C_1$-$C_6$)Alkylendioxy aufweisen kann, aufweisen kann;
Heterocyclyl($C_1$-$C_6$)alkanoyl, worin der heterocyclische Anteil eine gestättigte 3- bis 8-gliedrige heteromonocyclische Gruppe ist, die 1 bis 2 Sauerstoffatom(e) und 1 bis 3 Stickstoffatom(e) enthält;
Heterocyclylsulfonyl, worin der heterocyclische Anteil eine ungestättigte kondensierte heterocyclische Gruppe ist, die 1 bis 4 Stickstoffatom(e) enthält;
N-Heterocyclyl-carbamoyl, worin der heterocyclische Anteil eine ungesättigte 3- bis 8-gliedrige heteromonocyclische Gruppe ist, die 1 bis 4 Stickstoffatom(e) enthält, die 1 bis 3 ($C_1$-$C_6$)Alkyl oder Halogen aufweisen kann;
N-Heterocyclyl-carbamoyl, worin der heteocyclische Anteil eine ungesättigte kondensierte heterocyclische Gruppe ist, die 1 bis 4 Stickstoffatom(e) enthält;
N-Heterocyclyl-carbamoyl, worin der heterocyclische Anteil eine ungesättigte kondensierte heterocyclische Gruppe ist, die 1 bis 2 Sauerstoffatom(e) und 1 bis 3 Stickstoffatom(e) enthält, die 1 bis 3 Halogenatome aufweisen kann;
N-Heterocyclyl-carbamoyl, worin der heterocyclische Anteil eine ungesättigte kondensierte heterocyclische Gruppe ist, die 1 bis 2 Schwefelatom(e) und 1 bis 3 Stickstoffatom(e) enthält;
N-Heterocyclyl-carbamoyl, worin der heterocyclische Anteil eine gesättigte 3- bis 8-gliedrige heteromonocyclische Gruppe ist, die 1 bis 2 Sauerstoffatom(e) und 1 bis 3 Stickstoffatom(e) enthält;
N-($C_1$-$C_6$)Alkyl-N-heterocyclylcarbamoyl, worin der heterocyclische Anteil eine ungesättigte 3- bis 8-gliedrige heteromonocyclische Gruppe ist, die 1 bis 4 Stickstoffatom(e) enthält; oder
N-Heterocyclyl($C_1$-$C_6$)alkylcarbamoyl, worin der heterocyclische Anteil eine ungesättigte 3- bis 8-gliedrige heteromonocyclische Gruppe ist, die 1 bis 4 Stickstoffatom(e) enthält;
$R^2$ Carboxy($C_1$-$C_6$)alkyl ist und
$R^4$ Carbamoyl($C_1$-$C_6$)alkyl ist.

**7.** Verfahren zur Herstellung des Fettsäurederivates nach Anspruch 1 oder eines Salzes davon, das umfaßt:

1) Umsetzen der Verbindung der Formel:

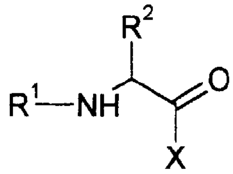

worin $R^1$ und $R^2$ jeweils wie im Anspruch 1 definiert sind, oder eines reaktiven Derivates an der Carboxygruppe oder eines Salzes davon mit der Verbindung der Formel:

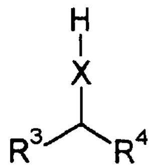

worin $R^3$, $R^4$ und X jeweils wie in Anspruch 1 definiert sind, oder einem Salz davon,

2) Umsetzen der Verbindung der Formel:

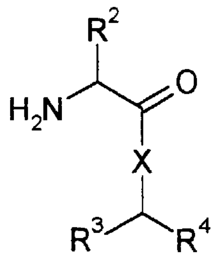

worin $R^2$, $R^3$, $R^4$ und X jeweils wie in Anspruch 1 definiert sind oder eines reaktiven Derivates an der Aminogruppe oder eines Salzes davon mit der Verbindung der Formel:

$$R^1{}_a - OH$$

worin $R^1{}_a$ eine Acylgruppe ist,
oder einem Salz davon, um die Verbindung der Formel:

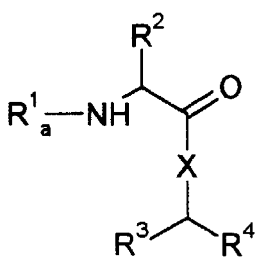

worin $R^2$, $R^3$, $R^4$ und X jeweils wie in Anspruch 1 definiert sind und
$R^1{}_a$ wie oben definiert ist

oder ein Salz davon zu ergeben,

3) Umsetzen der Verbindung der Formel:

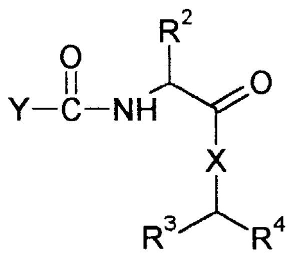

worin $R^2$, $R^3$, $R^4$ und X jeweils wie in Anspruch 1 definiert sind und
Y eine Abgangsgruppe ist,
oder eines Salzes davon mit einem Amidierungsmittel, um eine Verbindung der Formel:

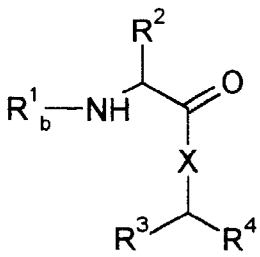

worin $R^2$, $R^3$, $R^4$ und X jeweils wie in Anspruch 1 definiert sind und
$R^1_b$ amidiertes Carboxy ist,
oder ein Salz davon zu ergeben,

4) Unterwerfen der Verbindung der Formel:

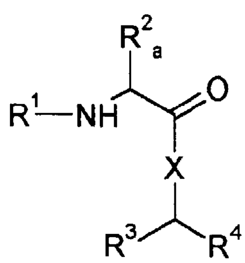

worin $R^1$, $R^3$, $R^4$ und X jeweils wie in Anspruch 1 definiert sind und
$R^2_a$ geschütztes Carboxy($C_1$-$C_6$)alkyl ist
oder eines Salzes davon der Eliminierungsreaktion der Carboxyschutzgruppe, um eine Verbindung der Formel:

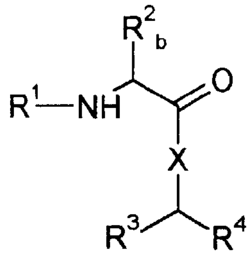

worin $R^1$, $R^3$, $R^4$ und X jeweils wie in Anspruch 1 definiert sind und
$R^2_b$ Carboxy($C_1$-$C_6$)alkyl ist,
oder ein Salz davon zu ergeben,

5) Umsetzen der Verbindung der Formel:

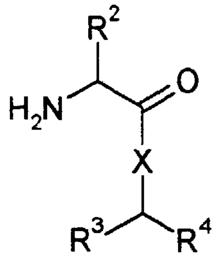

worin $R^2$, $R^3$, $R^4$ und X jeweils wie in Anspruch 1 definiert sind, ode eines reaktiven Derivates an der Aminogruppe oder eines Salzes davon mit einer aliphatischen ($C_1$-$C_6$)Kohlenwasserstoffverbindung die mit Oxo substituiert ist, und die einen oder mehrere geeignete Substituent(en) aufweisen kann in Gegenwart eines Reduktionsmittels, um eine Verbindung der Formel:

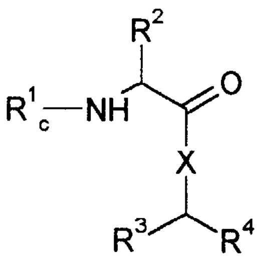

worin $R^2$, $R^3$, $R^4$ und X jeweils wie oben definiert sind und
$R^1_c$ eine aliphatische ($C_1$-$C_6$)Kohlenwasserstoffgruppe ist, die einen oder mehrere geeignete Substituent(en) aufweist, oder ein Salz davon zu erhalten,

6) Umsetzen der Verbindung der Formel:

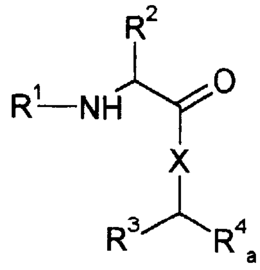

worin $R^1$, $R^2$, $R^3$ und X jeweils wie in Anspruch 1 definiert sind und
$R^4_a$ Carboxy oder Carboxy($C_1$-$C_6$)alkyl ist,
oder ein Salz davon mit einem Amidierungsmittel, um eine Verbindung der Formel:

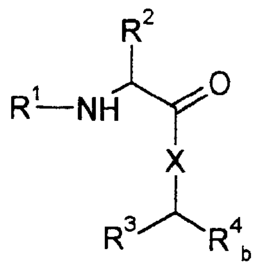

worin $R^1$, $R^2$, $R^3$ und X jeweils wie in Anspruch 1 definiert sind und
$R^4_b$ amidiertes Carboxy oder amidiertes Carboxy($C_1$-$C_6$)-alkyl ist
oder ein Salz davon zu ergeben, oder

7) Umsetzen der Verbindung der Formel:

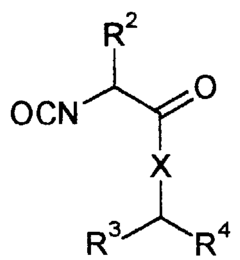

worin $R^2$, $R^3$, $R^4$ und X jeweils wie in Anspruch 1 definiert sind oder ein Salz davon mit einem Amidierungsmittel, um eine Verbindung der Formel:

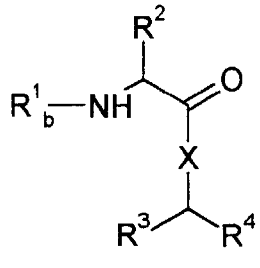

worin $R^2$, $R^3$, $R^4$ und X jeweils wie in Anspruch 1 definiert sind oder ein Salz davon zu ergeben.

**8.** Pharmazeutische Zusammensetzung, die als aktiven Bestandteil ein Fettsäurederivat nach Anspruch 1 oder ein pharmazeutisch verträgliches Salz davon in Mischung mit einem pharmazeutisch verträglichen Träger oder Exzipienten umfaßt.

**9.** Verwendung eines Fettsäurederivates nach Anspruch 1 oder eines pharmazeutisch verträglichen Salzes davon für die Herstellung eines Medikamentes.

**10.** Fettsäurederivat nach Anspruch 1 oder ein pharmazeutisch verträgliches Salze davon zur Verwendung als Medikament.

**11.** Verwendung eines Fettsäurederivates nach Anspruch 1 oder eines pharmazeutisch verträglichen Salzes davon für die Herstellung eines Medikamentes zur Prävention oder Behandlung von Pancreatitis, Hepatitis, chronischem Nierenversagen, Schock, Arthritis, Atemwegserkrankungen, Herzerkrankung, allergischen Erkrankungen, Thrombosen, Arteriosklerose, Schmerzen, Autoimmunerkrankungen, Hauterkrankungen, entzündliche Darmerkankungen, Augenerkrankung, Nasenerkrankungen, Gicht, Trauma-induzierten Entzündungen oder Lebererkrankungen.

## Revendications

**1.** Dérivé d'acide gras répondant à la formule suivante :

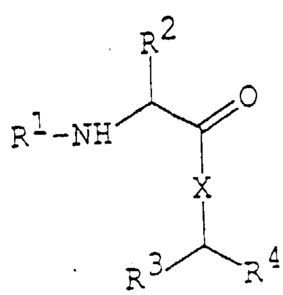

dans laquelle

$R^1$ est un groupe acyle ou un groupe hydrocarboné aliphatique en $C_1$ à $C_6$ qui peut avoir un ou plusieurs substituants appropriés,
$R^2$ est un groupe acyl(alkyle en $C_1$ à $C_6$),
$R^3$ est un groupe (alkyle en $C_1$ à $C_6$) ou (alkyle en $C_7$ à $C_{20}$),
$R^4$ est un groupe acyle, un groupe acyl(alkyle en $C_1$ à $C_6$) ou un groupe cyano(alkyle en $C_1$ à $C_6$) et
X est -O- ou

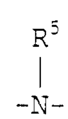

[où $R^5$ est un atome d'hydrogène ou un groupe acyle (alkyle en $C_1$ à $C_6$)],

et un de ses sels pharmaceutiquement acceptables.

**2.** Dérivé selon la revendication 1, dans lequel

$R^1$ est un groupe carboxy protégé ; alcénoyle en $C_3$ à $C_6$ ; (aryle en $C_1$ à $C_6$)alcanoyle ; aryl(alcénoyle en $C_1$ à $C_6$) ; arylsulfonyle qui peut avoir un ou plusieurs substituants appropriés ;
hétérocyclique-(alcanoyle en $C_1$ à $C_6$) qui peut avoir un ou plusieurs substituants appropriés ;

hétérocyclique-sulfonyle ; carboxy amidé ; (alcanoyle en $C_7$ à $C_{20}$) qui peut avoir un ou plusieurs substituants appropriés ; alkyle en $C_1$ à $C_6$ qui peut avoir un ou plusieurs substituants appropriés ; alcényle en $C_2$ à $C_6$ qui peut avoir un ou plusieurs substituants appropriés ; ou alcynyle en $C_2$ à $C_6$ qui peut avoir un ou plusieurs substituants appropriés,

$R^2$ est un groupe carboxy(alkyle en $C_2$ à $C_6$) ou (carboxy protégé)(alkyle en $C_2$ à $C_6$),

$R^4$ est un groupe alkoxy protégé ; un groupe carboxy amidé ; un groupe carboxy(alkyle en $C_2$ à $C_6$) ; un groupe (carboxy protégé (alkyle en $C_2$ à $C_6$) ; un groupe (carboxy amidé)(alkyle en $C_1$ à $C_6$) ou un groupe cyano (alkyle en $C_1$ à $C_6$),

X est -O- ou

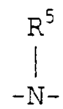

[où $R^5$ est un atome d'hydrogène ou un groupe (carboxy amidé) (alkyle en $C_1$ à $C_6$)].

**3.** Dérivé selon la revendication 2, dans lequel

$R^1$ est un groupe (alkoxy en $C_1$ à $C_6$)carbonyle ; (aryloxy en $C_6$ à $C_{10}$)carbonyle ; (alcénoyle en $C_3$ à $C_6$) ; (aryle en $C_6$ à $C_{10}$)(alcanoyle en $C_1$ à $C_6$) qui peut avoir 1 ou 3 groupes alkoxy en $C_1$ à $C_6$ ou aryle en $C_6$ à $C_{10}$) ; (aryle en $C_6$ à $C_{10}$) (alcénoyle en $C_3$ à $C_6$) ; (aryle en $C_6$ à $C_{10}$)sulfonyle qui peut avoir 1 ou 3 groupes di(alkylamino) ; (alcanoyle en $C_1$ à $C_6$)hétérocyclique, dans lequel la partie hétérocyclique est un groupe hétéromonocyclique insaturé à 3 à 8 maillons contenant 1 à 4 atomes d'azote, qui peut avoir 1 à 3 groupes alkyle en $C_1$ à $C_6$ ; (alcanoyle en $C_1$ à $C_6$)-hétérocyclique dans lequel la partie hétérocyclique est un groupe hétérocyclique condensé insaturé contenant 1 à 4 atomes d'azote, qui peut avoir 1 à 3 groupes (alkoxy en $C_1$ à $C_6$) carbonyle ; (alcanoyle en $C_1$ à $C_6$)hétérocyclique, dans lequel la partie hétérocyclique est un groupe hétéromonocyclique à 3 à 8 maillons, saturé contenant 1 à 4 atomes d'azote, qui peut avoir 1 à 3 groupes (alkoxy en $C_1$ à $C_6$)carbonyle ou (aryle en $C_1$ à $C_6$)-(alkyle en $C_1$ à $C_6$) qui peut avoir un groupe (alkylène en $C_1$ à $C_6$)dioxy ; (alcanoyle en $C_1$ à $C_6$)hétérocyclique, dans lequel la partie hétérocyclique est un groupe hétéromonocyclique à 3 à 8 maillons, saturé contenant 1 à 2 atomes d'oxygène et 1 à 3 atomes d'azote ; un groupe sulfonyle hétérocyclique, dans lequel la partie hétérocyclique est un groupe hétérocyclique condensé insaturé contenant 1 à 4 atomes d'azote ; N-hétérocyclique-carbamoyle, dans lequel la partie hétérocyclique est un groupe hétéromonocyclique à 3 à 8 maillons, insaturé, contenant 1 à 4 atomes d'azote, qui peut avoir 1 à 3 groupes alkyle en $C_1$ à $C_6$ ou un halogène ; un groupe N-hétérocyclique-carbamoyle, dans lequel la partie hétérocyclique est un groupe hétérocyclique condensé insaturé contenant 1 à 4 atomes d'azote ; un groupe N-hétérocyclique-carbamoyle, dans lequel la partie hétérocyclique est un groupe hétérocyclique condensé insaturé contenant 1 à 2 atomes d'oxygène et 1 à 3 atomes d'azote, qui peut avoir 1 à 3 atomes d'halogène ; un groupe N-hétérocyclique-carbamoyle, dans lequel la partie hétérocyclique est un groupe hétérocyclique condensé insaturé contenant 1 à 2 atomes de soufre et 1 à 3 atomes d'azote ; un groupe N-hétérocyclique-carbamoyle, dans lequel la partie hétérocyclique est un groupe hétéromonocyclique à 3 à 8 maillons, saturé, contenant 1 à 2 atomes d'oxygène et 1 à 3 atomes d'azote ; un groupe N-(alkyle en $C_1$ à $C_6$)-N-hétérocyclique-carbamoyle, dans lequel la partie hétérocyclique est un groupe hétéromonocyclique à 3 à 8 maillons, insaturé, contenant 1 à 4 atomes d'azote ; un groupe N-hétérocyclique-(alkyle en $C_1$ à $C_6$)-carbamoyle, dans lequel la partie hétérocyclique est un groupe hétéromonocyclique à 3 à 8 maillons, insaturé, contenant 1 à 4 atomes d'azote ; un groupe N-(alkyle en $C_1$ à $C_6$)-carbamoyle qui peut avoir 1 à 3 groupes hydroxy ; un groupe alcanoyle en $C_7$ à $C_{20}$ qui peut avoir 1 à 3 groupes hydroxy ; ou un groupe alkyle en $C_1$ à $C_6$ qui peut avoir 1 à 3 groupes hétéromonocycliques à 3 à 8 maillons, insaturé, contenant 1 à 4 atomes d'azote ;

$R^2$ est un groupe carboxy(alkyle en $C_1$ à $C_6$) ; (alkoxy en $C_1$ à $C_6$)-carbonyl(alkyle en $C_1$ à $C_6$) ou (aryle en $C_6$ à $C_{10}$)(alkoxy en $C_1$ à $C_6$)carbonyle ;

$R^4$ est un groupe (alkoxy en $C_1$ à $C_6$)carbonyle ; carbamoyle ; N-(alkyle en $C_1$ à $C_6$)carbamoyle ; carboxy(alkyle en $C_1$ à $C_6$) ; (alkoxy en $C_1$ à $C_6$)-carbonyl(alkyle en $C_1$ à $C_6$) ; halo(alkoxy en $C_1$ à $C_6$)carbonyl(alkyle en $C_1$ à $C_6$) ;
aryle en $C_6$ à $C_{10}$)(alkoxy en $C_1$ à $C_6$)carbonyl(alkyle en $C_1$ à $C_6$) ; carbamoyl(alkyle en $C_1$ à $C_6$) ;
N-(alkyle en $C_1$ à $C_6$)carbamoyl(alkyle en $C_1$ à $C_6$) ;
N-(aryle en $C_6$ à $C_{10}$)lalkyle en $C_1$ à $C_6$)carbamoyl-(alkyle en $C_1$ à $C_6$) ; ou cyano(alkyle en $C_1$ à $C_6$) et
X est -O- ou

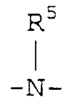

[où $R^5$ est un atome d'hydrogène ; un groupe carbamoyl(alkyle en $C_1$ à $C_6$) ; ou N-(alkyle en $C_1$ à $C_6$)-carbamoyl(alkyle en $C_1$ à $C_6$).

**4.** Dérivé selon la revendication 3, dans lequel

$R^3$ est un groupe alkyle en $C_7$ à $C_{20}$,
$R^4$ est un groupe (alkoxy en $C_1$ à $C_6$)carbonyle, un groupe carbamoyle ; un groupe N-(alkyle en $C_1$ à $C_6$) carbamoyle ; un groupe carboxy(alkyle en $C_1$ à $C_6$) ; un groupe (alkoxy en $C_1$ à $C_6$)carbonyl(alkyle en $C_1$ à $C_6$) ;
un groupe halo(alkoxy en $C_1$ à $C_6$)carbonyl(alkyle en $C_1$ à $C_6$) ;
un groupe phényl(alkoxy en $C_1$ à $C_6$)carbonyl(alkyle en $C_1$ à $C_6$) ;
un groupe carbamoyl(alkyle en $C_1$ à $C_6$) ;
un groupe N-(alkyle inférieur)carbamoyl(alkyle en $C_1$ à $C_6$) ; ou
un groupe N-phényl(alkyle en $C_1$ à $C_6$)carbamoyl(alkyle en $C_1$ à $C_6$) ; et
X est -O-.

**5.** Dérivé selon la revendication 4, dans lequel

$R^1$ est un groupe (alkoxy en $C_1$ à $C_6$)carbonyle ; phénoxycarbonyle ; (alcénoyle en $C_3$ à $C_6$) ; phényl(alcanoyle en $C_1$ à $C_6$) qui peut avoir 1 à 3 groupes alkoxy en $C_1$ à $C_6$ ou phényle ;
naphtyl(alcanoyle en $C_1$ à $C_6$) ; phényl(alcénoyle en $C_3$ à $C_6$) ;
N-(alkyle en $C_1$ à $C_6$)carbonyle qui peut avoir 1 à 3 groupes hydroxy ;
un groupe alcanoyle en $C_7$ à $C_{20}$ qui peut avoir 1 à 3 groupes hydroxy ; ou un groupe naphtylsulfonyle qui peut avoir 1 à 3 groupes di(alkyle en $C_1$ à $C_6$)-amino ;
$R^2$ est un groupe carboxy(alkyle en $C_1$ à $C_6$) ; et
$R^4$ est un groupe carbamoyl(alkyle en $C_1$ à $C_6$).

**6.** Dérivé selon la revendication 4, dans lequel

$R^1$ est un groupe (alcanoyle en $C_1$ à $C_6$)-hétérocyclique, dans lequel la partie hétérocyclique est un groupe hétéromonocyclique à 3 à 8 maillons, insaturé, contenant 1 à 4 atomes d'azote, qui peut avoir 1 à 3 groupes (alkyle en $C_1$ à $C_6$) ;
(alcanoyle en $C_1$ à $C_6$)-hétérocyclique, dans lequel la partie hétérocyclique est un groupe hétérocyclique condensé, insaturé, contenant 1 à 4 atomes d'azote, qui peut avoir 1 à 3 groupes (alkoxy en $C_1$ à $C_6$) carbonyle ;
(alcanoyle en $C_1$ à $C_6$)-hétérocyclique, dans lequel la partie hétérocyclique est un groupe hétéromonocyclique à 3 à 8 maillons, saturé, contenant 1 à 4 atomes d'azote qui peut avoir 1 à 3 groupes (alkoxy en $C_1$ à $C_6$)carbonyle ou (aryle en $C_6$ à $C_{10}$)-(alkyle en $C_1$ à $C_6$) qui peut avoir un groupe (alkylène en $C_1$ à $C_6$)dioxy ;
un groupe (alcanoyle en $C_1$ à $C_6$)hétérocyclique, dans lequel la partie hétérocyclique est un groupe hétéromonocyclique à 3 à 8 maillons, saturé, contenant 1 à 2 atomes d'oxygène et 1 à 3 atomes d'azote ;
un groupe hétérocyclique-sulfonyle, dans lequel la partie hétérocyclique est un groupe hétérocyclique condensé insaturé contenant 1 à 4 atomes d'azote ;
un groupe N-hétérocyclique-carbamoyle, dans lequel la partie hétérocyclique est un groupe hétérocyclique à 3 à 8 maillons, insaturé, contenant 1 à 4 atomes d'azote, qui peut avoir 1 à 3 groupes (alkyle en $C_1$ à $C_6$) ou atomes d'halogène ;

un groupe N-hétérocyclique-carbamoyle, dans lequel la partie hétérocyclique est un groupe hétérocyclique condensé, insaturé, contenant 1 à 4 atomes d'azote ;
un groupe N-hétérocyclique-carbamoyle, dans lequel la partie hétérocyclique est un groupe hétérocyclique condensé, insaturé, contenant 1 à 2 atomes d'oxygène et 1 à 3 atomes d'azote, qui peut avoir 1 à 3 atomes d'halogène ;
un groupe N-hétérocyclique-carbamoyle, dans lequel la partie hétérocyclique est un groupe hétérocyclique condensé, insaturé, contenant 1 à 2 atomes de soufre et 1 à 3 atomes d'azote ;
un groupe N-hétérocyclique-carbamoyle, dans lequel la partie hétérocyclique est un groupe hétéromonocyclique à 3 à 8 maillons, saturé, contenant 1 à 2 atomes d'oxygène et 1 à 3 atomes d'azote ;
un groupe N-(alkyle en $C_1$ à $C_6$)-N-hétérocycliquecarbamoyle, dans lequel la partie hétérocyclique est un groupe hétéromonocyclique à 3 à 8 maillons, insaturé, contenant 1 à 4 atomes d'azote ; ou
un groupe N-hétérocyclique (alkyle en $C_1$ à $C_6$)-carbamoyle, dans lequel la partie hétérocyclique est un groupe hétéromonocyclique à 3 à 8 maillons, insaturé, contenant 1 à 4 atomes d'azote

$R^2$ est un groupe carboxy(alkyle en $C_1$ à $C_6$) ; et
$R^4$ est un groupe carbamoyl(alkyle en $C_1$ à $C_6$).

**7.** Procédé de préparation du dérivé d'acide gras selon la revendication 1 ou d'un de ses sels, qui comprend

1) le fait de faire réagir le composé répondant à la formule :

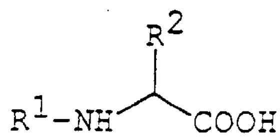

dans laquelle $R^1$ et $R^2$ sont chacun tels que définis dans la revendication 1, ou un de ses dérivés réactifs sur le groupe carboxy ou un de ses sels, avec le composé répondant à la formule :

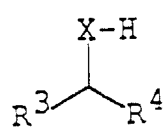

dans laquelle $R^3$, $R^4$ et X sont chacun tels que définis dans la revendication 1, ou un de ses sels,
2) le fait de faire réagir un composé répondant à la formule :

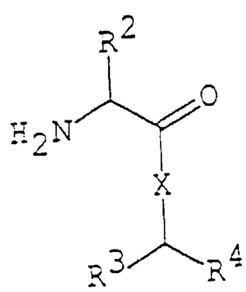

dans laquelle $R^2$, $R^3$, $R^4$ et X sont chacun tels que définis dans la revendication 1, ou un de ses dérivés réactifs sur le groupe amino ou un de ses sels, avec le composé répondant à la formule :

$$R_a^1 - OH$$

dans laquelle $R_a^1$ est un groupe acyle,

ou un de ses dérivés réactifs sur le groupe acyle ou un de ses sels, pour donner le composé répondant à la formule :

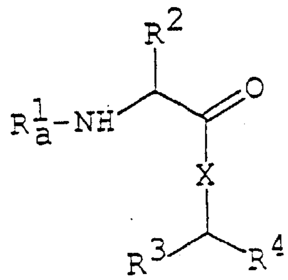

dans laquelle $R^2$, $R^3$, $R^4$ et X sont chacun tels que définis dans la revendication 1, et $R^1_a$ est tel que défini ci-dessus ou un de ses sels.

3) le fait de faire réagir le composé répondant à la formule :

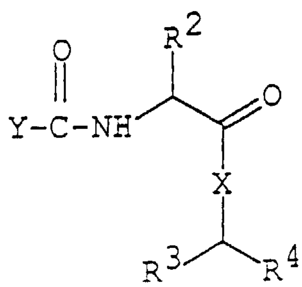

dans laquelle $R^2$, $R^3$, $R^4$ et X sont chacun tels que définis dans la revendication 1, et Y est un groupe partant, ou un de ses sels, avec un agent d'amidation, pour donner le composé répondant à la formule :

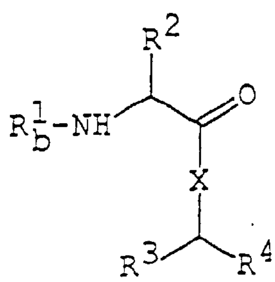

dans laquelle $R^2$, $R^3$, $R^4$ et X sont chacun tels que définis dans la revendication 1, et $R^1_b$ est un groupe carboxy amidé, ou un de ses sels,

4) le fait de soumettre le composé répondant à la formule :

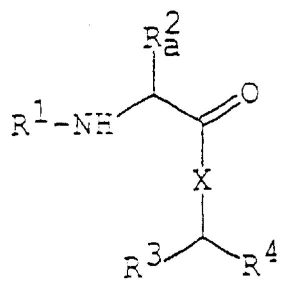

dans laquelle $R^1$, $R^3$, $R^4$ et X sont chacun tels que définis dans la revendication 1, et $R^2_a$ est un groupe (carboxy protégé)(alkyle en $C_1$ à $C_6$), ou un de ses sels, à une réaction d'élimination du groupe protecteur du groupe carboxy pour donner le composé répondant à la formule :

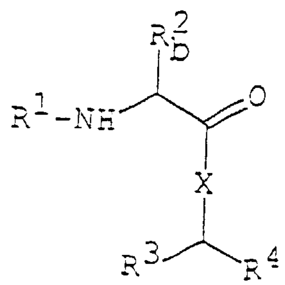

dans laquelle $R^1$, $R^3$, $R^4$ et X sont chacun tels que définis dans la revendication 1, et
$R^2_b$ est un groupe carboxy(alkyle en $C_1$ à $C_6$),
ou un de ses sels,
5) le fait de faire réagir le composé répondant à la formule :

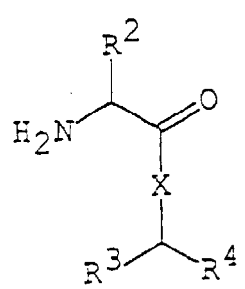

dans laquelle $R^2$, $R^3$, $R^4$ et X sont chacun tels que définis dans la revendication 1 ou un de ses dérivés réactifs sur le groupe amino ou un de ses sels, avec un composé hydrocarboné aliphatique en $C_1$ à $C_6$ substitué par un groupe oxo qui peut avoir un ou plusieurs substituants appropriés, en présence d'un agent réducteur, pour donner le composé répondant à la formule :

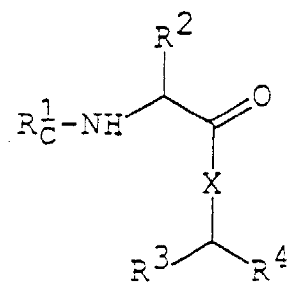

dans laquelle $R^2$, $R^3$, $R^4$ et X sont chacun tels que définis ci-dessus, et $R^1_c$ est un groupe hydrocarboné aliphatique en $C_1$ à $C_6$ qui peut avoir un ou plusieurs substituants appropriés, ou un de ses sels,

6) le fait de faire réagir le composé répondant à la formule :

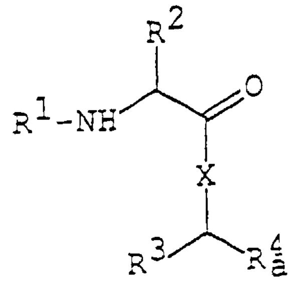

dans laquelle $R^1$, $R^2$, $R^3$ et X sont chacun tels que définis dans la revendication 1, et $R^4_a$ est un groupe carboxy ou carboxy(alkyle en $C_1$ à $C_6$), ou un de ses sels, avec un agent d'amidation, pour donner le composé répondant à la formule :

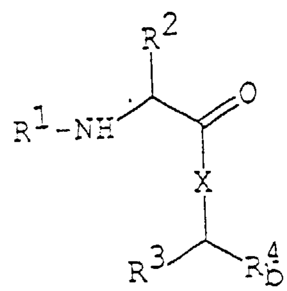

dans laquelle $R^1$, $R^2$, $R^3$ et X sont chacun tels que définis dans la revendication 1, et $R^4_b$ est un groupe carboxy amidé ou (carboxy amidé)(alkyle en $C_1$ à $C_6$), ou un de ses sels, ou

7) le fait de faire réagir le composé répondant à la formule :

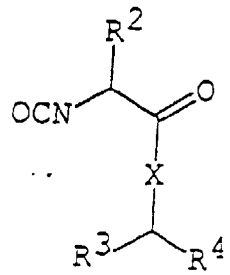

dans laquelle $R^2$, $R^3$, $R^4$ et X sont chacun tels que définis dans la revendication 1, ou un de ses sels, avec un agent d'amidation, pour donner le composé répondant à la formule :

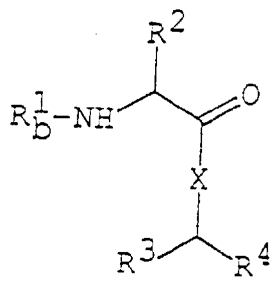

dans laquelle $R^2$, $R^3$, $R^4$ et X sont chacun tels que définis dans la revendication 1, ou un de ses sels.

**8.** Composition pharmaceutique qui comprend, comme ingrédient actif, un dérivé d'acide gras selon la revendication 1 ou un de ses sels pharmaceutiquement acceptables en mélange avec des supports ou excipients pharmaceutiquement acceptables.

**9.** Utilisation d'un dérivé d'acide gras selon la revendication 1 ou d'un de ses sels pharmaceutiquement acceptables pour la fabrication d'un médicament.

**10.** Dérivé d'acide gras selon la revendication 1 ou un de ses sels pharmaceutiquement acceptables pour l'utilisation comme médicament.

**11.** Utilisation d'un dérivé d'acide gras selon la revendication 1 ou d'un de ses sels pharmaceutiquement acceptables pour la fabrication d'un médicament pour la prévention et/ou le traitement de la pancréatite, de l'hépatite, de l'insuffisance rénale chronique, du choc, de l'arthrite, des maladies respiratoires, des maladies cardiaques, des maladies allergiques, de la thrombose, de l'artériosclérose, de la douleur, des maladies auto-immunes, des maladies dermiques, des infections intestinales inflammatoires, des maladies ophtalmiques, des maladies nasales, de la goutte, de l'inflammation provoquée par un traumatisme ou des maladies hépatiques.